# EUROPEAN PATENT SPECIFICATION

(11) **EP 3 448 993 B1**
(45) Date of publication and mention of the grant of the patent: **30.04.2025**
(21) Application number: 17790648.4
(22) Date of filing: 01.05.2017
(51) Int. Cl.: A61K 35/30, C12N 9/88, A61K 38/51, A61K 39/40, A61K 48/00, A61K 39/00, C07K 16/10, C07K 16/12, C07K 16/30

(54) **THE IN VIVO USE OF CHONDROITINASE AND/OR HYALURONIDASE TO ENHANCE DELIVERY OF AN AGENT**
IN-VIVO-VERWENDUNG VON CHONDROITINASE UND/ODER HYALURONIDASE ZUR VERBESSERTEN FREISETZUNG EINES WIRKSTOFFS
UTILISATION IN VIVO DE CHONDROÏTINASE ET/OU D'HYALURONIDASE POUR AMÉLIORER L'ADMINISTRATION D'UN AGENT

(30) Priority: 29.04.2016 US 201662329593 P; 13.05.2016 US 201662336501 P; 21.04.2017 US 201762488605 P
(43) Date of publication of application: 06.03.2019
(73) Proprietor: Inovio Pharmaceuticals, Inc., Plymouth Meeting, PA 19462 (US)
(72) Inventor: SMITH, Trevor, Plymouth Meeting, PA 19462 (US); SCHOMMER, Nina, Plymouth Meeting, PA 19462 (US); BRODERICK, Kate, Plymouth Meeting, PA 19462 (US); YUNG, Bryan, Plymouth Meeting, PA 19462 (US); SCHULTHEIS, Katherine, Plymouth Meeting, PA 19462 (US)
(74) Representative: Leonard, Thomas Charles
(86) International application number: PCT/US2017/030447
(87) International publication number: WO 2017/190147

(56) References cited:
- EP-A1- 2 428 229
- WO-A2-2004/016761
- US-A1- 2006 104 968
- US-A1- 2011 066 111
- WILEMS THOMAS S ET AL: "Sustained dual drug delivery of anti-inhibitory molecules for treatment of spinal cord injury", JOURNAL OF CONTROLLED RELEASE, ELSEVIER, AMSTERDAM, NL, vol. 213, 27 June 2015 (2015-06-27), pages 103 - 111, XP029259353, ISSN: 0168-3659, DOI: 10.1016/J.JCONREL.2015.06.031
- ZHANG YU ET AL: "Combination of chondroitinase ABC, glial cell line-derived neurotrophic factor and Nogo A antibody delayed-release microspheres promotes the functional recovery of spinal cord injury.", THE JOURNAL OF CRANIOFACIAL SURGERY NOV 2013, vol. 24, no. 6, November 2013 (2013-11-01), pages 2153 - 2157, XP009516626, ISSN: 1536-3732
- FLINGAI SELEEKE ET AL: "Protection against dengue disease by synthetic nucleic acid antibody prophylaxis/immunotherapy", SCIENTIFIC REPORTS,, vol. 5, 29 July 2015 (2015-07-29), pages 12616 - 1, XP008179057, ISSN: 2045-2322
- R. K. BATRA ET AL: "Retroviral Gene Transfer Is Inhibited by Chondroitin Sulfate Proteoglycans/Glycosaminoglycans in Malignant Pleural Effusions", JOURNAL OF BIOLOGICAL CHEMISTRY, vol. 272, no. 18, 2 May 1997 (1997-05-02), pages 11736 - 11743, XP055077848, ISSN: 0021-9258, DOI: 10.1074/jbc.272.18.11736
- DATABASE MEDLINE [online] US NATIONAL LIBRARY OF MEDICINE (NLM), BETHESDA, MD, US; 2011, YAMAZAKI TATSUYA ET AL: "Passive immune-prophylaxis against influenza virus infection by the expression of neutralizing anti-hemagglutinin monoclonal antibodies from plasmids.", XP002797990, Database accession no. NLM21266754
- VANDERMEULEN ET AL: "Optimisation of intradermal DNA electrotransfer for immunisation", JOURNAL OF CONTROLLED RELEASE, ELSEVIER, AMSTERDAM, NL, vol. 124, no. 1-2, 14 November 2007 (2007-11-14), pages 81 - 87, XP022343691, ISSN: 0168-3659, DOI: 10.1016/J.JCONREL.2007.08.010
- YAMAZAKI TATSUYA ET AL: "Passive immune-prophylaxis against influenza virus infection by the expression of neutralizing anti-hemagglutinin monoclonal antibodies from plasmids", MEDLINE, US NATIONAL LIBRARY OF MEDICINE (NLM), BETHESDA, MD, US, 1 January 2011 (2011-01-01), XP002797990
- XIA ET AL.: "Antisense vimentin cDNA combined with chondroitinase ABC promotes axon regeneration and functional recovery following spinal cord injury in rats", NEUROSCI LETT., vol. 590, 2015, pages 74 - 79, XP055439552
- LIN ET AL.: "Chondroitinase ABC has a long-lasting effect on chondroitin sulphate glycosaminoglycan content in the injured rat brain", J NEUROCHEM., vol. 104, no. 2, 2008, pages 400 - 408, XP002546067
- ZHAO ET AL.: "Combination treatment with anti-Nogo-A and chondroitinase ABC is more effective than single treatments at enhancing functional recovery after spinal cord injury", EUR J NEUROSCI., vol. 38, no. 6, 2013, pages 2946 - 61, XP055439602
- YAZDI ET AL.: "Antibody-mediated inhibition of Nogo-A signaling promotes neurite growth in PC-12 cells", J TISSUE ENG., vol. 7, 28 January 2016 (2016-01-28), XP055439603
- DMITRIEVA ET AL.: "Chondroitinase ABC I-mediated enhancement of oncolytic virus spread and antitumor efficacy", CLIN CANCER RES., vol. 17, no. 6, 2011, pages 1362 - 1372, XP055439606
- KIM ET AL.: "A novel single-chain antibody redirects adenovirus to IL 13R alpha 2-expressing brain tumors", SCI REP., vol. 5, 2015, pages 18133, XP055439608

## Description

### CROSS REFERENCE TO RELATED APPLICATIONS

This application claims priority to U.S. Provisional Application No. 62/329,593 filed on April 29, 2016, U.S. Provisional Application No. 62/336,501, filed on May 13, 2016, and U.S. Provisional Application No. 62/488,605, filed on April 21, 2017.

### FIELD

This invention relates to a DNA plasmid encoding a monoclonal antibody for use in a method of treating a disease or disorder in a subject, in combination with chondroitinase AC or chondroitinase ABC, the method comprising:
administering to the subject chondroitinase AC or chondroitinase ABC prior to or concurrently with the DNA plasmid encoding a monoclonal antibody, and
administering the DNA plasmid via electroporation.

### INTRODUCTION

Glycosaminoglycans (GAGs) are complex linear polysaccharides of the extracellular matrix (ECM). GAGs are characterized by repeating disaccharide structures of an N-substituted hexosamine and an uronic acid (in, e.g., hyaluronan (HA), chondroitin sulfate (CS), chondroitin (C), dermatan sulfate (DS), heparan sulfate (HS), heparin (H)) or a galactose (in, e.g., keratan sulfate (KS)). Except for hyaluronan, all exist covalently bound to core proteins. The GAGs with their core proteins are structurally referred to as proteoglycans (PGs).

Chondroitin sulfate proteoglycans (CSPGs) are major components of extracellular matrices and have diverse functional roles. For example, CSPGs are generally secreted from cells and are structural components of a variety of human tissues, including cartilage, and are known to be involved in certain cell processes, such as cell adhesion, cell growth, receptor binding, cell migration, and interaction with other extracellular matrix constituents. These other extracellular matrix constituents may be laminin, fibronectin, tenascin, and/or collagen. Hyaluronan is also one of the main components of the extracellular matrix, especially in soft connective tissues. In connective tissue, the water of hydration associated with hyaluronan creates spaces between tissues, thus creating an environment conducive to cell movement and proliferation. Hyaluronan plays a key role in biological phenomena associated with cell motility including rapid development, regeneration, repair, embryogenesis, embryological development, wound healing, angiogenesis, cell regulation, cell development, cellular differentiation, and cell migration. Hyaluronan production increases in proliferating cells and may have a role in tumorigenesis.

FLINGAI SELEEKE et al., SCIENTIFIC REPORTS, vol. 5, 12616 (2015) relates to protection against dengue disease by synthetic nucleic acid antibody prophylaxis/immunotherapy.

XIA YONGZHI et al., NEUROSCI LETT, vol. 590, 74-79 (2015) relates to antisense vimentin cDNA combined with chondroitinase ABC which may promote axon regeneration and functional recovery following spinal cord injury in rats.

The extracellular matrix includes proteins and polysaccharide molecules, assembled in a dense, organized network in the extracellular space of most tissues. As one of the main components of the extracellular matrix, CSPGs and hyaluronan exert influence on the characteristics of the extracellular matrix by means of their viscous solution forming properties. There remains a need in the art for a means to traverse tissues and extracellular matrices to deliver an agent to a subject in a safe, cost effective, and efficient manner.

### SUMMARY

The invention is defined in the appended set of claims. The description may contain additional disclosures of technical information, which although not part of the claimed invention, is provided to place the invention in a broader technical context and to illustrate possible related technical developments. The references to methods of treatment in the subsequent paragraphs of this description are to be interpreted as references to the compounds, pharmaceutical compositions and medicaments of the present invention for use in those methods.
Aspects of the invention include agents for use in methods of delivering said agents to a subject, wherein the methods comprise administering to the subject a chondroitinase polypeptide or a polynucleotide encoding a chondroitinase polypeptide in an amount sufficient to degrade a chondroitin sulfate proteoglycan (CSPG), and administering the agent to the subject. The CSPG may be, for example, Aggrecan (CSPG1), Versican (CSPG2), Neurocan (CSPG3), CSPG4 (melanoma-associated chondroitin sulfate proteoglycan, NG2), CSPG5, SMC3 (CSPG6, Structural maintenance of chromosome 3), Brevican (CSPG7), CD44 (CSPG8, cluster of differentiation 44), Phosphacan, or combinations thereof. Specifically, the invention is a DNA plasmid encoding a monoclonal antibody for use in a method of treating a disease or disorder in a subject, in combination with chondroitinase AC or chondroitinase ABC, the method comprising:
administering to the subject chondroitinase AC or chondroitinase ABC prior to or concurrently with the DNA plasmid encoding a monoclonal antibody, and
administering the DNA plasmid via electroporation.
The agent may illicit an immune response or enhance an immune response in a subject.

Other aspects of the invention include agents for use in methods of treating a disease or disorder in a subject, wherein the methods comprise administering to the subject a chondroitinase polypeptide or a polynucleotide encoding a chondroitinase polypeptide; and administering to the subject the agent.

In any of the methods of the disclosure described herein, the agent may be a polynucleotide, a polypeptide, a small molecule, or a combination thereof, for example. In the invention, the agent is a DNA plasmid. The DNA plasmid encodes a monoclonal antibody. As part of the disclosure, the agent may comprise a polypeptide. The polypeptide may comprise a monoclonal antibody. The monoclonal antibody may be expressed in vivo. In the invention, the agent is administered to the subject via electroporation. In the invention, the chondroitinase is chondroitinase AC or chondroitinase ABC.

The chondroitinase polypeptide and the monoclonal antibody may be encoded by the same polynucleotide or separate polynucleotides. The polynucleotide encoding the chondroitinase polypeptide and the polynucleotide encoding the monoclonal antibody may be comprised within the same vector or separate vectors.

In any of the herein described methods, the chondroitinase polypeptide or the polynucleotide encoding the chondroitinase polypeptide may be administered to the subject prior to administration of the agent. The chondroitinase polypeptide or the polynucleotide encoding the chondroitinase polypeptide may be administered to the subject at least about 15 minutes to about 24 hours prior to administration of the agent. The chondroitinase polypeptide or the polynucleotide encoding the chondroitinase polypeptide, and the agent may be administered to the subject concurrently. The chondroitinase polypeptide or the polynucleotide encoding the chondroitinase polypeptide, and the agent may be administered to the subject subcutaneously or intramuscularly. The chondroitinase polypeptide or the chondroitinase polypeptide encoded by the polynucleotide hydrolyzes CSPG and leads to disorganization of an extracellular matrix of the subject.

Other aspects of the invention include also administering a hyaluronidase polypeptide or a polynucleotide encoding a hyaluronidase polypeptide in any of the herein described methods in an amount sufficient to degrade a glycosaminoglycan. The glycosaminoglycan may comprise hyaluronan. The hyaluronidase polypeptide or the polynucleotide encoding the hyaluronidase polypeptide may be administered at the same time as the chondroitinase. The hyaluronidase polypeptide or the polynucleotide encoding the hyaluronidase polypeptide may be administered to the subject prior to administration of the agent. The hyaluronidase polypeptide or the polynucleotide encoding the hyaluronidase polypeptide may be administered to the subject at least about 15 minutes to about 24 hours prior to administration of the agent. The hyaluronidase polypeptide or the polynucleotide encoding the hyaluronidase polypeptide, and the agent may be administered to the subject concurrently. The hyaluronidase polypeptide or the polynucleotide encoding the hyaluronidase polypeptide, and the agent may be administered to the subject subcutaneously or intramuscularly.

The chondroitinase polypeptide or the polynucleotide encoding the chondroitinase polypeptide, and the agent may be co-formulated prior to administration. The chondroitinase polypeptide or the polynucleotide encoding the chondroitinase polypeptide, the hyaluronidase polypeptide or a polynucleotide encoding a hyaluronidase polypeptide, and the agent may be co-formulated prior to administration.

The disclosure provides for other aspects and embodiments that will be apparent in light of the following detailed description and accompanying Figures.

### BRIEF DESCRIPTION OF THE DRAWINGS

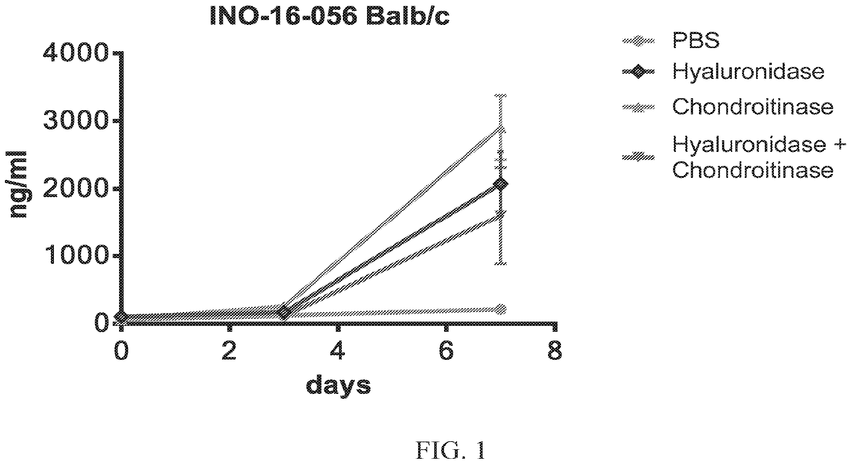

**Figure 1** shows the levels (ng/ml) of hIgG measured by ELISA between days 0-7 in groups 1 to 4 (Balb/c mice, 6-7 weeks). Group 1 (grey) PBS and pGX9214, group 2 hyaluronidase pretreatment and pGX9214 (blue), group 3 chondroitinase and pGX9214 (green), group 4 hyaluronidase/ chondroitinase pretreatment and pGX9214 (brown).

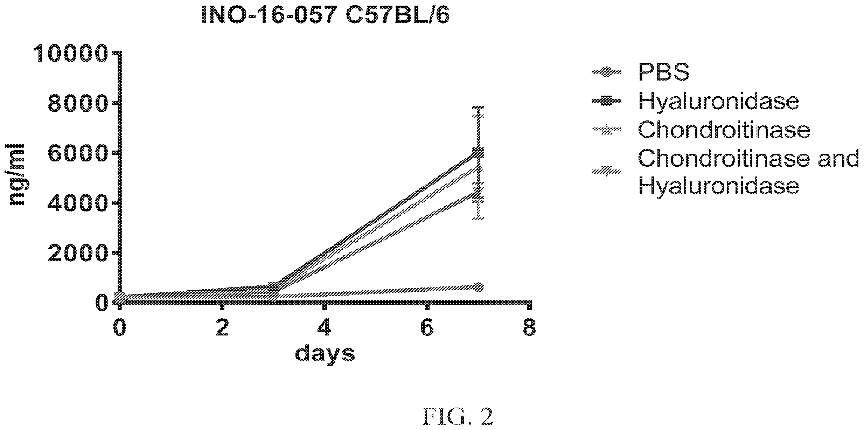

**Figure 2** shows levels (ng/ml) of hIgG measured by ELISA between days 0-7 in groups 1 to 4 (C57BL/6, 6-7 weeks). Group 1 (grey) PBS and pGX9214, group 2 hyaluronidase pretreatment and pGX9214 (blue), group 3 chondroitinase and pGX9214 (green), group 4 hyaluronidase/ chondroitinase pretreatment and pGX9214 (brown).

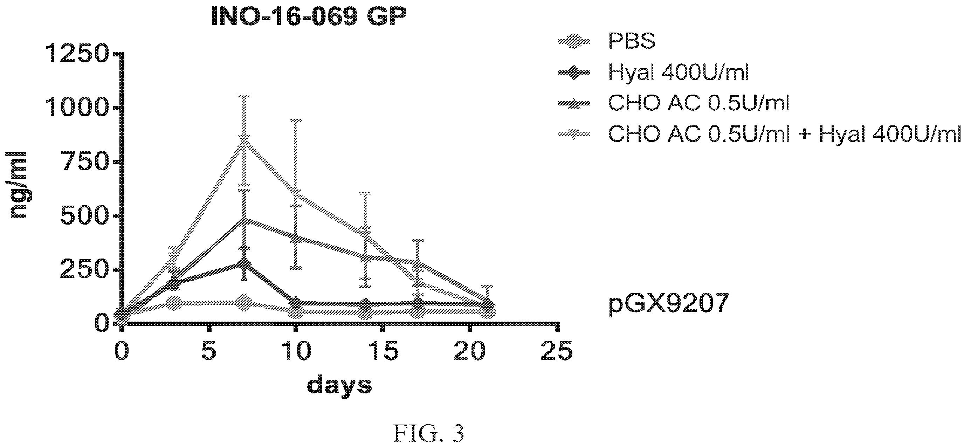

**Figure 3** shows levels (ng/ml) of hIgG measured by ELISA between days 0-21 in groups 1 to 4. Group 1 (grey) PBS and pGX9214, group 2 hyaluronidase pretreatment and pGX9214 (dark red), group 3 chondroitinase and pGX9214 (bright red), group 4 hyaluronidase/ chondroitinase pretreatment and pGX9214 (rose - Hartley Guinea pig).

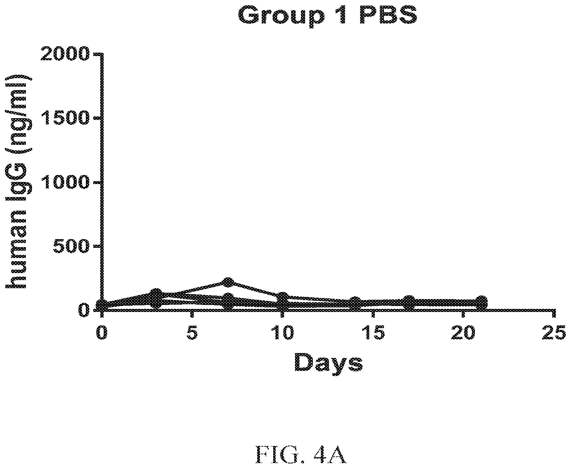

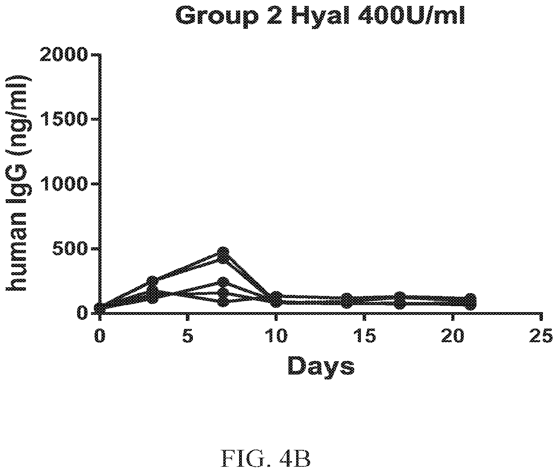

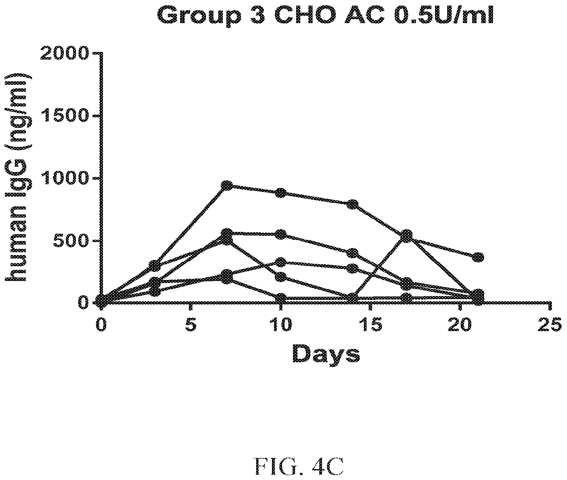

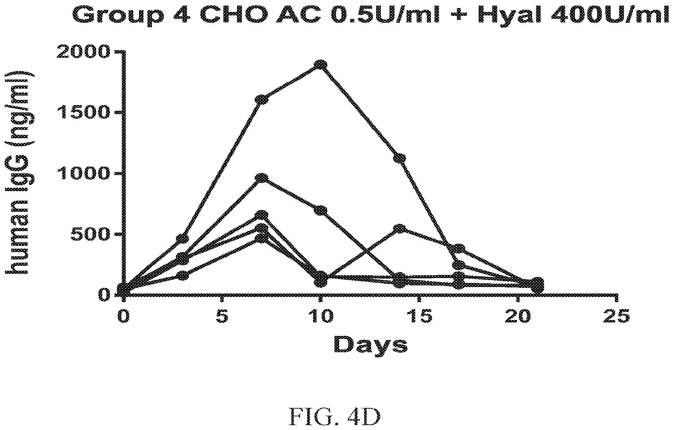

**Figure 4** shows levels (ng/ml) of hIgG measured by ELISA between days 0-21 in individual guinea pigs in groups 1 to 4. Figure 4A: Group 1 PBS. Figure 4B: group 2 hyaluronidase pretreatment with 400U/ml. Figure 4C: group 3 chondroitinase (0.5U/ml). Figure 4D: group 4 hyaluronidase 400U/ml and chondroitinase 0.5U/ml pretreatment.

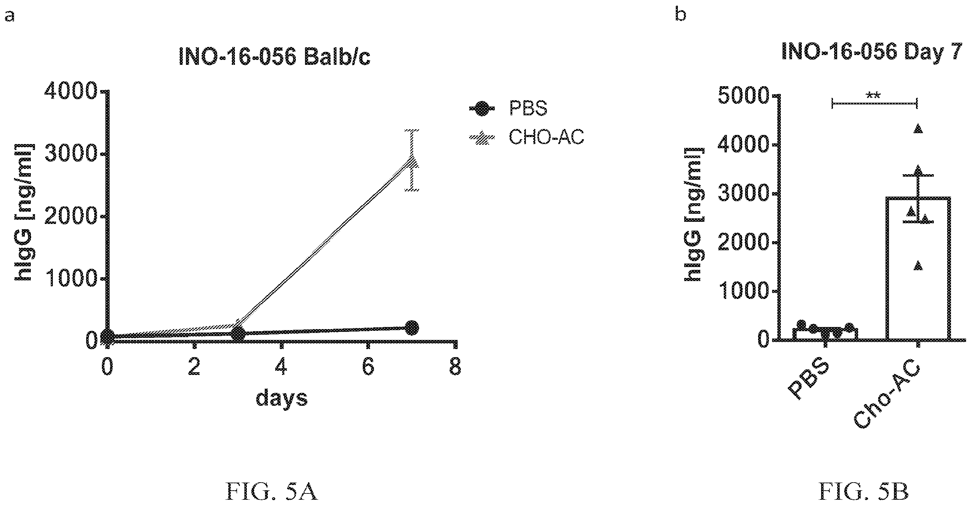

**Figure 5** shows chondroitinase enhances plasmid-encoded hlgG expression in Balb/c mice. (a) The levels of hIgG [ng/ml] measured by ELISA between days 0-7 in groups 1 and 2 (Balb/c mice, 6-7 weeks). Group 1 (grey) PBS and pGX9214, group 2 chondroitinase pretreatment and pGX9214 (black). (b) Significant enhancement of hIgG by chondroitinase at day 7. Statistics performed by Mann Whitney test, P = 0.0079.

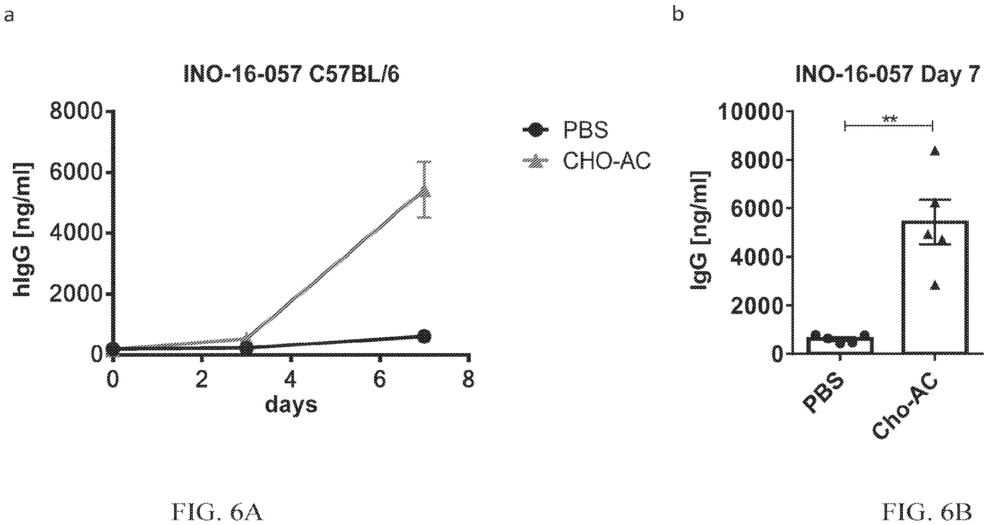

**Figure 6** shows chondroitinase enhances plasmid-encoded hIgG expression in C57BL/6 mice. (a) The levels of hIgG [ng/ml] measured by ELISA between days 0-7 in groups 1 and 2 (Balb/c mice, 6-7 weeks). Group 1 (grey) PBS and pGX9214, group 2 chondroitinase pretreatment and pGX9214 (black). (b) Significant enhancement of hIgG by chondroitinase at day 7. Statistics performed by Mann Whitney test, P = 0.0079.

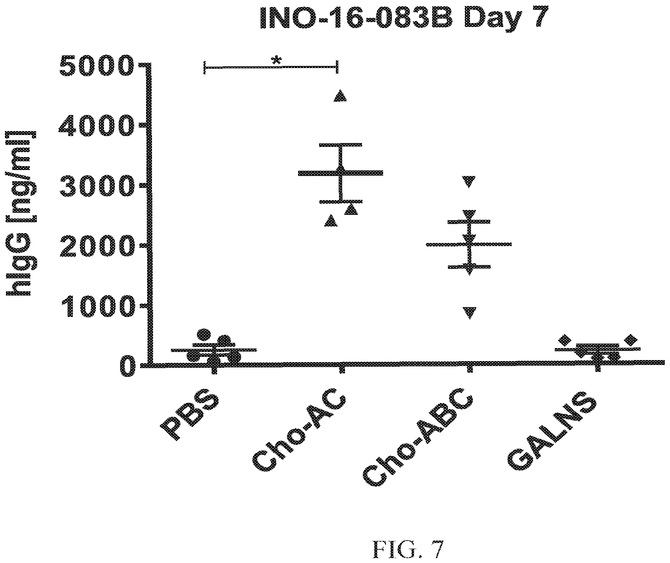

**Figure 7** shows an investigation of the ability of different versions of chondroitinase to enhance DMAb expression. Graph represents hIgG levels (ng/ ml) measured by ELISA at day 7 in groups 1-4 (Balb/c). Mice were treated with either treated with PBS (control group 1), Chondroitinase AC, clinical grade Chondroitinase ABC or the recombinant protein GALNS. All groups received an injection of pGX9203 followed by electroporation. Statistics performed by Kruskal-Wallis test, **P = 0.0026.

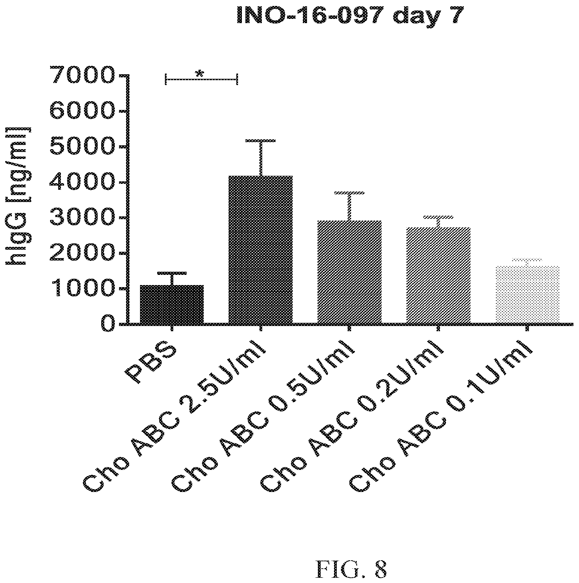

**Figure 8** shows increasing Chondroitinase ABC dose further enhances DNA-based protein expression. Balb/c mice were treated with increasing doses of Chondroitinase ABC 30 minutes prior pDNA (pGX9207) injection and electroporation. Levels of hIgG were measured by ELISA. Statistics performed by Kruskal-Wallis test, *P = 0.0357.

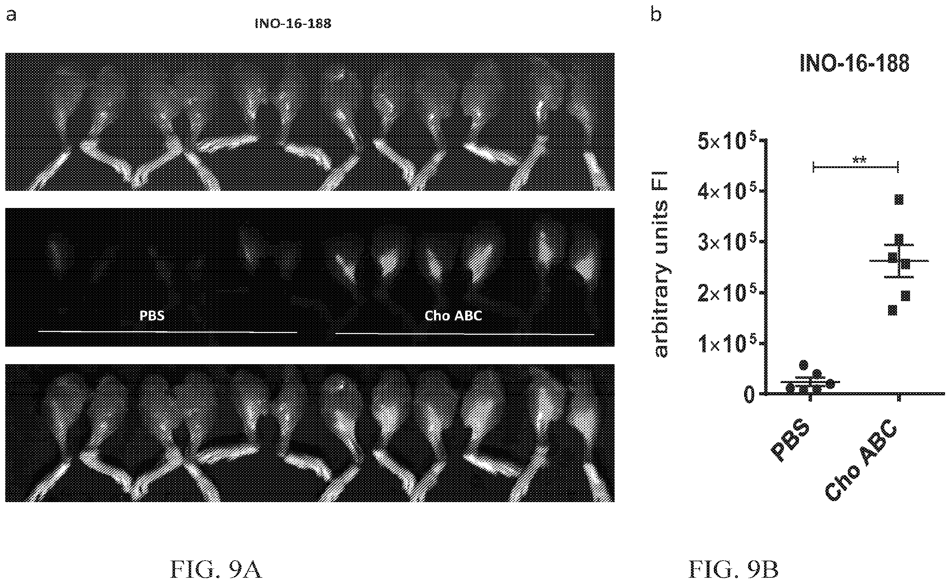

**Figure 9** shows chondroitinase administration results in enhanced fluorescent protein expression. (a) Left and right hindlimbs of Balb/c mice treated with either Chondroitinase or PBS into the skeletal muscle. Visualization of the reporter protein expression was performed by a fluorescence imager (Protein Simple). (b) Arbitrary units fluorescence intensity parallel reporter gene expression and were quantified by using AlphaView SA software. Statistics performed by Mann Whitney test, P = 0.0022.

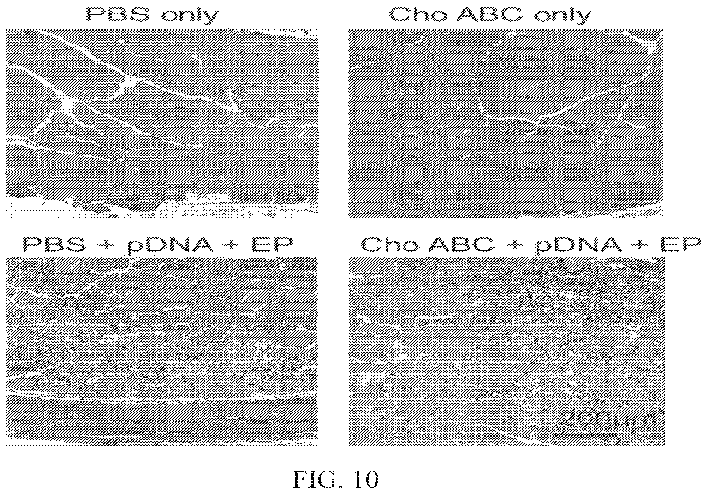

**Figure 10** shows representative histopathology of murine hindlimb skeletal muscle performed by H&E staining. Top panels show tissue treated with either PBS only (control) or Chondroitinase ABC only. Pretreated (30 min) muscles with either chondroitinase or PBS (control) before pDNA delivery are presented in the bottom figures. Results were analyzed by a slide scanner and CaseViewer software (3DHISTECH). Scale = 200 µm.

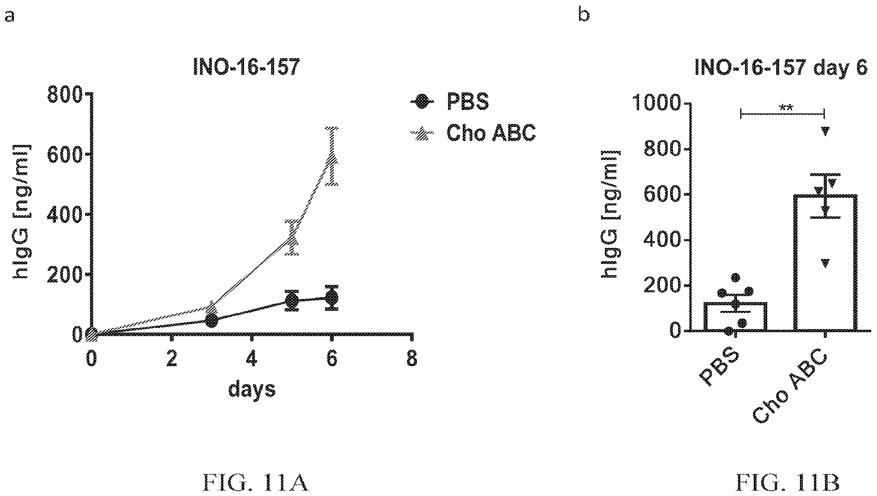

**Figure 11** shows chondroitinase ABC enhances plasmid-encoded hIgG expression in New Zealand rabbits (9 weeks). (a) Presented are the levels of hIgG [ng/ml] measured by ELISA between days 0 and 6 in group 2 (chondroitinase-pretreated, grey) and in group 1 (PBS control, black). (b) Graph shows hIgG levels from day 6 measured by ELISA. Statistics performed by Mann Whitney test, P = 0.0043.

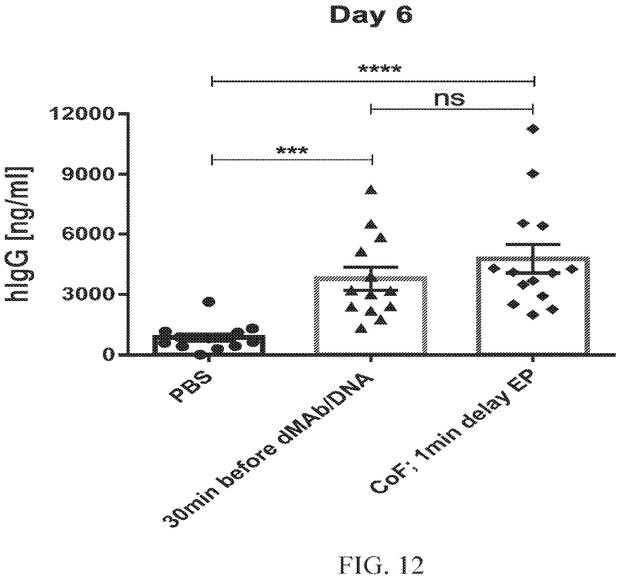

**Figure 12** shows co-formulation of chondroitinase with pDNA (pGX9207) in mice (Balb/c; 14 mice per group). Graph shows hIgG levels [ng/ml] from day 6 measured by ELISA. Statistics performed by Mann Whitney test, ****P < 0.0001.

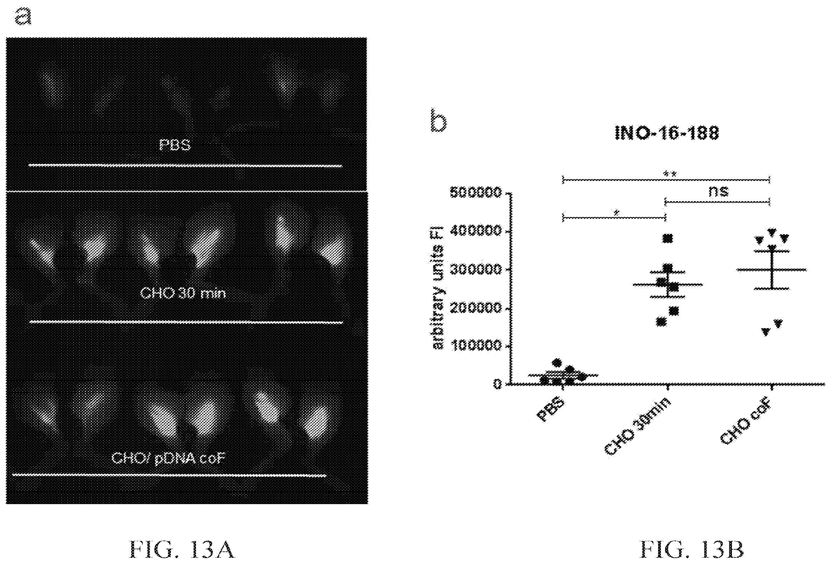

**Figure 13** shows chondroitinase administration results in enhanced fluorescent protein expression. (a) Left and right hindlimbs of Balb/c mice treated with either Chondroitinase or PBS into the skeletal muscle. Visualization of the reporter protein expression was performed by a fluorescence imaging system. (b) Arbitrary units fluorescence intensity parallel reporter gene expression and were quantified by using AlphaView SA software. Statistics performed by Mann Whitney test, **P = 0.0004.

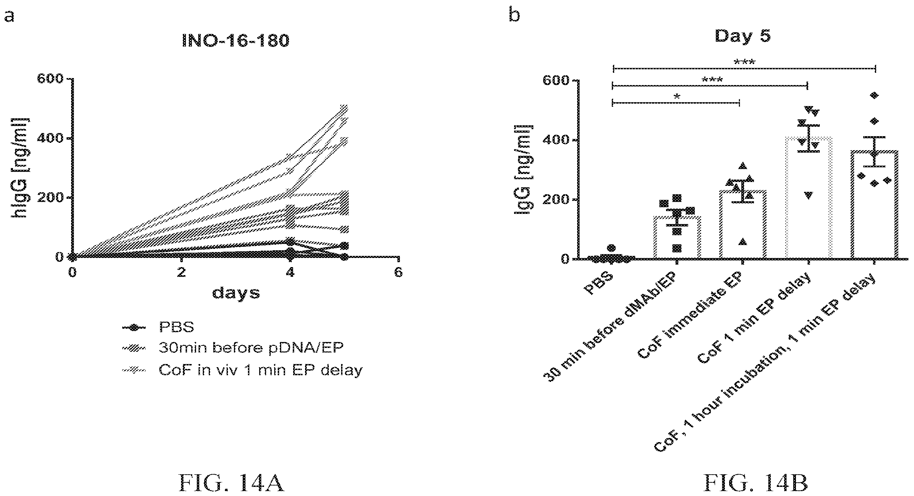

**Figure 14** shows co-formulation of chondroitinase with pDNA (pGX9207) in rabbits (New Zealand rabbits; 6 rabbits per group). (a) Graph shows serum hIgG levels [ng/ml] from day 0 to day 5 measured by ELISA. Comparison of groups 1, 2 and 4. (b) Rabbit serum hIgG levels of all groups measured at day 5. Statistics performed by Mann Whitney test, ****P < 0.0001.

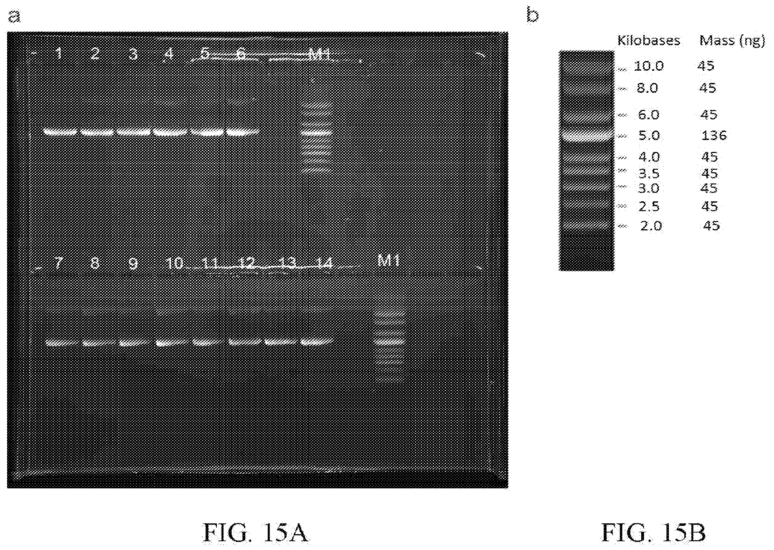

**Figure 15** shows agarose gel electrophoresis of chondroitinase (2.5 U/ ml)/ pDNA (pGX9207, 250 ng per well) coformulated samples. (a) Lanes with even numbers present pDNA samples containing Chondroitinase ABC, lanes with odd numbers refer to PBS negative controls. Lanes 1-2: No incubation of the sample before gel electrophoresis, lanes 3-4: incubation at RT (21⁰C) for 10 min, lanes 5-6: 6°C, 120 min, lanes 7-8: RT, 120 min, lanes 9-10: 6°C, 24 hrs, Lanes 11-12: RT, 24 hrs, lanes 13-14: 6°C, 10 min. M1 = marker. (b) Ladder for supercoiled DNA, 2-10 kb (New England Biolabs).

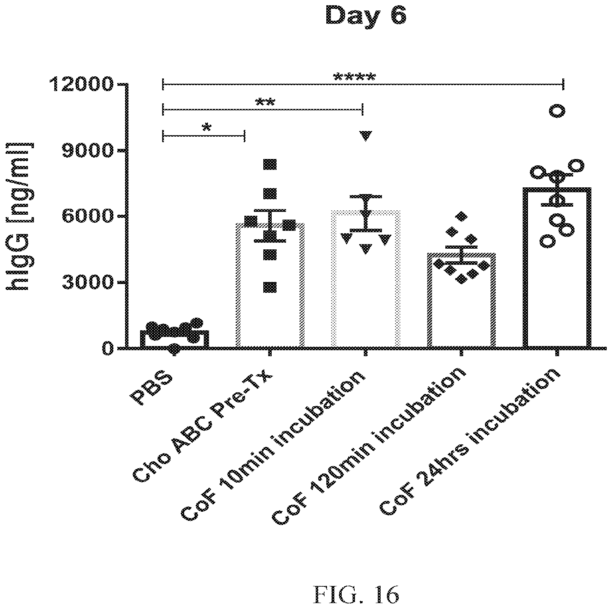

**Figure 16** shows storage of chondroitinase/ pDNA coformulation for 24 hours does not affect enhanced gene expression in Balb/c mice. Graph represents serum hIgG levels (ng/ ml) measured by ELISA at day 6. Mice were treated with coformulations into the left hindlimb skeletal muscle and electroporation was performed after 1 min of drug injection. Prior to treatments, coformulation samples were incubated at 10 min, 120 min and 24 hours at 4 ⁰C. Controls: Mice were pretreated with either chondroitinase or PBS 30 min before pDNA administration and electroporation. Statistics performed by Kruskal-Wallis test, ****P = 0.0001.

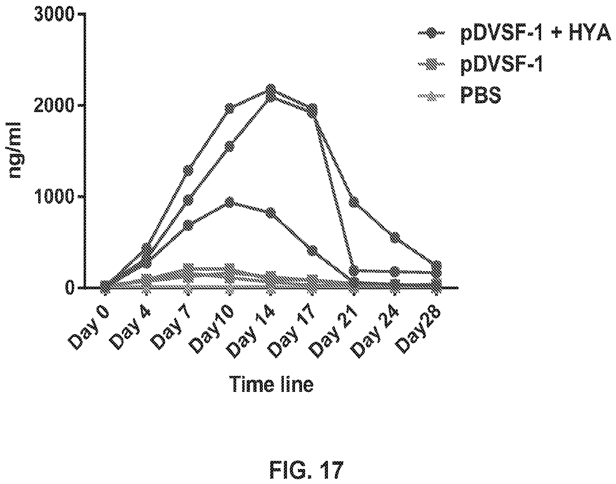

**Figure 17** shows the levels (ng/ml of hIgG measured by ELISA between days 0-28 in groups 1, 2 and 3. Group 1 was treated with pDVSF-1 and hyaluronidase pretreatment, Group 2 was treated with pDVSF only, and Group 3 was treated with PBS only.

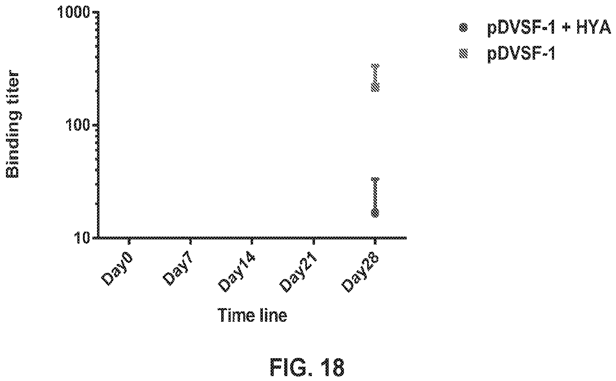

**Figure 18** shows the anti-hIgG binding titers of groups 1 and 2.

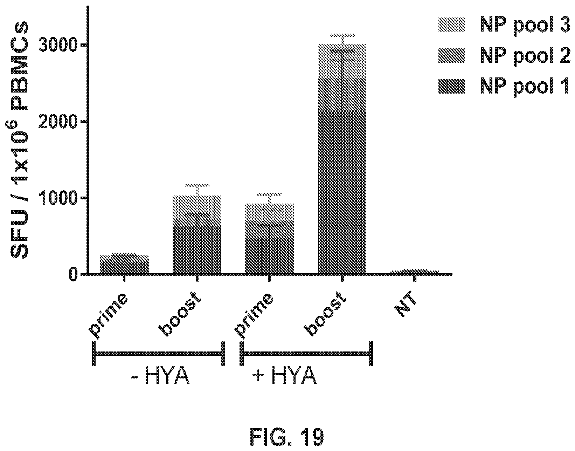

**Figure 19** shows the mean (+/- SEM) IFN-y (spots per million) response in PBMCs of groups 1-3 to the Influenza NP peptide pools in Example 2.

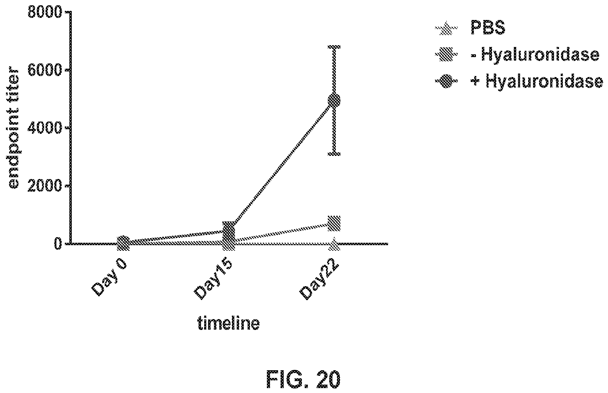

**Figure 20** shows the anti-Influenza NP IgG binding titers for groups 1-3 in Example 2.

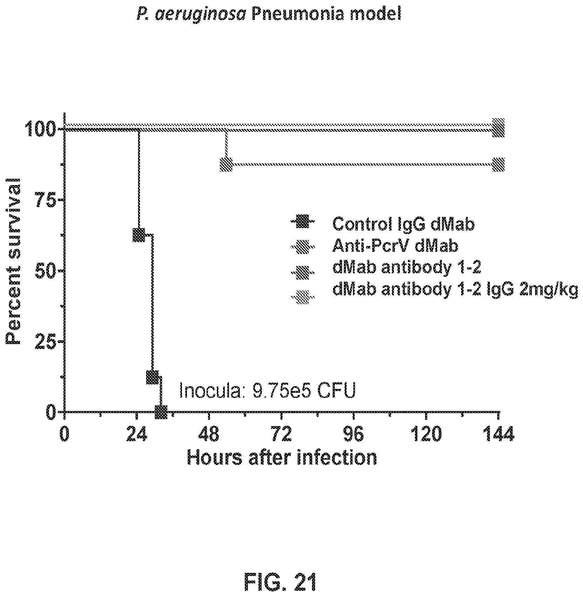

**Figure 21** shows the *P. aeruginosa* acute pneumonia model. Electroporation of anti-PcrV or DMAb-antibody 1-2 plasmid DNA yields expression of active IgG in mice. Potent protective activity observed for both anti-Pseudomonal DMAbs and DMAb-antibody 1-2 IgG.

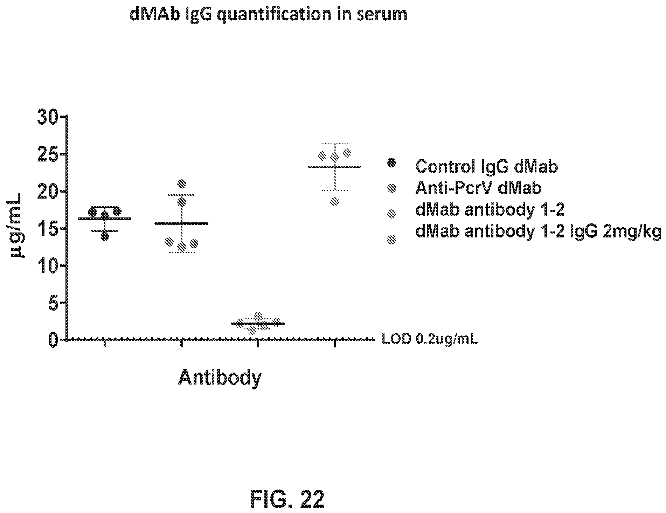

**Figure 22** shows IgG quantification of DMAb expression in serum. Serum was evaluated for DMAb expression prior to P. aeruginosa infection. Despite similar survival profiles, anti-PcrV DMAb expression was 5-fold greater than DMAb-antibody1-2.

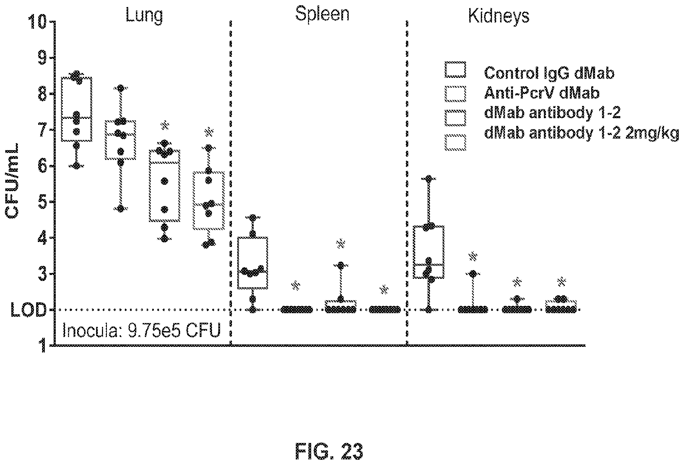

**Figure 23** shows the reduction of organ burden by anti-Pseudomonal DMAbs. DMAb-antibody1-2 and IgG reduces burden in the lung. Anti-PcrV and DMAb-antibody1-2 significantly reduce systemic spread of bacteria. Tissues collected 24 hour post-infection. LOD = limit of detection; * = P<0.05 by Kruskal-Wallis and Dunn's multiple comparison test vs. control IgG DMAb.

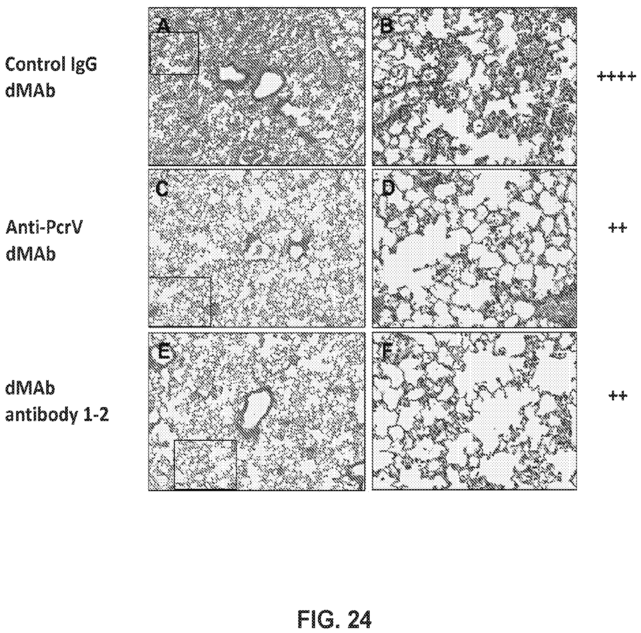

**Figure 24** shows the histology of acute pneumonia at 48 hours post-infection (H&E). A. Control IgG lungs exhibit areas of severe alveolar infiltrates comprised of neutrophils and macrophages and hemorrhage (10x). C. Mild pneumonia and occasional bronchiolar debris (10x). E. DMAb-antibody1-2 DNA group with mild alveolitis (10x). B, D, F. Insets at 40x from A. C. D., respectively.

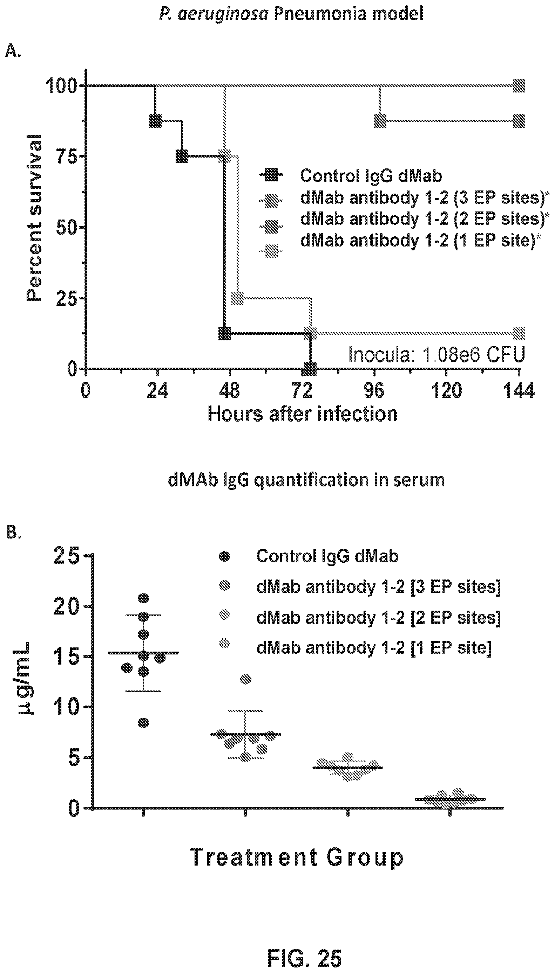

**Figure 25** shows that DMAb-antibodyl-2 exhibits concentration dependent protective activity. A. Animals were received DNA at 1, 2 or 3 sites prior to EP. B. Quantification of serum IgG. * indicates P<0.05 via Log-Rank test.

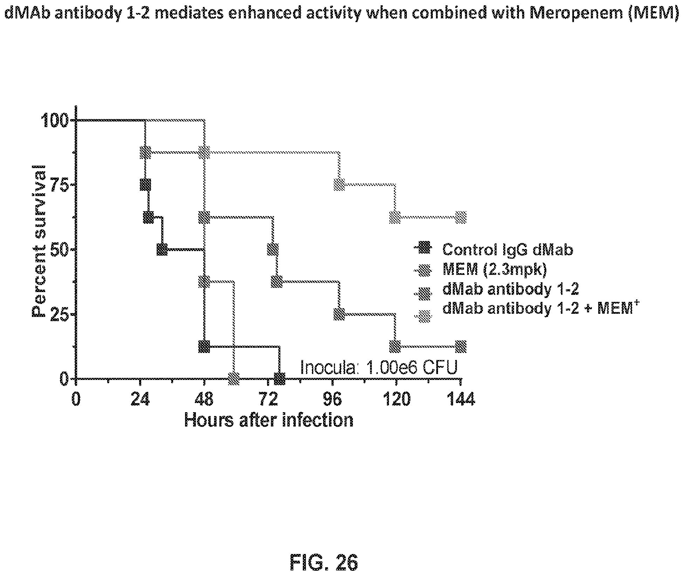

**Figure 26** shows subtherapeutic dosages of DMAb-antibody1-2 and meropenem (MEM) exhibit enhanced activity against P. aeruginosa pneumonia. Animals were received DNA at 1, 2 or 3 sites prior to EP. B. Quantification of serum IgG. * indicates P<0.05 via Log-Rank test.

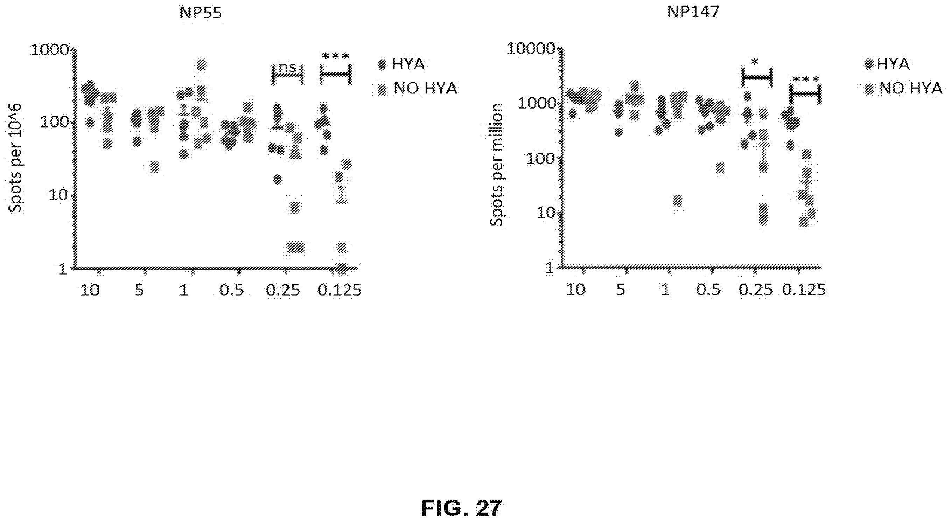

**Figure 27** shows the mean (+/- SEM) IFN-γ (spots per million) response in splenocytes to NP55 and NP147 peptide epitopes 14 days after pGX2013 immunization.

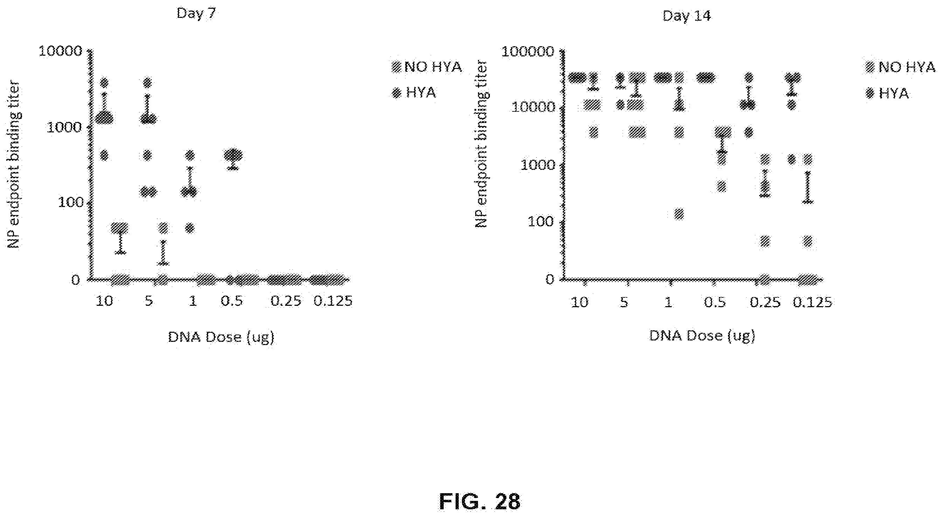

**Figure 28** shows the anti-NP IgG binding titers for groups 7 and 14 days after immunization with pGX2013.

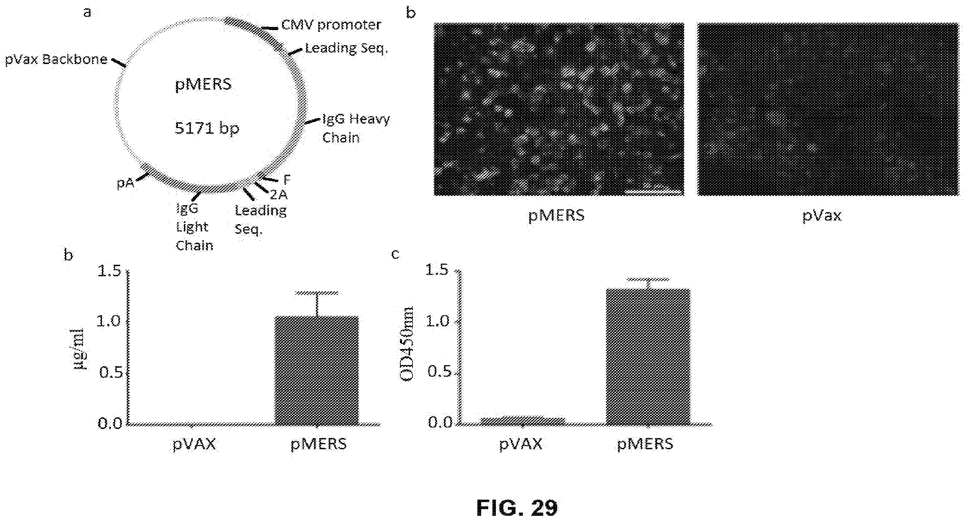

**Figure 29** shows in vitro expression of anti-MERS-CoV human IgG. (a) Diagrammatic illustration of the anti-MERS-CoV antigen DMAb plasmid DNA pMERS. CMV promoter situated upstream of the antibody heavy and light chain sequences separated by furin and 2A cleavage sites. (b & c) 293T cell cultures were transfected 1 µg/ml of pVax or pMERS and culture supernatants harvested after 48 hours. (b) human IgG levels in the supernatant were assayed for by ELISA. (c) IgG binding to MERS CoV antigen was measured by ELISA.

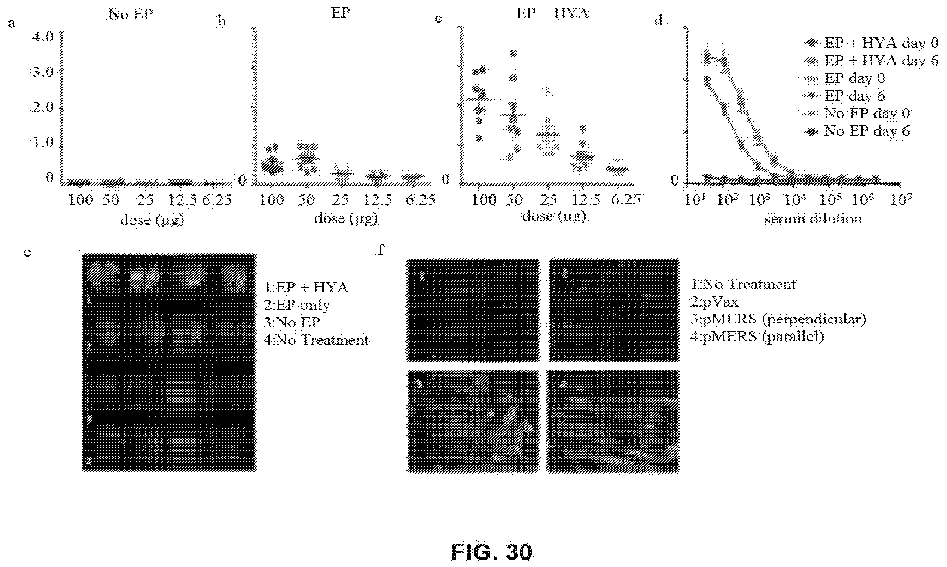

**Figure 30** shows enhanced in vivo expression of DMAb in BALB/c mice
   6.25 to 100 µg of pMERS was administered into the TA muscle of BALB/c mice (4-8 mice per group), (a) by injection only (No EP), (b) injection with EP (EP) and (c) injection with EP into HYA-treated muscle (EP + HYA). (a-c) Serum human IgG levels were quantified (data points represent the Mean+/-SEM) by ELISA on day 6 after pMERS delivery. (d) MERS CoV antigen binding of reciprocal serum dilutions measured by ELISA on day 0 and day 6 after pMERS delivery. (e) BALB/c mice TA muscles were harvested 72 hours after administration pRFP (25 µg) reporter gene with the protocol employed in (a), (b) or (c) or no treatment in inserts 1-4, respectively. Images illustrate reporter gene expression. Immunofluorescence images of sections of the TA muscle treated with pMERS or pVax (100 µg) delivered with EP + HYA, and harvested 72 hours later (f). hIgG was detected with anti-human IgG followed by a FITC-labelled secondary antibody (green). DAPI stain in blue. Panel 1. No treatment. Panel 2. pVax. Panels 3 & 4. pMERS. Panels 1-3 display a crosssectional image perpendicular to muscle fibers, and in Panel 4 the image is along the muscle fibers.

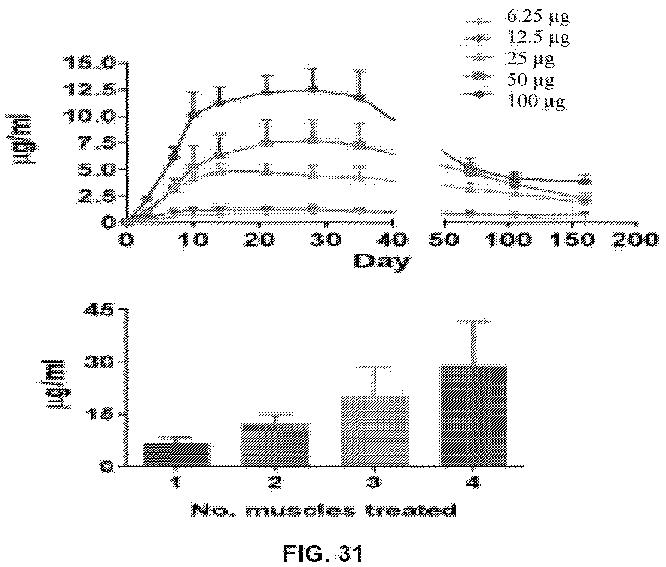

**Figure 31** shows increased and sustained DMAb expression in Crl:Nu-Foxn1nu mice. 6.25 to 100 µg of pMERS was administered with EP into the HYA pretreated TA muscle of Crl:Nu-Foxn1nu mice (8 mice per group). Serum hIgG was quantified by ELISA on days 0 to 160 (a). (b) 100 µg of pMERS was administered as in (a) to 1 (left TA), 2 (right and left TA), 3 (right and left TA, and left Quad) or 4 (right and left TA, and left and right Quad) muscle. Serum hIgG was quantified by ELISA on day 21.

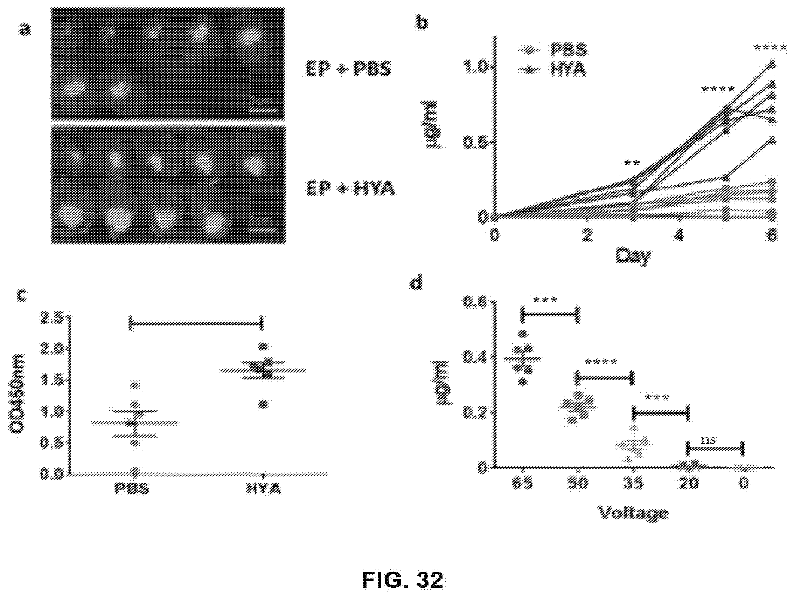

**Figure 32** shows in vivo DMAb expression in the New Zealand white rabbit. (a) Reporter gene expression in the rabbit TA muscle sections 72 hours after delivery of pGFP (0.2 mg) with EP in PBS- (top panel) or HYA- (bottom panel) treated muscle. (b&c) 2 mg of pMERS was administered with EP into the Quad muscle (pre-treated with PBS or HYA) of rabbits (6 per group). Serum hIgG was quantified on days 3, 5 and 6 (a), and MERS CoV antigen binding measured (b) by ELISA on day 6 after delivery. (c) 2 mg of pMERS was administered with EP at voltage setting of 20 to 65 V into the Quad muscle (treated with HYA) of rabbits (6 per group), and serum hIgG was quantified on day 5 after delivery. (c & d) Values are depicted as mean +/- SEM (n = 6/group). ****p < 0.0001, ***p < 0.001, **p < 0.01, and ns = non-significant. P values are from unpaired, two-tailed Mann-Whitney tests.

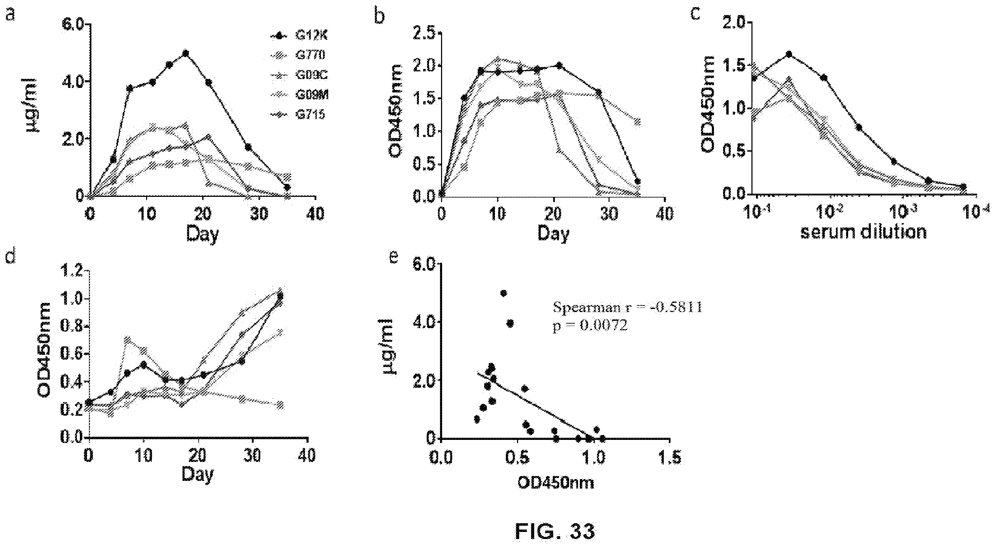

**Figure 33** shows In vivo DMAb expression in the rhesus macaques. 13.5 mg of pMERS was administered with EP into the quad muscles (pre-treated with HYA) of rhesus macaques (5 per group). Serum hIgG was quantified (a) and MERS CoV antigen binding measured by ELISA on days 0 to 35 (b), and day 17 reciprocal serum dilution (c) for each rhesus macaque. (d) Antibody response to human IgG (ADA) was measured by direct ELISA in the serum of rhesus macaques. (e) The correlation of DMAb levels and anti-human IgG antibody binding levels in the serum is depicted. Plotted are data points (hIgG (µg/ml) with corresponding ADA (OD450nm)) after and including the peak hIgG (µg/ml) value was reached in each Rhesus macaque. P value and Spearman correlation coefficient calculated using GraphPad Prism 6 software.

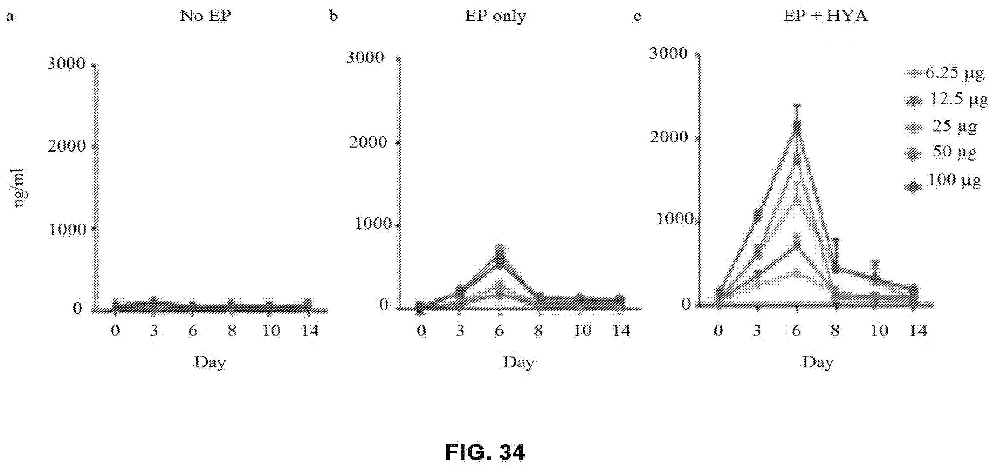

**Figure 34** shows In vivo expression kinetics of DMAb in BALB/c mice. (a-c) 6.25 to 100 µg of pMERS was administered into the TA muscle of BALB/c mice (4-8 mice per group), (a) by injection only (No EP), (b) injection with EP (EP) and (c) injection with EP into HYA-treated muscle (EP + HYA). (a-c) Serum human IgG levels were quantified (data points represent the Mean+/-SEM) by ELISA on day 0 to 14 after pMERS delivery.

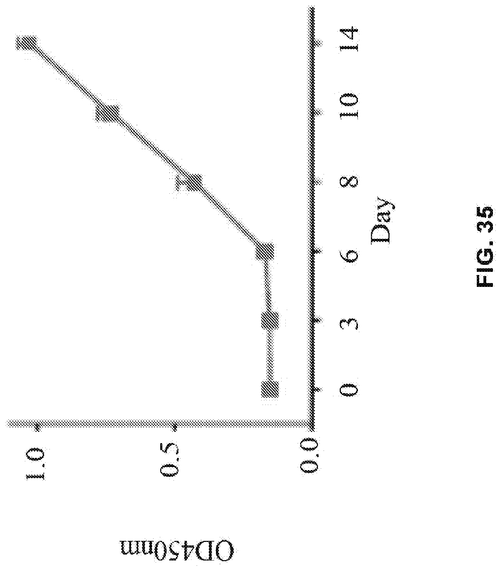

**Figure 35** shows anti-antibody response to human IgG in pMERS treated BALB/c mice. Antibody response to human IgG (ADA) was measured by direct ELISA in the serum of BALB/c mice on days 0 to 14 after pMERS delivery with EP.

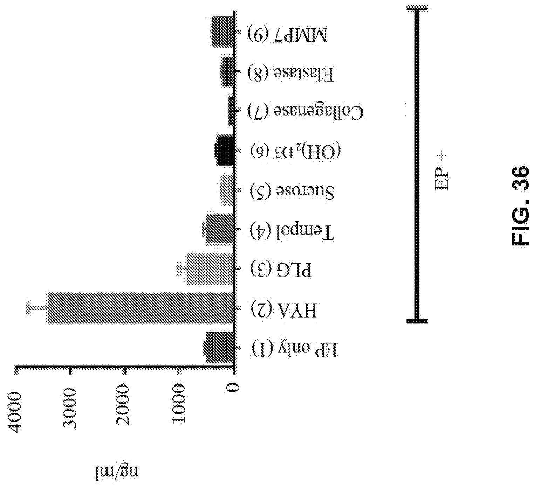

**Figure 36** shows screen of pDNA delivery reagents reported to enhance gene expression. 100 µg of pMERS was administered into the TA muscle of BALB/c mice (5 mice per group). The target TA muscle was pretreated 30 min before pMERS delivery with PBS, HYA, 7% Sucrose, Collagenase D, Elastase or MMP7 in groups 1, 2, 5, 7, 8, and 9 respectively. pMERS was coformulated with Poly-L-Glutamic acid (Group 3) or Tempol (Group 4) or (OH)3D3 (Group 6), and there was no pretreatment. Serum human IgG levels were quantified (data points represent the mean+/-SEM) by ELISA on day 6 after pMERS delivery.

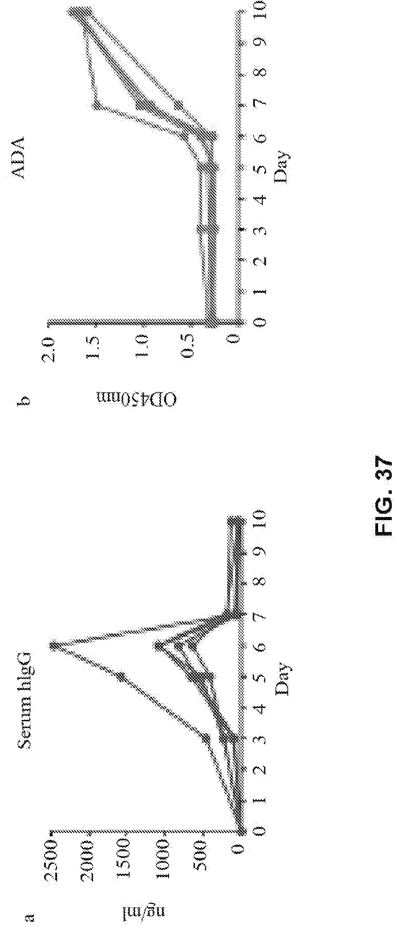

**Figure 37** shows DMAb expression in rabbits. (a and b) 2 mg of pGX9207 was administered with EP into the Quad muscle (pre-treated with HYA) of New Zealand white rabbits (6 per group). Serum hIgG was (a) and anti-human IgG (ADA) binding (b) was assayed by ELISA on days 0 to 10.

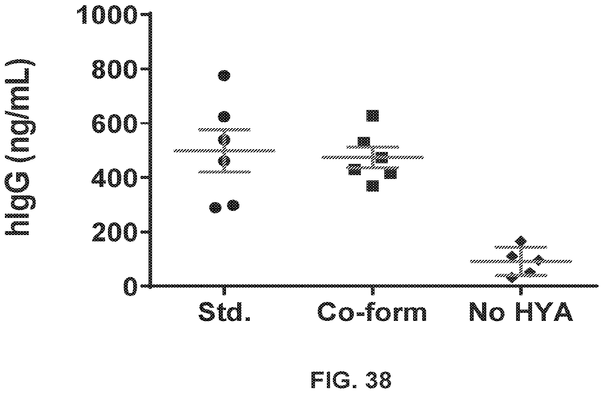

**Figure 38** shows co-formulation study of HYA with pDNA in rabbits. Six New Zealand white rabbits per group. 2 mg pGX9207 in left quad muscle. Two sites. HYA (Intropharma) 200U per site. CELLECTRA^{®}-5P pDNA/HYA co-formulated 5 minutes before administration. Blood was sampled on day 5. Experiment number INO-16-158b.

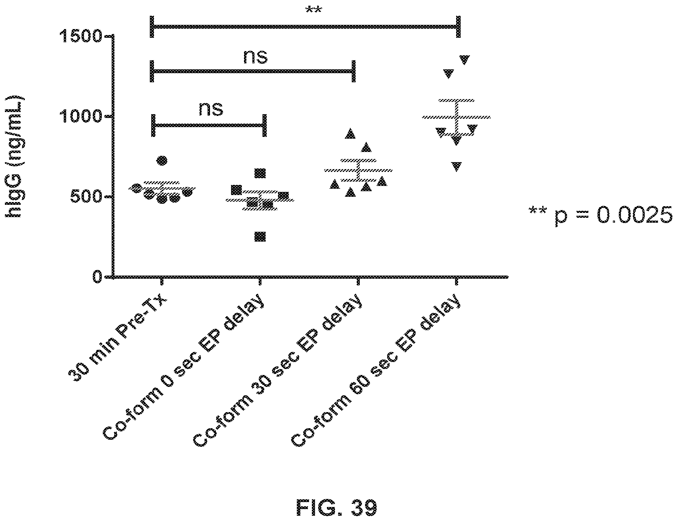

**Figure 39** shows co-formulation of HYA with pDNA in rabbits with EP delay. Six New Zealand white rabbits per group. 2 mg pGX9207 in left quad muscle. Two sites. HYA (Intropharma) 200 U per site. CELLECTRA^{®}-5P pDNA/HYA co-formulated 5 minutes before administration. Blood was sampled on day 5. Experiment number INO-16-158a.

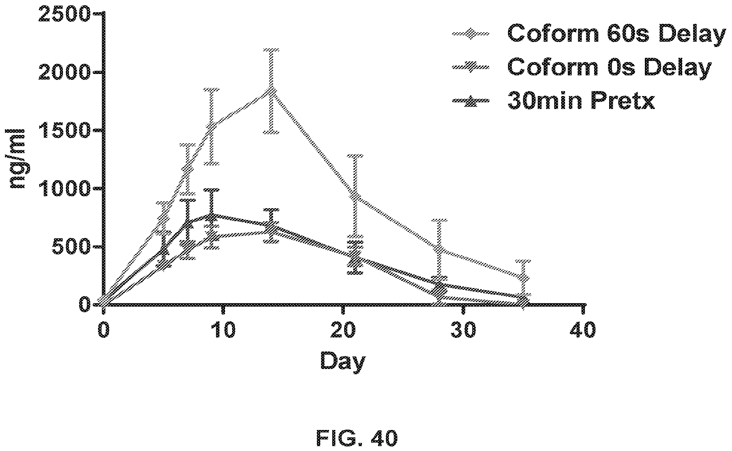

**Figure 40** shows co-formulation of HYA with pDNA in rhesus macaques. pGX9207 (pMERS) delivered into quad muscle with CELLECTRA^{®}-5P. 4 sites. 1 mg pDNA per site. Intropharma (bovine testes purified HYA). pDNA /HYA co-formulated 5 minutes before administration. Experiment number INO-16-194.

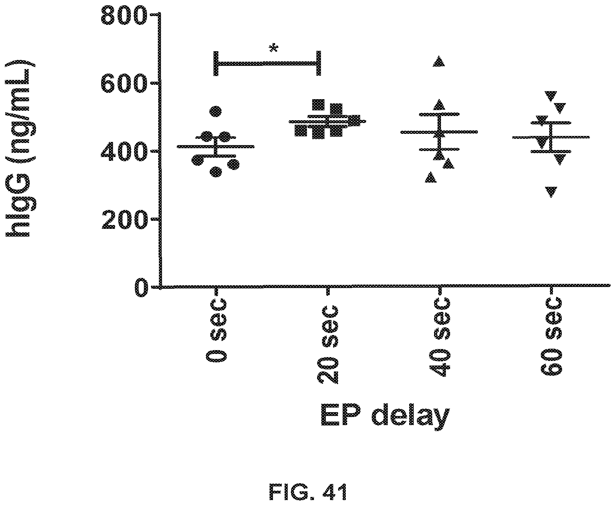

**Figure 41** shows optimization of EP delay with Hylenex (human recombinant hyaluronidase). pGX9207 HYA co-formulation. Two Tx's in left quad. Day 5 hlgG serum levels are depicted. Experiment number INO-16-279.

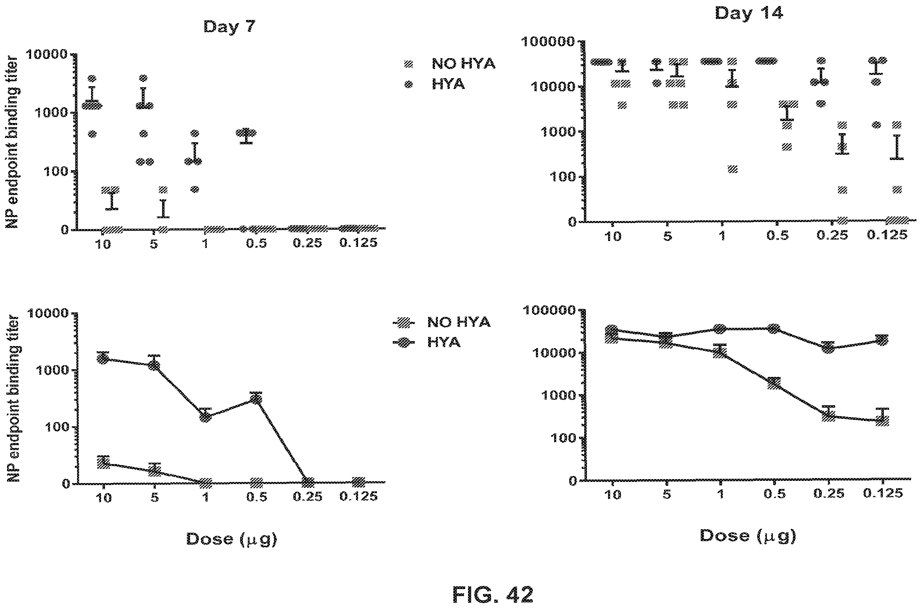

**Figure 42** shows DNA vaccine dose sparing effect of HYA co-formulation. BALB/c mice. Day 0 influenza pNP IM CELLECTRA-3P, day 7 and 14 ELISA. Experiment number INO-16-218.

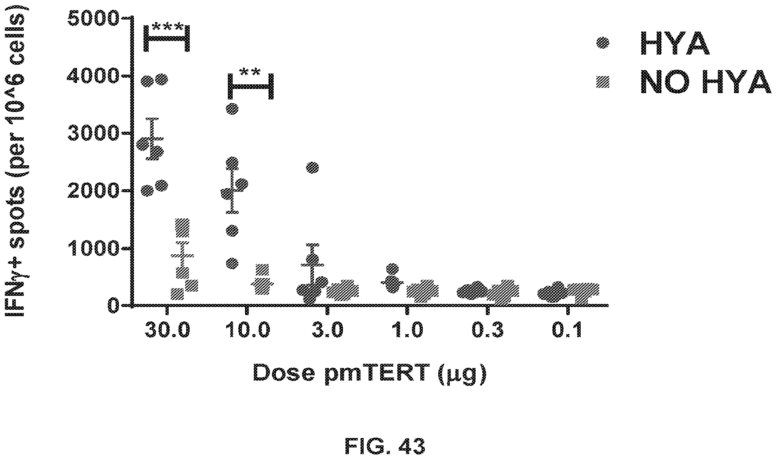

**Figure 43** shows augmentation of immune response to tumor antigen with DNA vaccine HYA formulation. B6 mice. Day 0 and 14 pmTERT IM CELLECTRA^{®}-3P, day 21 IFN-gamma ELISpot against native mouse TERT peptide pools. Experiment number INO-17-018.

### DETAILED DESCRIPTION

The present invention relates to compositions for and methods of delivering a DNA plasmid encoding a monoclonal antibody to a subject. The methods may include administering to the subject a DNA plasmid encoding a monoclonal antibody, and a chondroitinase polypeptide or a polynucleotide encoding a chondroitinase polypeptide in an amount sufficient to hydrolyze sulfate groups of CSPGs. In the invention, the chondroitinase is chondroitinase AC or chondroitinase ABC. The chondroitinase may hydrolyze the sulfate groups of CSPGs in the subject. This disorganization may lead to disorganization of the extracellular matrix and thereby facilitate the delivery of the agent. Methods may further include administering to the subject a hyaluronidase polypeptide or a polynucleotide encoding a hyaluronidase polypeptide.

The present invention also relates to administration of hyaluronidase along with an agent to a subject. The hyaluronidase may be administered as a polypeptide or as a nucleic acid encoding hyaluronidase or a fragment or variant thereof. The hyaluronidase may facilitate delivery of the agent to the subject. As a result, the hyaluronidase may enhance an immune response in the subject and/or enhance expression of the agent in the subject.

### 1) Definitions

Unless otherwise defined, all technical and scientific terms used herein have the same meaning as commonly understood by one of ordinary skill in the art. In case of conflict, the present document, including definitions, will control. Preferred methods and materials are described below, although methods and materials similar or equivalent to those described herein can be used in practice or testing of the present invention. The materials, methods, and examples disclosed herein are illustrative only and not intended to be limiting.

The terms "comprise(s)," "include(s)," "having," "has," "can," "contain(s)," and variants thereof, as used herein, are intended to be open-ended transitional phrases, terms, or words that do not preclude the possibility of additional acts or structures. The singular forms "a," "an," and "the" include plural references unless the context clearly dictates otherwise. The present disclosure also contemplates other embodiments "comprising," "consisting of," and "consisting essentially of," the embodiments or elements presented herein, whether explicitly set forth or not.

The term "about" as used herein as applied to one or more values of interest, refers to a value that is similar to a stated reference value. In certain aspects, the term "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

"Antibody" may mean an antibody of classes IgG, IgM, IgA, IgD, or IgE, or fragments, fragments or derivatives thereof, including Fab, F(ab')2, Fd, and single chain antibodies, and derivatives thereof. The antibody may be an antibody isolated from the serum sample of mammal, a polyclonal antibody, a monoclonal antibody, affinity purified antibody, or mixtures thereof which exhibits sufficient binding specificity to a desired epitope or a sequence derived therefrom. The antibody may be a synthetic antibody as described herein.

"Antibody fragment" or "fragment of an antibody" as used interchangeably herein refers to a portion of an intact antibody comprising the antigen-binding site or variable region. The portion does not include the constant heavy chain domains (i.e., CH2, CH3, or CH4, depending on the antibody isotype) of the Fc region of the intact antibody. Examples of antibody fragments include, but are not limited to, Fab fragments, Fab' fragments, Fab'-SH fragments, F(ab')2 fragments, Fd fragments, Fv fragments, diabodies, single-chain Fv (scFv) molecules, single-chain polypeptides containing only one light chain variable domain, single-chain polypeptides containing the three CDRs of the light-chain variable domain, single-chain polypeptides containing only one heavy chain variable region, and single-chain polypeptides containing the three CDRs of the heavy chain variable region.

"Fragment" as used herein means a nucleic acid sequence or a portion thereof that encodes a polypeptide capable of eliciting an immune response in a mammal. The fragments can be DNA fragments selected from at least one of the various nucleotide sequences that encode protein fragments set forth below. "Fragment" may also refer to a polypeptide sequence or a portion thereof that is capable of eliciting an immune response in a mammal.

"Immune response" as used herein means the activation of a host's immune system, e.g., that of a mammal, in response to the introduction of antigen. The immune response can be in the form of a cellular or humoral response, or both.

"Operably linked" as used herein means that expression of a gene is under the control of a promoter with which it is spatially connected. A promoter can be positioned 5' (upstream) or 3' (downstream) of a gene under its control. The distance between the promoter and a gene can be approximately the same as the distance between that promoter and the gene it controls in the gene from which the promoter is derived. As is known in the art, variation in this distance can be accommodated without loss of promoter function.

A "peptide," "protein," or "polypeptide" as used herein can mean a linked sequence of amino acids and can be natural, synthetic, or a modification or combination of natural and synthetic.

"Polynucleotide" or "oligonucleotide" or "nucleic acid" as used herein means at least two nucleotides covalently linked together. A polynucleotide can be single stranded or double stranded, or can contain portions of both double stranded and single stranded sequence. The polynucleotide can be DNA, both genomic and cDNA, RNA, or a hybrid. The polynucleotide can contain combinations of deoxyribo- and ribo-nucleotides, and combinations of bases including uracil, adenine, thymine, cytosine, guanine, inosine, xanthine hypoxanthine, isocytosine, isoguanine, and synthetic or non-naturally occurring nucleotides and nucleosides. Polynucleotides can be obtained by chemical synthesis methods or by recombinant methods.

"Promoter" as used herein means a synthetic or naturally-derived molecule which is capable of conferring, activating, or enhancing expression of a nucleic acid in a cell. A promoter can comprise one or more specific transcriptional regulatory sequences to further enhance expression and/or to alter the spatial expression and/or temporal expression of same. A promoter can also comprise distal enhancer or repressor elements, which can be located as much as several thousand base pairs from the start site of transcription. A promoter can be derived from sources including viral, bacterial, fungal, plants, insects, and animals. A promoter can regulate the expression of a gene component constitutively or differentially with respect to the cell, the tissue, or organ in which expression occurs, or with respect to the developmental stage at which expression occurs, or in response to external stimuli such as physiological stresses, pathogens, metal ions, or inducing agents. Representative examples of promoters include the bacteriophage T7 promoter, bacteriophage T3 promoter, SP6 promoter, lac operator-promoter, tac promoter, SV40 late promoter, SV40 early promoter, RSV-LTR promoter, CMV IE promoter, SV40 early promoter or SV40 late promoter, and the CMV IE promoter.

"Subject" as used herein can mean a mammal. The mammal can be a human, chimpanzee, dog, cat, horse, cow, mouse, or rat.

"Treatment" or "treating," as used herein can mean protection of an animal from a disease through means of preventing, suppressing, repressing, or completely eliminating the disease. Preventing the disease can include administering a composition of the present invention to an animal prior to onset of the disease. Suppressing the disease involves administering a composition of the present invention to an animal after induction of the disease, but before its clinical appearance. Repressing the disease involves administering a composition of the present invention to an animal after clinical appearance of the disease.

"Variant" as used herein with respect to a nucleic acid means (i) a portion or fragment of a referenced nucleotide sequence; (ii) the complement of a referenced nucleotide sequence or portion thereof; (iii) a nucleic acid that is substantially identical to a referenced nucleic acid or the complement thereof; or (iv) a nucleic acid that hybridizes under stringent conditions to the referenced nucleic acid, complement thereof, or a sequences substantially identical thereto.

"Variant" can further be defined as a peptide or polypeptide that differs in amino acid sequence by the insertion, deletion, or conservative substitution of amino acids, but retains at least one biological activity. Representative examples of "biological activity" include the ability to be bound by a specific antibody or to promote an immune response. Variant can also mean a protein with an amino acid sequence that is substantially identical to a referenced protein with an amino acid sequence that retains at least one biological activity. A conservative substitution of an amino acid, i.e., replacing an amino acid with a different amino acid of similar properties (e.g., hydrophilicity, degree and distribution of charged regions) is recognized in the art as typically involving a minor change. These minor changes can be identified, in part, by considering the hydropathic index of amino acids, as understood in the art (Kyte et al., J. Mol. Biol. 1982, 157, 105-132). The hydropathic index of an amino acid is based on a consideration of its hydrophobicity and charge. It is known in the art that amino acids of similar hydropathic indexes can be substituted and still retain protein function. In one aspect, amino acids having hydropathic indexes of ± 2 are substituted. The hydrophilicity of amino acids can also be used to reveal substitutions that would result in proteins retaining biological function. A consideration of the hydrophilicity of amino acids in the context of a peptide permits calculation of the greatest local average hydrophilicity of that peptide, a useful measure that has been reported to correlate well with antigenicity and immunogenicity. Substitution of amino acids having similar hydrophilicity values can result in peptides retaining biological activity, for example immunogenicity, as is understood in the art. Substitutions can be performed with amino acids having hydrophilicity values within ±2 of each other. Both the hydrophobicity index and the hydrophilicity value of amino acids are influenced by the particular side chain of that amino acid. Consistent with that observation, amino acid substitutions that are compatible with biological function are understood to depend on the relative similarity of the amino acids, and particularly, the side chains of those amino acids, as revealed by the hydrophobicity, hydrophilicity, charge, size, and other properties.

A variant may be a nucleic acid sequence that is substantially identical over the full length of the full gene sequence or a fragment thereof. The nucleic acid sequence may be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the gene sequence or a fragment thereof. A variant may be an amino acid sequence that is substantially identical over the full length of the amino acid sequence or fragment thereof. The amino acid sequence may be 80%, 81%, 82%, 83%, 84%, 85%, 86%, 87%, 88%, 89%, 90%, 91%, 92%, 93%, 94%, 95%, 96%, 97%, 98%, 99%, or 100% identical over the full length of the amino acid sequence or a fragment thereof.

"Vector" as used herein means a nucleic acid sequence containing an origin of replication. A vector can be a viral vector, bacteriophage, bacterial artificial chromosome, or yeast artificial chromosome. A vector can be a DNA or RNA vector. A vector can be a selfreplicating extrachromosomal vector, and preferably, is a DNA plasmid.

For the recitation of numeric ranges herein, each intervening number there between with the same degree of precision is explicitly contemplated. For example, for the range of 6-9, the numbers 7 and 8 are contemplated in addition to 6 and 9, and for the range 6.0-7.0, the number 6.0, 6.1, 6.2, 6.3, 6.4, 6.5, 6.6, 6.7, 6.8, 6.9, and 7.0 are explicitly contemplated.

### 2) Use of Chondroitinase to Enhance Agent Delivery

The present invention relates to administration of chondroitinase along with an agent to a subject. The chondroitinase may be administered as a polypeptide or as a nucleic acid encoding chondroitinase or a fragment or variant thereof. The chondroitinase may facilitate delivery of the agent to the subject. As a result, the chondroitinase may enhance an immune response in the subject and/or enhance expression of the agent in the subject.

### a) Chondroitinase

A chondroitinase or fragment thereof may be administered to a subject. In the invention, the chondroitinase is chondroitinase AC or chondroitinase ABC. As part of the disclosure, but not part of the invention, the chondroitinase may be any chondroitinase. For example, the chondroitinase may be an N-acetylgalactosamine-4-sulfatase, an N-acetylgalactosamine-6-sulfatase, or a chondroitin ABC lyase. The chondroitinase may be chondroitinase AC. The chondroitinase may be recombinant chondroitinase. The chondroitinase may catalyze the hydrolysis of a chondroitin sulfate proteoglycan (CSPG). The chondroitinase may hydrolyze the 4-sulfate groups of the N-acetyl-D-galactosamine 4-sulfate units of chondroitin sulfate and/or dermatan sulfate. The chondroitinase may hydrolyze the 4-sulfate groups of N-acetyl glucosamine 4-sulfate. The chondroitinase may hydrolyze the 6-sulfate groups of the N-acetyl-D-galactosamine 6-sulfate units of chondroitin sulfate and of the D-galactose 6-sulfate units of keratin sulfate. The chondroitin ABC lyase may catalyze the degradation of polysaccharides containing 1,4-beta-D-hexosaminyl and 1,3-beta-D-glucuronosyl or 1,3-alpha-L-iduronosyl linkages to disaccharides containing 4-deoxy-beta-D-gluc-4-enuronosyl groups. The chondroitin ABC lyase may act on chondroitin 4-sulfate, chondroitin 6-sulfate, and dermatan sulfate.

The CSPG may be Aggrecan (CSPG1), Versican (CSPG2), Neurocan (CSPG3), CSPG4 (melanoma-associated chondroitin sulfate proteoglycan, NG2), CSPG5, SMC3 (CSPG6, Structural maintenance of chromosome 3), Brevican (CSPG7), CD44 (CSPG8, cluster of differentiation 44), Phosphacan, and combinations thereof.

By catalyzing the hydrolysis of CSPGs, constituents of the extracellular matrix (ECM), chondroitinase lowers the viscosity of the CSPGs and the extracellular matrix, thereby increasing tissue permeability. Administration of chondroitinase, or a polynucleotide encoding chondroitinase, may lead to hydrolysis of CSPGs, thereby leading to disorganization of an extracellular matrix of the subject. Disorganization of an extracellular matrix of the subject may thereby facilitate delivery or administration of an agent.

The chondroitinase may be a chondroitinase derived from a bacterium. The bacterium may be *Flavobacterium heparinum,* for example. The chondroitinase may be recombinantly produced in a bacterium.

In some embodiments, a chondroitinase polypeptide or fragment thereof may be administered.

In some embodiments, a polynucleotide encoding a chondroitinase polypeptide or fragment thereof may be administered. The chondroitinase polypeptide may be expressed from the polynucleotide encoding the chondroitinase polypeptide *in vivo.*

### 3) Use of Hyaluronidase

The methods may also comprise further administering to the subject a hyaluronidase polypeptide or a polynucleotide encoding a hyaluronidase polypeptide in an amount to degrade a glycosaminoglycan, such as hyaluronan. Administration of chondroitinase and hyaluronidase may lead to an additive or synergistic effect on agent expression, immune response, or concentration of agent in the subject's serum, for example. The agent concentration detected in the serum of a subject exposed to chondroitinase and hyaluronidase combination treatment may be higher as compared to serum levels of the agent in a subject treated with a single chondroitinase or hyaluronidase enzyme, for example.

The present invention also relates to administration of hyaluronidase along with an agent to a subject. The hyaluronidase may be administered as a polypeptide or as a nucleic acid encoding hyaluronidase or a fragment or variant thereof. The hyaluronidase may facilitate delivery of the agent to the subject. As a result, the hyaluronidase may enhance an immune response in the subject and/or enhance expression of the agent in the subject. The method of administering hyaluronidase to a subject may or may not include administration of chondroitinase. The agent may be any agent as described herein. The use of hyaluronidase may be in conjunction with any agent, described timing of administration, mode of administration, method of treatment, or method of delivery, as described herein.

The hyaluronidase polypeptide or the polynucleotide encoding the hyaluronidase polypeptide and the agent may be administered to the subject concurrently. The hyaluronidase polypeptide or the polynucleotide encoding the hyaluronidase polypeptide may be administered concurrently with the chondroitinase polypeptide, or the polynucleotide encoding the chondroitinase polypeptide, and the agent. The methods of delivery and times of administration may be the same as, or similar, to those described herein. See section 4 b), for example.

The hyaluronidase polypeptide or the polynucleotide encoding the hyaluronidase polypeptide and the agent may be administered to the subject consecutively. The hyaluronidase polypeptide or the polynucleotide encoding the hyaluronidase polypeptide and the chondroitinase polypeptide, or the polynucleotide encoding the chondroitinase polypeptide, and the agent may be administered to the subject consecutively. The methods of delivery and times of administration may be the same as, or similar, to those described herein. See section 4 b), for example. The chondroitinase polypeptide or the polynucleotide encoding the chondroitinase polypeptide, the hyaluronidase polypeptide or a polynucleotide encoding a hyaluronidase polypeptide, and the agent may be co-formulated prior to administration. The chondroitinase polypeptide or the polynucleotide encoding the chondroitinase polypeptide, and the hyaluronidase polypeptide or a polynucleotide encoding a hyaluronidase polypeptide, may be co-formulated prior to administration. Any coformulation of chondroitinase polypeptide or the polynucleotide encoding the chondroitinase polypeptide, the hyaluronidase polypeptide or a polynucleotide encoding a hyaluronidase polypeptide, and the agconent may occur, for example, 1 minute, 5 minutes, 10 minutes, 15 minutes, 20 minutes, 45 minutes, 1 hour, 5 hours, 10 hours, 24 hours, 5 days, 7 days, 20 days, 30 day, 40 days, 50 days, 100 days, 200 days, 300 days, 1 year, 400 days, 1.5 years, or 2 years prior to administration. Any co-formulation of chondroitinase polypeptide, or the polynucleotide encoding the chondroitinase polypeptide, and the agent may occur, for example, 1 minute, 5 minutes, 10 minutes, 15 minutes, 20 minutes, 45 minutes, 1 hour, 5 hours, 10 hours, 24 hours, 5 days, 7 days, 20 days, 30 days, 40 days, 50 days, 100 days, 200 days, 300 days, 1 year, 400 days, 1.5 years, or 2 years prior to administration. Any coformulation of hyaluronidase polypeptide, or a polynucleotide encoding a hyaluronidase polypeptide, and the agent may occur, for example, 1 minute, 5 minutes, 10 minutes, 15 minutes, 20 minutes, 45 minutes, 1 hour, 5 hours, 10 hours, 24 hours, 5 days, 7 days, 20 days, 30 day, 40 days, 50 days, 100 days, 200 days, 300 days, 1 year, 400 days, 1.5 years, or 2 years prior to administration. Any co-formulation of chondroitinase polypeptide or the polynucleotide encoding the chondroitinase polypeptide, and the hyaluronidase polypeptide or a polynucleotide encoding a hyaluronidase polypeptide, may occur, for example, 1 minute, 5 minutes, 10 minutes, 15 minutes, 20 minutes, 45 minutes, 1 hour, 5 hours, 10 hours, 24 hours, 5 days, 7 days, 20 days, 30 day, 40 days, 50 days, 100 days, 200 days, 300 days, 1 year, 400 days, 1.5 years, or 2 years prior to administration days prior to administration.

The chondroitinase and/or hyaluronidase and/or agent can be administered via electroporation (EP), such as by a method described in U.S. Patent No. 7,664,545. In the invention, the DNA plasmid is administered via electroporation. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359. The electroporation may be carried out via a minimally invasive device. Electroporation may occur before or after administration of the chondroitinase and/or hyaluronidase and/or agent, for example. Electroporation may occur concomitant with the administration of the chondroitinase and/or hyaluronidase and/or agent, for example. There may be a delay between the administration of the chondroitinase, hyaluronidase, agent, or any co-formulation thereof, and EP. For example, EP may be administered 5 seconds, 10 seconds, 20 seconds, 30 seconds, 45 seconds, 1 minute, 2 minutes, 3 minutes, 4 minutes, or 5 minutes after the administration of chondroitinase, hyaluronidase, agent, antigen, or any coformulation thereof.

Hyaluronidase may refer to a polypeptide that degrades hyaluronic acid. "Hyaluronic acid" and "hyaluronan" are used herein interchangeably. Hyaluronan is an anionic, nonsulfated glycosaminoglycan. Hyaluronan is a polymer of disaccharides, each disaccharide comprising D-glucuronic acid and D-N-acetylglucosamine, linked via alternating β -1,4 and β -1,3 glycosidic bonds. Hyaluronan may comprise thousands of disaccharide repeats in length. Hyaluronan may have a molecular weight of about 1 kDa to about 5,000 kDa or more.

Hyaluronidases are a family of glycosaminoglycan endoglucosaminidases, wherein a glutamate residue in the hyaluronidase hydrolyzes the β-1,4 linkages of hyaluronan and chondroitin sulfates through an acid-base catalytic mechanism.

By catalyzing the hydrolysis of hyaluronan, a constituent of the extracellular matrix (ECM), hyaluronidase lowers the viscosity of hyaluronan and the extracellular matrix, thereby increasing tissue permeability. Administration of hyaluronidase, or a polynucleotide encoding hyaluronidase, may lead to hydrolysis of hyaluronan, thereby leading to disorganization of an extracellular matrix of the subject. Disorganization of an extracellular matrix of the subject may thereby facilitate delivery or administration of an agent.

The hyaluronidase can be a hyaluronidase derived from a mammalian origin, a reptilian or hymenopteran hyaluronate glycanohydrolase, a hyaluronate glycanohydrolase from the salivary gland of the leech, or a bacterial origin. Bacterial hyaluronidases may include, for example, streptococcal, pneumococcal, and clostridial hyaluronidases.

### 4) Agent

An agent may be administered to the subject. As part of the disclosure, the agent may comprise a polypeptide, a polynucleotide, a small molecule, an antigen, or any combination thereof. The agent may comprise a recombinant nucleic acid sequence encoding an antibody, a fragment thereof, a variant thereof, or a combination thereof, as detailed in, for example, PCT/US2014/070188. In the invention the agent is a DNA-encoding monoclonal antibody (DMAb).

### i) Polypeptide

**In** some embodiments of the disclosure, the agent comprises a polypeptide. The polypeptide may comprise an antibody, an antigen, an enzyme, or other protein, or any combination thereof. The polypeptide may be derived from a mammalian, animal, bacterial, or viral origin. The polypeptide may be a heterologous polypeptide, i.e., derived from different sources or organisms. In some embodiments, the polypeptide comprises an antibody. In some embodiments of the disclosure, but not the invention, the antibody is a polyclonal antibody. In the invention, the antibody is a monoclonal antibody encoded by the DNA plasmid.

### (1) Antibody

As described above, the polypeptide can comprise an antibody, a fragment thereof, a variant thereof, or a combination thereof. The antibody can bind or react with a desired target molecule, which may be the antigen, which is described in more detail below, a ligand, including a ligand for a receptor, a receptor, including a ligand-binding site on the receptor, a ligand-receptor complex, and a marker, including a cancer marker.

The antibody may comprise a heavy chain and a light chain complementarity determining region ("CDR") set, respectively interposed between a heavy chain and a light chain framework ("FR") set which provide support to the CDRs and define the spatial relationship of the CDRs relative to each other. The CDR set may contain three hypervariable regions of a heavy or light chain V region. Proceeding from the N-terminus of a heavy or light chain, these regions are denoted as "CDR1," "CDR2," and "CDR3," respectively. An antigen-binding site, therefore, may include six CDRs, comprising the CDR set from each of a heavy and a light chain V region.

The proteolytic enzyme papain preferentially cleaves IgG molecules to yield several fragments, two of which (the F(ab) fragments) each comprise a covalent heterodimer that includes an intact antigen-binding site. The enzyme pepsin is able to cleave IgG molecules to provide several fragments, including the F(ab')₂ fragment, which comprises both antigen-binding sites. Accordingly, the antibody can be the Fab or F(ab')_{2.} The Fab can include the heavy chain polypeptide and the light chain polypeptide. The heavy chain polypeptide of the Fab can include the VH region and the CH1 region. The light chain of the Fab can include the VL region and CL region.

The antibody can be an immunoglobulin (Ig). The Ig can be, for example, IgA, IgM, IgD, IgE, and IgG. The immunoglobulin can include the heavy chain polypeptide and the light chain polypeptide. The heavy chain polypeptide of the immunoglobulin can include a VH region, a CH1 region, a hinge region, a CH2 region, and a CH3 region. The light chain polypeptide of the immunoglobulin can include a VL region and CL region.

The antibody of the disclosure can be a polyclonal antibody. The antibody of the invention is a monoclonal antibody. The antibody can be a chimeric antibody, a single chain antibody, an affinity matured antibody, a human antibody, a humanized antibody, or a fully human antibody. The humanized antibody can be an antibody from a non-human species that binds the desired antigen having one or more complementarity determining regions (CDRs) from the non-human species and framework regions from a human immunoglobulin molecule.

The antibody can be a bispecific antibody, a fragment thereof, a variant thereof, or a combination thereof. The bispecific antibody can bind or react with two antigens, for example, two of the antigens described below in more detail. The bispecific antibody can be comprised of fragments of two of the antibodies described herein, thereby allowing the bispecific antibody to bind or react with two desired target molecules, which may include the antigen, which is described below in more detail, a ligand, including a ligand for a receptor, a receptor, including a ligand-binding site on the receptor, a ligand-receptor complex, and a marker, including a cancer marker.

The antibody can be a bifunctional antibody, a fragment thereof, a variant thereof, or a combination thereof. The bifunctional antibody can bind or react with the antigen described below. The bifunctional antibody can also be modified to impart an additional functionality to the antibody beyond recognition of and binding to the antigen. Such a modification can include, but is not limited to, coupling to factor H or a fragment thereof. Factor H is a soluble regulator of complement activation and thus, may contribute to an immune response via complement-mediated lysis (CML).

As described above, the antibody can be generated in the subject upon administration of the composition to the subject. The antibody may have a half-life within the subject. In some embodiments, the antibody may be modified to extend or shorten its half-life within the subject the subject. Such modifications are described below in more detail.

### ii) Polynucleotide

In some embodiments, the agent comprises a polynucleotide. In some embodiments, the agent is a polypeptide encoded by a polynucleotide, as detailed above. The polynucleotide encodes an antibody. The antibody is a monoclonal antibody. The polynucleotide encoding a monoclonal antibody may facilitate in vivo expression and formation of the monoclonal antibody.

In some embodiments, the chondroitinase polypeptide and the monoclonal antibody are encoded by the same polynucleotide. In some embodiments, the chondroitinase polypeptide and the monoclonal antibody are encoded by separate polynucleotides.

### (1) Vector

One or more vectors may include a polynucleotide. In some embodiments, the polynucleotide encoding the chondroitinase polypeptide and the polynucleotide encoding the agent are comprised within the same vector. In some embodiments, the polynucleotide encoding the chondroitinase polypeptide and the polynucleotide encoding the agent are comprised within separate vectors. The one or more vectors can be capable of expressing the agent. The one or more vectors can be an expression construct, which is generally a plasmid that is used to introduce a specific gene into a target cell. Once the expression vector is inside the cell, the polypeptide that is encoded by the gene is produced by the cellulartranscription and translation machinery ribosomal complexes. The plasmid is frequently engineered to contain regulatory sequences that act as enhancer and promoter regions and lead to efficient transcription of the gene carried on the expression vector. In on embodiment, the vectors of the present invention can express large amounts of stable messenger RNA, and therefore polypeptides.

### (a) Expression Vectors

The vector can be a circular plasmid or a linear nucleic acid. The circular plasmid and linear nucleic acid are capable of directing expression of a particular nucleotide sequence in an appropriate subject cell. The vector can have a promoter operably linked to the antigen-encoding nucleotide sequence, or the adjuvant-encoding nucleotide sequence, which may be operably linked to termination signals. The vector can also contain sequences required for proper translation of the nucleotide sequence. The vector comprising the nucleotide sequence of interest may be chimeric, meaning that at least one of its components is heterologous with respect to at least one of its other components. The expression of the nucleotide sequence in the expression cassette may be under the control of a constitutive promoter or of an inducible promoter, which initiates transcription only when the host cell is exposed to some particular external stimulus. In the case of a multicellular organism, the promoter can also be specific to a particular tissue or organ or stage of development.

### (b) Circular and Linear Vectors

The vector may be a circular plasmid, which may transform a target cell by integration into the cellular genome or exist extrachromosomally (e.g., autonomous replicating plasmid with an origin of replication).

The vector can be pVAX, pcDNA3.0, or provax, or any other expression vector capable of expressing DNA encoding the agent, and enabling a cell to translate the sequence to an agent.

Also provided herein is a linear nucleic acid, or linear expression cassette ("LEC"), that is capable of being efficiently delivered to a subject via electroporation and expressing one or more desired agents. The LEC may be any linear DNA devoid of any phosphate backbone. The DNA may encode one or more agents. The LEC may contain a promoter, an intron, a stop codon, and/or a polyadenylation signal. The expression of the agent may be controlled by the promoter. The LEC may not contain any antibiotic resistance genes and/or a phosphate backbone. The LEC may not contain other nucleic acid sequences unrelated to the desired agent gene expression.

The LEC may be derived from any plasmid capable of being linearized. The plasmid may be capable of expressing the agent. The plasmid can be pNP (Puerto Rico/34) or pM2 (New Caledonia/99). The plasmid may be WLV009, pVAX, pcDNA3.0, or provax, or any other expression vector capable of expressing DNA encoding the agent, and enabling a cell to translate the sequence to an agent.

The LEC can be pcrM2. The LEC can be pcrNP. pcrNP and pcrMR can be derived from pNP (Puerto Rico/34) and pM2 (New Caledonia/99), respectively.

### (c) Promoter, Intron, Stop Codon, and Polyadenylation Signal

The vector may have a promoter. A promoter may be any promoter that is capable of driving gene expression and regulating expression of the isolated nucleic acid. Such a promoter is a cis-acting sequence element required for transcription via a DNA dependent RNA polymerase, which transcribes the agent sequence described herein. Selection of the promoter used to direct expression of a heterologous nucleic acid depends on the particular application. The promoter may be positioned about the same distance from the transcription start in the vector as it is from the transcription start site in its natural setting. However, variation in this distance may be accommodated without loss of promoter function.

The promoter may be operably linked to the nucleic acid sequence encoding the agent and signals required for efficient polyadenylation of the transcript, ribosome binding sites, and translation termination. The promoter may be operably linked to the nucleic acid sequence encoding the agent and signals required for efficient polyadenylation of the transcript, ribosome binding sites, and translation termination.

The promoter may be a CMV promoter, SV40 early promoter, SV40 later promoter, metallothionein promoter, murine mammary tumor virus promoter, Rous sarcoma virus promoter, polyhedrin promoter, or another promoter shown effective for expression in eukaryotic cells.

The vector may include an enhancer and an intron with functional splice donor and acceptor sites. The vector may contain a transcription termination region downstream of the structural gene to provide for efficient termination. The termination region may be obtained from the same gene as the promoter sequence or may be obtained from different genes.

### iii) Small Molecule

As part of the disclosure, the agent may comprise a small molecule. Small molecules may include, for example, pharmaceuticals or drugs, organic compounds, organometallic compounds, antigens, hormones, vitamins, antibiotics, cofactors, cytokines, steroids, carbohydrates, sugars, alcohols, polyenes, alkaloids, glycosides, flavonoids, carboxylates, pyrroles, phenazines, fatty acids, amines, nucleobases and their derivatives (e.g., nucleotides and nucleosides), amino acids, and cellular metabolites.

### iv) Antigen

An antigen may be administered to the subject. "Antigen" can be anything that has the ability to generate an immune response in a subject. An antigen may be a polynucleotide, a polypeptide, or a combination thereof. The polynucleotide can also include additional sequences that encode linker or tag sequences that are linked to the antigen by a peptide bond. An antigen can comprise a small molecule, as detailed above.

An antigen can be contained in a polynucleotide, a polypeptide, or a fragment thereof, or a variant thereof, or a combination thereof from any number of organisms, for example, a virus, a parasite, a bacterium, a fungus, or a mammal. The antigen can be associated with an autoimmune disease, allergy, or asthma. In other embodiments, the antigen can be associated with cancer, herpes, influenza, hepatitis B, hepatitis C, human papilloma virus (HPV), or human immunodeficiency virus (HIV).

An antigen may be recognized and bound by an antibody. Some antigens can induce a strong immune response. Other antigens can induce a weak immune response. An antigen may originate from within the body or from the external environment. An antigen can be a foreign antigen or a self-antigen.

In some embodiments, the antibody, as described above, may bind or react with the antigen.

In some embodiments, the chondroitinase polypeptide and the agent comprising a polynucleotide are encoded by the same polynucleotide or separate polynucleotides. In some embodiments, the chondroitinase polypeptide, the agent comprising a polynucleotide, and the antigen are comprised within the same vector or separate vectors.

The antigen can be anything that induces an immune response in a subject. Purified antigens are not usually strongly immunogenic on their own and are therefore combined with the adjuvant as described above. The immune response induced by the antigen can be boosted or increased when combined with the adjuvant. Such an immune response can be a humoral immune response and/or a cellular immune response. In some embodiments, the combination of the adjuvant and the antigen can boost or increase a cellular immune response in the subject.

The antigen can be a nucleic acid sequence, an amino acid sequence, or a combination thereof. The nucleic acid sequence can be DNA, RNA, cDNA, a variant thereof, a fragment thereof, or a combination thereof. The nucleic acid sequence can also include additional sequences that encode linker or tag sequences that are linked to the antigen by a peptide bond. The amino acid sequence can be a protein, a peptide, a variant thereof, a fragment thereof, or a combination thereof.

The antigen can be contained in a protein, a nucleic acid, or a fragment thereof, or a variant thereof, or a combination thereof from any number of organisms, for example, a virus, a parasite, a bacterium, a fungus, or a mammal. The antigen can be associated with an autoimmune disease, allergy, or asthma. In other embodiments, the antigen can be associated with cancer, herpes, influenza, hepatitis B, hepatitis C, human papilloma virus (HPV), or human immunodeficiency virus (HIV). Preferably, the antigen can be associated with influenza or HIV.

Some antigens can induce a strong immune response. Other antigens can induce a weak immune response. The antigen can elicit a greater immune response when combined with an adjuvant.

### (1) Viral Antigens

The antigen can be a viral antigen, or fragment thereof, or variant thereof. The viral antigen can be from a virus from one of the following families: *Adenoviridae, Arenaviridae, Bunyaviridae, Caliciviridae, Coronaviridae, Filoviridae, Hepadnaviridae, Herpesviridae, Orthomyxoviridae, Papovaviridae, Paramyxoviridae, Parvoviridae, Picornaviridae, Poxviridae, Reoviridae, Retroviridae, Rhabdoviridae,* or *Togaviridae.* The viral antigen can be from papilloma viruses, for example, human papilloma virus (HPV), human immunodeficiency virus (HIV), polio virus, hepatitis B virus, hepatitis C virus, smallpox virus (Variola major and minor), vaccinia virus, influenza virus, rhinoviruses, dengue fever virus, equine encephalitis viruses, rubella virus, yellow fever virus, Norwalk virus, hepatitis A virus, human T-cell leukemia virus (HTLV-I), hairy cell leukemia virus (HTLV-II), California encephalitis virus, Hanta virus (hemorrhagic fever), rabies virus, Ebola fever virus, Marburg virus, measles virus, mumps virus, respiratory syncytial virus (RSV), herpes simplex 1 (oral herpes), herpes simplex 2 (genital herpes), herpes zoster (varicellazoster, a.k.a., chickenpox), cytomegalovirus (CMV), for example human CMV, Epstein-Barr virus (EBV), flavivirus, foot and mouth disease virus, chikungunya virus, lassa virus, arenavirus, lymphocytic choriomeningitis virus (LCMV), or cancer causing virus.

### (a) Hepatitis Antigen

The antigen may be a hepatitis virus antigen (i.e., hepatitis antigen), or fragment thereof, or variant thereof. The hepatitis antigen can be an antigen or immunogen from hepatitis A virus (HAV), hepatitis B virus (HBV), hepatitis C virus (HCV), hepatitis D virus (HDV), and/or hepatitis E virus (HEV). In some embodiments, the hepatitis antigen can be a heterologous nucleic acid molecule(s), such as a plasmid(s), which encodes one or more of the antigens from HAV, HBV, HCV, HDV, and HEV. The hepatitis antigen can be full-length or immunogenic fragments of full-length proteins.

The hepatitis antigen can comprise consensus sequences and/or one or more modifications for improved expression. Genetic modifications, including codon optimization, RNA optimization, and the addition of a highly efficient immunoglobulin leader sequence to increase the immunogenicity of the constructs, can be included in the modified consensus sequences. The consensus hepatitis antigen may comprise a signal peptide such as an immunoglobulin signal peptide such as an IgE or IgG signal peptide, and in some embodiments, may comprise an HA tag. The immunogens can be designed to elicit stronger and broader cellular immune responses than corresponding codon optimized immunogens.

The hepatitis antigen can be an antigen from HAV. The hepatitis antigen can be a HAV capsid protein, a HAV non-structural protein, a fragment thereof, a variant thereof, or a combination thereof.

The hepatitis antigen can be an antigen from HCV. The hepatitis antigen can be a HCV nucleocapsid protein (i.e., core protein), a HCV envelope protein (e.g., E1 and E2), a HCV non-structural protein (e.g., NS1, NS2, NS3, NS4a, NS4b, NS5a, and NS5b), a fragment thereof, a variant thereof, or a combination thereof.

The hepatitis antigen can be an antigen from HDV. The hepatitis antigen can be a HDV delta antigen, fragment thereof, or variant thereof.

The hepatitis antigen can be an antigen from HEV. The hepatitis antigen can be a HEV capsid protein, fragment thereof, or variant thereof.

The hepatitis antigen can be an antigen from HBV. The hepatitis antigen can be a HBV core protein, a HBV surface protein, a HBV DNA polymerase, a HBV protein encoded by gene X, fragment thereof, variant thereof, or combination thereof. The hepatitis antigen can be a HBV genotype A core protein, a HBV genotype B core protein, a HBV genotype C core protein, a HBV genotype D core protein, a HBV genotype E core protein, a HBV genotype F core protein, a HBV genotype G core protein, a HBV genotype H core protein, a HBV genotype A surface protein, a HBV genotype B surface protein, a HBV genotype C surface protein, a HBV genotype D surface protein, a HBV genotype E surface protein, a HBV genotype F surface protein, a HBV genotype G surface protein, a HBV genotype H surface protein, fragment thereof, variant thereof, or combination thereof. The hepatitis antigen can be a consensus HBV core protein, or a consensus HBV surface protein.

In some embodiments, the hepatitis antigen can be a HBV genotype A consensus core DNA sequence construct, an IgE leader sequence linked to a consensus sequence for HBV genotype A core protein, or a HBV genotype A consensus core protein sequence.

In other embodiments, the hepatitis antigen can be a HBV genotype B consensus core DNA sequence construct, an IgE leader sequence linked to a consensus sequence for HBV genotype B core protein, or a HBV genotype B consensus core protein sequence.

In still other embodiments, the hepatitis antigen can be a HBV genotype C consensus core DNA sequence construct, an IgE leader sequence linked to a consensus sequence for HBV genotype C core protein, or a HBV genotype C consensus core protein sequence.

In some embodiments, the hepatitis antigen can be a HBV genotype D consensus core DNA sequence construct, an IgE leader sequence linked to a consensus sequence for HBV genotype D core protein, or a HBV genotype D consensus core protein sequence.

In other embodiments, the hepatitis antigen can be a HBV genotype E consensus core DNA sequence construct, an IgE leader sequence linked to a consensus sequence for HBV genotype E core protein, or a HBV genotype E consensus core protein sequence.

In some embodiments, the hepatitis antigen can be a HBV genotype F consensus core DNA sequence construct, an IgE leader sequence linked to a consensus sequence for HBV genotype F core protein, or a HBV genotype F consensus core protein sequence.

In other embodiments, the hepatitis antigen can be a HBV genotype G consensus core DNA sequence construct, an IgE leader sequence linked to a consensus sequence for HBV genotype G core protein, or a HBV genotype G consensus core protein sequence.

In some embodiments, the hepatitis antigen can be a HBV genotype H consensus core DNA sequence construct, an IgE leader sequence linked to a consensus sequence for HBV genotype H core protein, or a HBV genotype H consensus core protein sequence.

In still other embodiments, the hepatitis antigen can be a HBV genotype A consensus surface DNA sequence construct, an IgE leader sequence linked to a consensus sequence for HBV genotype A surface protein, or a HBV genotype A consensus surface protein sequence.

In some embodiments, the hepatitis antigen can be a HBV genotype B consensus surface DNA sequence construct, an IgE leader sequence linked to a consensus sequence for HBV genotype B surface protein, or a HBV genotype B consensus surface protein sequence.

In other embodiments, the hepatitis antigen can be a HBV genotype C consensus surface DNA sequence construct, an IgE leader sequence linked to a consensus sequence for HBV genotype C surface protein, or a HBV genotype C consensus surface protein sequence.

In still other embodiments, the hepatitis antigen can be a HBV genotype D consensus surface DNA sequence construct, an IgE leader sequence linked to a consensus sequence for HBV genotype D surface protein, or a HBV genotype D consensus surface protein sequence.

In some embodiments, the hepatitis antigen can be a HBV genotype E consensus surface DNA sequence construct, an IgE leader sequence linked to a consensus sequence for HBV genotype E surface protein, or a HBV genotype E consensus surface protein sequence.

In other embodiments, the hepatitis antigen can be a HBV genotype F consensus surface DNA sequence construct, an IgE leader sequence linked to a consensus sequence for HBV genotype F surface protein, or a HBV genotype F consensus surface protein sequence.

In still other embodiments, the hepatitis antigen can be a HBV genotype G consensus surface DNA sequence construct, an IgE leader sequence linked to a consensus sequence for HBV genotype G surface protein, or a HBV genotype G consensus surface protein sequence.

In other embodiments, the hepatitis antigen can be a HBV genotype H consensus surface DNA sequence construct, an IgE leader sequence linked to a consensus sequence for HBV genotype H surface protein, or a HBV genotype H consensus surface protein sequence.

### (b) Human Papilloma Virus (HPV) Antigen

The antigen may be a human papilloma virus (HPV) antigen, or fragment thereof, or variant thereof. The HPV antigen can be from HPV types 16, 18, 31, 33, 35, 45, 52, and 58 which cause cervical cancer, rectal cancer, and/or other cancers. The HPV antigen can be from HPV types 6 and 11, which cause genital warts, and are known to be causes of head and neck cancer.

The HPV antigens can be the HPV E6 or E7 domains from each HPV type. For example, for HPV type 16 (HPV16), the HPV16 antigen can include the HPV16 E6 antigen, the HPV16 E7 antigen, fragments, variants, or combinations thereof. Similarly, the HPV antigen can be HPV 6 E6 and/or E7, HPV 11 E6 and/or E7, HPV 18 E6 and/or E7, HPV 31 E6 and/or E7, HPV 33 E6 and/or E7, HPV 52 E6 and/or E7, or HPV 58 E6 and/or E7, fragments, variants, or combinations thereof.

### (c) RSV Antigen

The antigen may be an RSV antigen or fragment thereof, or variant thereof. The RSV antigen can be a human RSV fusion protein (also referred to herein as "RSV F", "RSV F protein" and "F protein"), or fragment or variant thereof. The human RSV fusion protein can be conserved between RSV subtypes A and B. The RSV antigen can be a RSV F protein, or fragment or variant thereof, from the RSV Long strain (GenBank AAX23994.1). The RSV antigen can be a RSV F protein from the RSV A2 strain (GenBank AAB59858.1), or a fragment or variant thereof. The RSV antigen can be a monomer, a dimer or trimer of the RSV F protein, or a fragment or variant thereof. The RSV antigen can be an optimized amino acid RSV F amino acid sequence, or fragment or variant thereof.

The postfusion form of RSV F elicits high titer neutralizing antibodies in immunized animals and protects the animals from RSV challenge. The present invention utilizes this immunoresponse in the claimed vaccines. According to the invention, the RSV F protein can be in a prefusion form or a postfusion form.

The RSV antigen can also be human RSV attachment glycoprotein (also referred to herein as "RSV G", "RSV G protein" and "G protein"), or fragment or variant thereof. The human RSV G protein differs between RSV subtypes A and B. The antigen can be RSV G protein, or fragment or variant thereof, from the RSV Long strain (GenBank AAX23993). The RSV antigen can be RSV G protein from: the RSV subtype B isolate H5601, the RSV subtype B isolate H1068, the RSV subtype B isolate H5598, the RSV subtype B isolate H1123, or a fragment or variant thereof. The RSV antigen can be an optimized amino acid RSV G amino acid sequence, or fragment or variant thereof.

In other embodiments, the RSV antigen can be human RSV non-structural protein 1 ("NS1 protein"), or fragment or variant thereof. For example, the RSV antigen can be RSV NS1 protein, or fragment or variant thereof, from the RSV Long strain (GenBank AAX23987.1). The RSV antigen human can also be RSV non-structural protein 2 ("NS2 protein"), or fragment or variant thereof. For example, the RSV antigen can be RSV NS2 protein, or fragment or variant thereof, from the RSV Long strain (GenBank AAX23988.1). The RSV antigen can further be human RSV nucleocapsid ("N") protein, or fragment or variant thereof. For example, the RSV antigen can be RSV N protein, or fragment or variant thereof, from the RSV Long strain (GenBank AAX23989.1). The RSV antigen can be human RSV Phosphoprotein ("P") protein, or fragment or variant thereof. For example, the RSV antigen can be RSV P protein, or fragment or variant thereof, from the RSV Long strain (GenBank AAX23990.1). The RSV antigen also can be human RSV Matrix protein ("M") protein, or fragment or variant thereof. For example, the RSV antigen can be RSV M protein, or fragment or variant thereof, from the RSV Long strain (GenBank AAX23991.1).

In still other embodiments, the RSV antigen can be human RSV small hydrophobic ("SH") protein, or fragment or variant thereof. For example, the RSV antigen can be RSV SH protein, or fragment or variant thereof, from the RSV Long strain (GenBank AAX23992.1). The RSV antigen can also be human RSV Matrix protein2-1 ("M2-1") protein, or fragment or variant thereof. For example, the RSV antigen can be RSV M2-1 protein, or fragment or variant thereof, from the RSV Long strain (GenBank AAX23995.1). The RSV antigen can further be human RSV Matrix protein 2-2 ("M2-2") protein, or fragment or variant thereof. For example, the RSV antigen can be RSV M2-2 protein, or fragment or variant thereof, from the RSV Long strain (GenBank AAX23997.1). The RSV antigen human can be RSV Polymerase L ("L") protein, or fragment or variant thereof. For example, the RSV antigen can be RSV L protein, or fragment or variant thereof, from the RSV Long strain (GenBank AAX23996.1).

In further embodiments, the RSV antigen can have an optimized amino acid sequence of NS1, NS2, N, P, M, SH, M2-1, M2-2, or L protein. The RSV antigen can be a human RSV protein or recombinant antigen, such as any one of the proteins encoded by the human RSV genome.

In other embodiments, the RSV antigen can be, but is not limited to, the RSV F protein from the RSV Long strain, the RSV G protein from the RSV Long strain, the optimized amino acid RSV G amino acid sequence, the human RSV genome of the RSV Long strain, the optimized amino acid RSV F amino acid sequence, the RSV NS1 protein from the RSV Long strain, the RSV NS2 protein from the RSV Long strain, the RSV N protein from the RSV Long strain, the RSV P protein from the RSV Long strain, the RSV M protein from the RSV Long strain, the RSV SH protein from the RSV Long strain, the RSV M2-1 protein from the RSV Long strain, the RSV M2-2 protein from the RSV Long strain, the RSV L protein from the RSV Long strain, the RSV G protein from the RSV subtype B isolate H5601, the RSV G protein from the RSV subtype B isolate H1068, the RSV G protein from the RSV subtype B isolate H5598, the RSV G protein from the RSV subtype B isolate H1123, or fragment thereof, or variant thereof.

### (d) Influenza Antigen

The antigen may be an influenza antigen or fragment thereof, or variant thereof. The influenza antigens are those capable of eliciting an immune response in a mammal against one or more influenza serotypes. The antigen can comprise the full length translation product HA0, subunit HA1, subunit HA2, a variant thereof, a fragment thereof or a combination thereof. The influenza hemagglutinin antigen can be a consensus sequence derived from multiple strains of influenza A serotype H1, a consensus sequence derived from multiple strains of influenza A serotype H2, a hybrid sequence containing portions of two different consensus sequences derived from different sets of multiple strains of influenza A serotype H1 or a consensus sequence derived from multiple strains of influenza B. The influenza hemagglutinin antigen can be from influenza B.

The influenza antigen can also contain at least one antigenic epitope that can be effective against particular influenza immunogens against which an immune response can be induced. The antigen may provide an entire repertoire of immunogenic sites and epitopes present in an intact influenza virus. The antigen may be a consensus hemagglutinin antigen sequence that can be derived from hemagglutinin antigen sequences from a plurality of influenza A virus strains of one serotype such as a plurality of influenza A virus strains of serotype H1 or of serotype H2. The antigen may be a hybrid consensus hemagglutinin antigen sequence that can be derived from combining two different consensus hemagglutinin antigen sequences or portions thereof. Each of two different consensus hemagglutinin antigen sequences may be derived from a different set of a plurality of influenza A virus strains of one serotype such as a plurality of influenza A virus strains of serotype H1. The antigen may be a consensus hemagglutinin antigen sequence that can be derived from hemagglutinin antigen sequences from a plurality of influenza B virus strains.

In some embodiments, the influenza antigen can be H1 HA, H2 HA, H3 HA, H5 HA, or a BHA antigen. Alternatively, the influenza antigen can be a consensus hemagglutinin antigen comprising a consensus H1 amino acid sequence or a consensus H2 amino acid sequence. The consensus hemagglutinin antigen may be a synthetic hybrid consensus H1 sequence comprising portions of two different consensus H1 sequences, which are each derived from a different set of sequences from the other. An example of a consensus HA antigen that is a synthetic hybrid consensus H1 protein is a protein comprising the U2 amino acid sequence. The consensus hemagglutinin antigen may be a consensus hemagglutinin protein derived from hemagglutinin sequences from influenza B strains, such as a protein comprising the consensus BHA amino acid sequence.

The consensus hemagglutinin antigen may further comprise one or more additional amino acid sequence elements. The consensus hemagglutinin antigen may further comprise on its N-terminus an IgE or IgG leader amino acid sequence. The consensus hemagglutinin antigen may further comprise an immunogenic tag which is a unique immunogenic epitope that can be detected by readily available antibodies. An example of such an immunogenic tag is the 9 amino acid influenza HA Tag which may be linked on the consensus hemagglutinin C terminus. In some embodiments, consensus hemagglutinin antigen may further comprise on its N-terminus an IgE or IgG leader amino acid sequence and on its C terminus an HA tag.

The consensus hemagglutinin antigen may be a consensus hemagglutinin protein that consists of consensus influenza amino acid sequences or fragments and variants thereof. The consensus hemagglutinin antigen may be a consensus hemagglutinin protein that comprises non-influenza protein sequences and influenza protein sequences or fragments and variants thereof.

Examples of a consensus H1 protein include those that may consist of the consensus H1 amino acid sequence or those that further comprise additional elements such as an IgE leader sequence, or an HA Tag or both an IgE leader sequence and an HA Tag.

Examples of consensus H2 proteins include those that may consist of the consensus H2 amino acid sequence or those that further comprise an IgE leader sequence, or an HA Tag, or both an IgE leader sequence and an HA Tag.

Examples of hybrid consensus H1 proteins include those that may consist of the consensus U2 amino acid sequence or those that further comprise an IgE leader sequence, or an HA Tag, or both an IgE leader sequence and an HA Tag.

Examples of hybrid consensus influenza B hemagglutinin proteins include those that may consist of the consensus BHA amino acid sequence or it may comprise an IgE leader sequence, or an HA Tag, or both an IgE leader sequence and an HA Tag.

The consensus hemagglutinin protein can be encoded by a consensus hemagglutinin nucleic acid, a variant thereof or a fragment thereof. Unlike the consensus hemagglutinin protein which may be a consensus sequence derived from a plurality of different hemagglutinin sequences from different strains and variants, the consensus hemagglutinin nucleic acid refers to a nucleic acid sequence that encodes a consensus protein sequence and the coding sequences used may differ from those used to encode the particular amino acid sequences in the plurality of different hemagglutinin sequences from which the consensus hemagglutinin protein sequence is derived. The consensus nucleic acid sequence may be codon optimized and/or RNA optimized. The consensus hemagglutinin nucleic acid sequence may comprise a Kozak's sequence in the 5' untranslated region. The consensus hemagglutinin nucleic acid sequence may comprise nucleic acid sequences that encode a leader sequence. The coding sequence of an N terminal leader sequence is 5' of the hemagglutinin coding sequence. The N-terminal leader can facilitate secretion. The N-terminal leader can be an IgE leader or an IgG leader. The consensus hemagglutinin nucleic acid sequence can comprise nucleic acid sequences that encode an immunogenic tag. The immunogenic tag can be on the C terminus of the protein and the sequence encoding it is 3' of the HA coding sequence. The immunogenic tag provides a unique epitope for which there are readily available antibodies so that such antibodies can be used in assays to detect and confirm expression of the protein. The immunogenic tag can be an HA Tag at the C-terminus of the protein.

### (e) Human Immunodeficiency Virus (HIV) Antigen

The antigen may be an HIV antigen or fragment thereof, or variant thereof. HIV antigens can include modified consensus sequences for immunogens. Genetic modifications including codon optimization, RNA optimization, and the addition of a high efficient immunoglobin leader sequence to increase the immunogenicity of constructs can be included in the modified consensus sequences. The novel immunogens can be designed to elicit stronger and broader cellular immune responses than corresponding codon optimized immunogens.

In some embodiments, the HIV antigen can be a subtype A consensus envelope DNA sequence construct, an IgE leader sequence linked to a consensus sequence for Subtype A envelope protein, or a subtype A consensus Envelope protein sequence.

In other embodiments, the HIV antigen can be a subtype B consensus envelope DNA sequence construct, an IgE leader sequence linked to a consensus sequence for Subtype B envelope protein, or an subtype B consensus Envelope protein sequence.

In still other embodiments, the HIV antigen can be a subtype C consensus envelope DNA sequence construct, an IgE leader sequence linked to a consensus sequence for subtype C envelope protein, or a subtype C consensus envelope protein sequence.

In further embodiments, the HIV antigen can be a subtype D consensus envelope DNA sequence construct, an IgE leader sequence linked to a consensus sequence for Subtype D envelope protein, or a subtype D consensus envelope protein sequence.

In some embodiments, the HIV antigen can be a subtype B Nef-Rev consensus envelope DNA sequence construct, an IgE leader sequence linked to a consensus sequence for Subtype B Nef-Rev protein, or a Subtype B Nef-Rev consensus protein sequence.

In other embodiments, the HIV antigen can be a Gag consensus DNA sequence of subtype A, B, C and D DNA sequence construct, an IgE leader sequence linked to a consensus sequence for Gag consensus subtype A, B, C and D protein, or a consensus Gag subtype A, B, C and D protein sequence.

In still other embodiments, the HIV antigen can be a MPol DNA sequence or a MPol protein sequence. The HIV antigen can be nucleic acid or amino acid sequences of Env A, Env B, Env C, Env D, B Nef-Rev , Gag, or any combination thereof.

### (f) Lymphocytic Choriomeningitis Virus (LCMV) Antigen

The antigen may be an LCMV antigen or fragment thereof, or variant thereof. The LCMV antigen can comprise consensus sequences and/or one or more modifications for improved expression. Genetic modifications, including codon optimization, RNA optimization, and the addition of a highly efficient immunoglobulin leader sequence to increase the immunogenicity of constructs, can be included in the modified sequences. The LCMV antigen can comprise a signal peptide such as an immunoglobulin signal peptide (e.g., IgE or IgG signal peptide), and in some embodiments, may comprise an HA tag. The immunogens can be designed to elicit stronger and broader cellular immune responses than a corresponding codon optimized immunogen.

The LCMV antigen can be an antigen from LCMV Armstrong. The LCMV antigen can be an antigen from LCMV clone 13. The LCMV antigen can be a nucleoprotein (NP) from LCMV, a glycoprotein (GP; e.g., GP-1, GP-2, and GP-C) from LCMV, a L protein from LCMV, a Z polypeptide from LCMV, a fragment thereof, a variant thereof, or a combination thereof.

### (2) Parasite Antigens

The antigen can be a parasite antigen or fragment or variant thereof. The parasite can be a protozoa, helminth, or ectoparasite. The helminth (i.e., worm) can be a flatworm (e.g., flukes and tapeworms), a thorny-headed worm, or a round worm (e.g., pinworms). The ectoparasite can be lice, fleas, ticks, and mites.

The parasite can be any parasite causing the following diseases: Acanthamoeba keratitis, Amoebiasis, Ascariasis, Babesiosis, Balantidiasis, Baylisascariasis, Chagas disease, Clonorchiasis, Cochliomyia, Cryptosporidiosis, Diphyllobothriasis, Dracunculiasis, Echinococcosis, Elephantiasis, Enterobiasis, Fascioliasis, Fasciolopsiasis, Filariasis, Giardiasis, Gnathostomiasis, Hymenolepiasis, Isosporiasis, Katayama fever, Leishmaniasis, Lyme disease, Malaria, Metagonimiasis, Myiasis, Onchocerciasis, Pediculosis, Scabies, Schistosomiasis, Sleeping sickness, Strongyloidiasis, Taeniasis, Toxocariasis, Toxoplasmosis, Trichinosis, and Trichuriasis.

The parasite can be Acanthamoeba, Anisakis, Ascaris lumbricoides, Botfly, Balantidium coli, Bedbug, Cestoda (tapeworm), Chiggers, Cochliomyia hominivorax, Entamoeba histolytica, Fasciola hepatica, Giardia lamblia, Hookworm, Leishmania, Linguatula serrata, Liver fluke, Loa loa, Paragonimus - lung fluke, Pinworm, Plasmodium falciparum, Schistosoma, Strongyloides stercoralis, Mite, Tapeworm, Toxoplasma gondii, Trypanosoma, Whipworm, or Wuchereria bancrofti.

### (a) Malaria Antigen

The antigen may be a malaria antigen (i.e., PF antigen or PF immunogen), or fragment thereof, or variant thereof. The antigen can be from a parasite causing malaria. The malaria causing parasite can be Plasmodium falciparum. The Plasmodium falciparum antigen can include the circumsporozoite (CS) antigen.

In some embodiments, the malaria antigen can be nucleic acid molecules such as plasmids which encode one or more of the *P. falciparum* immunogens CS, LSA1, TRAP, CelTOS, and Ama1. The immunogens may be full length or immunogenic fragments of full length proteins. The immunogens comprise consensus sequences and/or modifications for improved expression.

In other embodiments, the malaria antigen can be a consensus sequence of TRAP, which is also referred to as SSP2, designed from a compilation of all full-length *Plasmodium falciparum* TRAP/SSP2 sequences in the GenBank database (28 sequences total).Consensus TRAP immunogens (i.e., ConTRAP immunogen) may comprise a signal peptide such as an immunoglobulin signal peptide such as an IgE or IgG signal peptide and in some embodiments, may comprise an HA tag.

In still other embodiments, the malaria antigen can be CelTOS, which is also referred to as Ag2 and is a highly conserved *Plasmodium* antigen. Consensus CelTOS antigens (i.e., ConCelTOS immunogen) may comprise a signal peptide such as an immunoglobulin signal peptide such as an IgE or IgG signal peptide and in some embodiments, may comprise an HA tag.

In further embodiments, the malaria antigen can be Ama1, which is a highly conserved *Plasmodium* antigen. The malaria antigen can also be a consensus sequence of Ama1 (i.e., ConAmaI immunogen) comprising in some instances, a signal peptide such as an immunoglobulin signal peptide such as an IgE or IgG signal peptide and in some embodiments, may comprise an HA tag.

In some embodiments, the malaria antigen can be a consensus CS antigen (i.e., Consensus CS immunogen) comprising in some instances, a signal peptide such as an immunoglobulin signal peptide such as an IgE or IgG signal peptide and in some embodiments, may comprise an HA tag.

In other embodiments, the malaria antigen can be a fusion protein comprising a combination of two or more of the PF proteins set forth herein. For example, fusion proteins may comprise two or more of Consensus CS immunogen, ConLSA1 immunogen, ConTRAP immunogen, ConCelTOS immunogen and ConAma1 immunogen linked directly adjacent to each other or linked with a spacer or one or more amino acids in between. In some embodiments, the fusion protein comprises two PF immunogens; in some embodiments the fusion protein comprises three PF immunogens; in some embodiments the fusion protein comprises four PF immunogens; and in some embodiments the fusion protein comprises five PF immunogens. Fusion proteins with two Consensus PF immunogens may comprise: CS and LSA1; CS and TRAP; CS and CelTOS; CS and Amal; LSA1 and TRAP; LSA1 and CelTOS; LSA1 and Amal; TRAP and CelTOS; TRAP and Amal; or CelTOS and Ama1. Fusion proteins with three Consensus PF immunogens may comprise: CS, LSA1 and TRAP; CS, LSA1 and CelTOS; CS, LSA1 and Amal; LSA1, TRAP and CelTOS; LSA1, TRAP and Amal; or TRAP, CelTOS and Ama1. Fusion proteins with four Consensus PF immunogens may comprise: CS, LSA1, TRAP and CelTOS; CS, LSA1, TRAP and Amal; CS, LSA1, CelTOS and Amal; CS, TRAP, CelTOS and Amal; or LSA1, TRAP, CelTOS and Ama1. Fusion proteins with five Consensus PF immunogens may comprise CS or CS-alt, LSA1, TRAP, CelTOS and Ama1.

In some embodiments, the fusion proteins comprise a signal peptide linked to the N terminus. In some embodiments, the fusion proteins comprise multiple signal peptides linked to the N terminus of each Consensus PF immunogen. In some embodiments, a spacer may be included between PF immunogens of a fusion protein. In some embodiments, the spacer between PF immunogens of a fusion protein may be a proteolyic cleavage site. In some embodiments, the spacer may be a proteolyic cleavage site recognized by a protease found in cells to which the vaccine is intended to be administered and/or taken up. In some embodiments, a spacer may be included between PF immunogens of a fusion protein, wherein the spacer is a proteolyic cleavage site recognized by a protease found in cells to which the vaccine is intended to be administered and/or taken up and the fusion protein comprises multiple signal peptides linked to the N terminus of each Consensus PF immunogens such that upon cleavage, the signal peptide of each Consensus PF immunogen translocates the Consensus PF immunogen to outside the cell.

### (3) Bacterial Antigens

The antigen can be a bacterial antigen or fragment or variant thereof. The bacterium can be from any one of the following phyla: Acidobacteria, Actinobacteria, Aquificae, Bacteroidetes, Caldiserica, Chlamydiae, Chlorobi, Chloroflexi, Chrysiogenetes, Cyanobacteria, Deferribacteres, Deinococcus-Thermus, Dictyoglomi, Elusimicrobia, Fibrobacteres, Firmicutes, Fusobacteria, Gemmatimonadetes, Lentisphaerae, Nitrospira, Planctomycetes, Proteobacteria, Spirochaetes, Synergistetes, Tenericutes, Thermodesulfobacteria, Thermotogae, and Verrucomicrobia.

The bacterium can be a gram positive bacterium or a gram negative bacterium. The bacterium can be an aerobic bacterium or an anerobic bacterium. The bacterium can be an autotrophic bacterium or a heterotrophic bacterium. The bacterium can be a mesophile, a neutrophile, an extremophile, an acidophile, an alkaliphile, a thermophile, a psychrophile, an halophile, or an osmophile.

The bacterium can be an anthrax bacterium, an antibiotic resistant bacterium, a disease causing bacterium, a food poisoning bacterium, an infectious bacterium, Salmonella bacterium, Staphylococcus bacterium, Streptococcus bacterium, or tetanus bacterium. The bacterium can be a mycobacteria, *Clostridium tetani, Yersinia pestis, Bacillus anthracis,* methicillin-resistant *Staphylococcus aureus* (MRSA), or *Clostridium difficile.* The bacterium can be *Mycobacterium tuberculosis.*

### (a) Mycobacterium tuberculosis Antigens

The antigen may be *a Mycobacterium tuberculosis* antigen (i.e., TB antigen or TB immunogen), or fragment thereof, or variant thereof. The TB antigen can be from the Ag85 family of TB antigens, for example, Ag85A and Ag85B. The TB antigen can be from the Esx family of TB antigens, for example, EsxA, EsxB, EsxC, EsxD, EsxE, EsxF, EsxH, EsxO, EsxQ, EsxR, EsxS, EsxT, EsxU, EsxV, and EsxW.

In some embodiments, the TB antigen can be nucleic acid molecules such as plasmids which encode one or more of the *Mycobacterium tuberculosis* immunogens from the Ag85 family and the Esx family. The immunogens can be full-length or immunogenic fragments of full-length proteins. The immunogens can comprise consensus sequences and/or modifications for improved expression. Consensus immunogens may comprise a signal peptide such as an immunoglobulin signal peptide such as an IgE or IgG signal peptide and in some embodiments, may comprise an HA tag.

### (4) Fungal Antigens

The antigen can be a fungal antigen or fragment or variant thereof. The fungus can be Aspergillus species, Blastomyces dermatitidis, *Candida* yeasts (e.g., *Candida albicans*), *Coccidioides, Cryptococcus neoformans, Cryptococcus gattii, dermatophyte, Fusarium species, Histoplasma capsulatum, Mucoromycotina, Pneumocystis jirovecii, Sporothrix schenckii, Exserohilum, or Cladosporium.*

### b) Administration

The chondroitinase and agent can be formulated in accordance with standard techniques well known to those skilled in the pharmaceutical art. Chondroitinase and agent may be comprised in the same or separate compositions. The hyaluronidase and agent can be formulated in accordance with standard techniques well known to those skilled in the pharmaceutical art. Hyaluronidase and agent may be comprised in the same or separate compositions. Such compositions can be administered in dosages and by techniques well known to those skilled in the medical arts taking into consideration such factors as the age, sex, weight, and condition of the particular subject, and the route of administration.

The chondroitinase and agent can be administered prophylactically or therapeutically. In prophylactic administration, the chondroitinase and agent can be administered in an amount sufficient to induce an immune response. In therapeutic applications, the chondroitinase and agent are administered to a subject in need thereof in an amount sufficient to elicit a therapeutic effect. The hyaluronidase and agent can be administered prophylactically or therapeutically. In prophylactic administration, the hyaluronidase and agent can be administered in an amount sufficient to induce an immune response. In therapeutic applications, the hyaluronidase and agent are administered to a subject in need thereof in an amount sufficient to elicit a therapeutic effect. An amount adequate to accomplish this is defined as a "therapeutically effective dose." Amounts effective for this use will depend on, e.g., the particular composition of the vaccine regimen administered, the manner of administration, the stage and severity of the disease, the general state of health of the patient, and the judgment of the prescribing physician.

The chondroitinase and agent, or the hyaluronidase and agent, for example, can be administered by methods well known in the art as described in Donnelly et al. (Ann. Rev. Immunol. 1997, 15, 617-648); U.S. Patent No. 5,580,859; U.S. Patent No. 5,703,055; and U.S. Patent No. 5,679,647. The polynucleotide can be complexed to particles or beads that can be administered to an individual, for example, using a vaccine gun. One skilled in the art would know that the choice of a pharmaceutically acceptable carrier, including a physiologically acceptable compound, depends, for example, on the route of administration of the expression vector.

The chondroitinase and agent can be delivered via a variety of routes. The hyaluronidase and agent can be delivered via a variety of routes. Typical delivery routes include parenteral administration, e.g., intradermal, intramuscular, adipose tissue delivery, or subcutaneous delivery. Other routes include oral administration, intranasal, and intravaginal routes. For a polynucleotide in particular, the chondroitinase and agent, or the hyaluronidase and agent, can be delivered to the interstitial spaces of tissues of an individual (U.S. Patent Nos. 5,580,859 and 5,703,055). The chondroitinase and agent, or the hyaluronidase and agent, can also be administered to muscle, or can be administered via intradermal or subcutaneous injections, or transdermally, such as by iontophoresis. Epidermal administration can also be employed. Epidermal administration can involve mechanically or chemically irritating the outermost layer of epidermis to stimulate an immune response to the irritant (U.S. Patent No. 5,679,647).

The chondroitinase and agent can be a liquid preparation such as a suspension, syrup or elixir. The chondroitinase and agent can also be a preparation for parenteral, subcutaneous, adipose tissue, intradermal, intramuscular or intravenous administration (e.g., injectable administration), such as a sterile suspension or emulsion. The hyaluronidase and agent can be a liquid preparation such as a suspension, syrup or elixir. The hyaluronidase and agent can also be a preparation for parenteral, subcutaneous, adipose tissue, intradermal, intramuscular or intravenous administration (e.g., injectable administration), such as a sterile suspension or emulsion.

The chondroitinase and agent, or the hyaluronidase and agent, can be incorporated into liposomes, microspheres or other polymer matrices (U.S. Patent No. 5,703,055; Gregoriadis, Liposome Technology, Vols. I to III, 2nd ed. 1993). Liposomes can consist of phospholipids or other lipids, and can be nontoxic, physiologically acceptable and metabolizable carriers that are relatively simple to make and administer.

The chondroitinase and agent, or the hyaluronidase and agent, can be administered via electroporation, such as by a method described in U.S. Patent No. 7,664,545. In the invention, the DNA plasmid is administered via electroporation. The electroporation can be by a method and/or apparatus described in U.S. Patent Nos. 6,302,874; 5,676,646; 6,241,701; 6,233,482; 6,216,034; 6,208,893; 6,192,270; 6,181,964; 6,150,148; 6,120,493; 6,096,020; 6,068,650; and 5,702,359. The electroporation may be carried out via a minimally invasive device.

The minimally invasive electroporation device ("MID") may be an apparatus for injecting the chondroitinase and agent described above and associated fluid into body tissue. The device may comprise a hollow needle, DNA cassette, and fluid delivery means, wherein the device is adapted to actuate the fluid delivery means in use so as to concurrently (for example, automatically) inject DNA into body tissue during insertion of the needle into the said body tissue. This has the advantage that the ability to inject the DNA and associated fluid gradually while the needle is being inserted leads to a more even distribution of the fluid through the body tissue. The pain experienced during injection may be reduced due to the distribution of the DNA being injected over a larger area.

The MID may inject the chondroitinase and agent into tissue without the use of a needle. The MID may inject the vaccine as a small stream or jet with such force that the vaccine pierces the surface of the tissue and enters the underlying tissue and/or muscle. The force behind the small stream or jet may be provided by expansion of a compressed gas, such as carbon dioxide through a micro-orifice within a fraction of a second. Examples of minimally invasive electroporation devices, and methods of using them, are described in published U.S. Patent Application No. 20080234655; U.S. Patent No. 6,520,950; U.S. Patent No. 7,171,264; U.S. Patent No. 6,208,893; U.S. Patent No. 6,009,347; U.S. Patent No. 6,120,493; U.S. Patent No. 7,245,963; U.S. Patent No. 7,328,064; and U.S. Patent No. 6,763,264.

The MID may comprise an injector that creates a high-speed jet of liquid that painlessly pierces the tissue. Such needle-free injectors are commercially available. Examples of needle-free injectors that can be utilized herein include those described in U.S. Patent Nos. 3,805,783; 4,447,223; 5,505,697; and 4,342,310.

A desired chondroitinase and agent, hyaluronidase and agent, in a form suitable for direct or indirect electrotransport may be introduced (e.g., injected) using a needle-free injector into the tissue to be treated, usually by contacting the tissue surface with the injector so as to actuate delivery of a jet of the agent, with sufficient force to cause penetration into the tissue. For example, if the tissue to be treated is mucosa, skin or muscle, the agent is projected towards the mucosal or skin surface with sufficient force to cause the agent to penetrate through the stratum corneum and into dermal layers, or into underlying tissue and muscle, respectively.

Needle-free injectors are well suited to deliver to all types of tissues, particularly to skin and mucosa. In some embodiments, a needle-free injector may be used to propel a liquid that contains the chondroitinase and agent to the surface and into the subject's skin or mucosa. Representative examples of the various types of tissues that can be treated using the invention methods include pancreas, larynx, nasopharynx, hypopharynx, oropharynx, lip, throat, lung, heart, kidney, muscle, breast, colon, prostate, thymus, testis, skin, mucosal tissue, ovary, blood vessels, or any combination thereof.

The MID may have needle electrodes that electroporate the tissue. By pulsing between multiple pairs of electrodes in a multiple electrode array, for example set up in rectangular or square patterns, provides improved results over that of pulsing between a pair of electrodes. Disclosed, for example, in U.S. Patent No. 5,702,359 entitled "Needle Electrodes for Mediated Delivery of Drugs and Genes" is an array of needles wherein a plurality of pairs of needles may be pulsed during the therapeutic treatment. In that application, needles were disposed in a circular array, but have connectors and switching apparatus enabling a pulsing between opposing pairs of needle electrodes. A pair of needle electrodes for delivering recombinant expression vectors to cells may be used. Such a device and system is described in U.S. Patent No. 6,763,264. Alternatively, a single needle device may be used that allows injection of the DNA and electroporation with a single needle resembling a normal injection needle and applies pulses of lower voltage than those delivered by presently used devices, thus reducing the electrical sensation experienced by the patient.

The MID may comprise one or more electrode arrays. The arrays may comprise two or more needles of the same diameter or different diameters. The needles may be evenly or unevenly spaced apart. The needles may be between 0.005 inches and 0.03 inches, between 0.01 inches and 0.025 inches; or between 0.015 inches and 0.020 inches. The needle may be 0.0175 inches in diameter. The needles may be 0.5 mm, 1.0 mm, 1.5 mm, 2.0 mm, 2.5 mm, 3.0 mm, 3.5 mm, 4.0 mm, or more spaced apart.

The MID may consist of a pulse generator and a two or more-needle vaccine injectors that deliver the vaccine and electroporation pulses in a single step. The pulse generator may allow for flexible programming of pulse and injection parameters via a flash card operated personal computer, as well as comprehensive recording and storage of electroporation and patient data. The pulse generator may deliver a variety of volt pulses during short periods of time. For example, the pulse generator may deliver three 15 volt pulses of 100 ms in duration. An example of such a MID is the Elgen 1000 system by Inovio Biomedical Corporation, which is described in U.S. Patent No. 7,328,064.

The MID may be a CELLECTRA (Inovio Pharmaceuticals, Plymouth Meeting, PA) device and system, which is a modular electrode system, that facilitates the introduction of a macromolecule, such as a DNA, into cells of a selected tissue in a body or plant. The modular electrode system may comprise a plurality of needle electrodes; a hypodermic needle; an electrical connector that provides a conductive link from a programmable constant-current pulse controller to the plurality of needle electrodes; and a power source. An operator can grasp the plurality of needle electrodes that are mounted on a support structure and firmly insert them into the selected tissue in a body or plant. The macromolecules are then delivered via the hypodermic needle into the selected tissue. The programmable constant-current pulse controller is activated and constant-current electrical pulse is applied to the plurality of needle electrodes. The applied constant-current electrical pulse facilitates the introduction of the macromolecule into the cell between the plurality of electrodes. Cell death due to overheating of cells is minimized by limiting the power dissipation in the tissue by virtue of constant-current pulses. The CELLECTRA device and system is described in U.S. Patent No. 7,245,963.

The MID may be an Elgen 1000 system (Inovio Pharmaceuticals). The Elgen 1000 system may comprise device that provides a hollow needle; and fluid delivery means, wherein the apparatus is adapted to actuate the fluid delivery means in use so as to concurrently (for example automatically) inject fluid, the described vaccine herein, into body tissue during insertion of the needle into the said body tissue. The advantage is the ability to inject the fluid gradually while the needle is being inserted leads to a more even distribution of the fluid through the body tissue. It is also believed that the pain experienced during injection is reduced due to the distribution of the volume of fluid being injected over a larger area.

In addition, the automatic injection of fluid facilitates automatic monitoring and registration of an actual dose of fluid injected. This data can be stored by a control unit for documentation purposes if desired.

It will be appreciated that the rate of injection could be either linear or nonlinear and that the injection may be carried out after the needles have been inserted through the skin of the subject to be treated and while they are inserted further into the body tissue.

Suitable tissues into which fluid may be injected include tumor tissue, skin or liver tissue but may be muscle tissue.

An apparatus for administration may further comprise needle insertion means for guiding insertion of the needle into the body tissue. The rate of fluid injection is controlled by the rate of needle insertion. This has the advantage that both the needle insertion and injection of fluid can be controlled such that the rate of insertion can be matched to the rate of injection as desired. It also makes the apparatus easier for a user to operate. If desired means for automatically inserting the needle into body tissue could be provided.

A user could choose when to commence injection of fluid. Ideally however, injection is commenced when the tip of the needle has reached muscle tissue and the apparatus may include means for sensing when the needle has been inserted to a sufficient depth for injection of the fluid to commence. This means that injection of fluid can be prompted to commence automatically when the needle has reached a desired depth (which will normally be the depth at which muscle tissue begins). The depth at which muscle tissue begins could for example be taken to be a preset needle insertion depth such as a value of 4 mm which would be deemed sufficient for the needle to get through the skin layer.

The sensing means may comprise an ultrasound probe. The sensing means may comprise a means for sensing a change in impedance or resistance. In this case, the means may not as such record the depth of the needle in the body tissue but will rather be adapted to sense a change in impedance or resistance as the needle moves from a different type of body tissue into muscle. Either of these alternatives provides a relatively accurate and simple to operate means of sensing that injection may commence. The depth of insertion of the needle can further be recorded if desired and could be used to control injection of fluid such that the volume of fluid to be injected is determined as the depth of needle insertion is being recorded.

An administration apparatus may further comprise: a base for supporting the needle; and a housing for receiving the base therein, wherein the base is moveable relative to the housing such that the needle is retracted within the housing when the base is in a first rearward position relative to the housing and the needle extends out of the housing when the base is in a second forward position within the housing. This is advantageous for a user as the housing can be lined up on the skin of a patient, and the needles can then be inserted into the patient's skin by moving the housing relative to the base.

As stated above, it is desirable to achieve a controlled rate of fluid injection such that the fluid is evenly distributed over the length of the needle as it is inserted into the skin. The fluid delivery means may comprise piston driving means adapted to inject fluid at a controlled rate. The piston driving means could for example be activated by a servo motor. However, the piston driving means may be actuated by the base being moved in the axial direction relative to the housing. It will be appreciated that alternative means for fluid delivery could be provided. Thus, for example, a closed container which can be squeezed for fluid delivery at a controlled or non-controlled rate could be provided in the place of a syringe and piston system.

Any type of injection may be used. It is however envisaged to be particularly useful in the field of electroporation and so it may further comprise means for applying a voltage to the needle. This allows the needle to be used not only for injection but also as an electrode during electroporation. This is particularly advantageous as it means that the electric field is applied to the same area as the injected fluid. There has traditionally been a problem with electroporation in that it is very difficult to accurately align an electrode with previously injected fluid and so users have tended to inject a larger volume of fluid than is required over a larger area and to apply an electric field over a higher area to attempt to guarantee an overlap between the injected substance and the electric field. Using the present invention, both the volume of fluid injected and the size of electric field applied may be reduced while achieving a good fit between the electric field and the fluid.

Administration of the chondroitinase polypeptide or the polynucleotide encoding the chondroitinase polypeptide and the agent may be concurrent or consecutive.

### i) Concurrent Administration

The chondroitinase polypeptide or the polynucleotide encoding the chondroitinase polypeptide and the agent may be administered to the subject concurrently. As used herein, "concurrently" and "simultaneously" are used interchangeably. For example, the chondroitinase polypeptide or the polynucleotide encoding the chondroitinase polypeptide and the agent may be co-formulated. When co-formulated, the chondroitinase polypeptide or the polynucleotide encoding the chondroitinase polypeptide and the agent may be administered to a subject in a single step. When co-formulated, the chondroitinase polypeptide or the polynucleotide encoding the chondroitinase polypeptide and the agent may be administered to a subject in a single injection step, for example.

The hyaluronidase polypeptide or the polynucleotide encoding the hyaluronidase polypeptide and the agent may be administered to the subject concurrently. As used herein, "concurrently" and "simultaneously" are used interchangeably. For example, the hyaluronidase polypeptide or the polynucleotide encoding the hyaluronidase polypeptide and the agent may be co-formulated. When co-formulated, the hyaluronidase polypeptide or the polynucleotide encoding the hyaluronidase polypeptide and the agent may be administered to a subject in a single step. When co-formulated, the hyaluronidase polypeptide or the polynucleotide encoding the hyaluronidase polypeptide and the agent may be administered to a subject in a single injection step, for example.

### ii) Consecutive Administration

The chondroitinase polypeptide or the polynucleotide encoding the chondroitinase polypeptide and the agent may be administered to the subject consecutively. As used herein, "consecutively" and "staggered over time" are used interchangeably. In some embodiments, the chondroitinase polypeptide or the polynucleotide encoding the chondroitinase polypeptide is administered to the subject prior to administration of the agent. The hyaluronidase polypeptide or the polynucleotide encoding the hyaluronidase polypeptide and the agent may be administered to the subject consecutively. As used herein, "consecutively" and "staggered over time" are used interchangeably. In some embodiments, the hyaluronidase polypeptide or the polynucleotide encoding the hyaluronidase polypeptide is administered to the subject prior to administration of the agent. The hyaluronidase polypeptide or the polynucleotide encoding the hyaluronidase polypeptide and/or the chrondroitinase polypeptide or the polynucleotide encoding the chrondroitinase polypeptide may be administered to the subject at least about 3 minutes, 5 minutes, 15 minutes, at least about 20 minutes, at least about 25 minutes, at least about 30 minutes, at least about 35 minutes, at least about 40 minutes, at least about 45 minutes, at least about 50 minutes, at least about 55 minutes, at least about 1 hour, at least about 1.5 hours, at least about 2 hours, at least about 2.5 hours, at least about 3 hours, at least about 3.5 hours, at least about 4 hours, at least about 4.5 hours, at least about 5 hours, at least about 5.5 hours, at least about 6 hours, at least about 7 hours, at least about 8 hours, at least about 9 hours, at least about 10 hours, at least about 11 hours, at least about 12 hours, at least about 15 hours, at least about 18 hours, at least about 21 hours, or at least about 24 hours prior to administration of the agent. The hyaluronidase polypeptide or the polynucleotide encoding the hyaluronidase polypeptide and/or the chrondroitinase polypeptide or the polynucleotide encoding the chrondroitinase polypeptide may be administered to the subject less than about 24 hours, less than about 21 hours, less than about 18 hours, less than about 15 hours, less than about 12 hours, less than about 11 hours, less than about 10 hours, less than about 9 hours, less than about 8 hours, less than about 7 hours, less than about 6 hours, less than about 5.5 hours, less than about 5 hours, less than about 4.5 hours, less than about 4 hours, less than about 3.5 hours, less than about 3 hours, less than about 2.5 hours, less than about 2 hours, less than about 1.5 hours, less than about 1 hour, less than about 55 minutes, less than about 50 minutes, less than about 45 minutes, less than about 40 minutes, less than about 35 minutes, less than about 30 minutes, less than about 25 minutes, less than about 20 minutes, less than about 10 minutes, less than about 5 minutes, or less than about 15 minutes prior to administration of the agent. The hyaluronidase polypeptide or the polynucleotide encoding the hyaluronidase polypeptide and/or the chrondroitinase polypeptide or the polynucleotide encoding the chrondroitinase polypeptide may be administered to the subject at least about 5 minutes to about 24 hours prior to administration of the agent.

### c) Methods

### i) Method of Delivering an Agent

The present invention is also directed to a method of delivering an agent to a subject. The methods may comprise administering to the subject a chondroitinase polypeptide or a polynucleotide encoding a chondroitinase polypeptide in an amount sufficient to degrade glycosaminoglycans, and administering to the subject the agent. The methods may further comprise administering to the subject a polynucleotide encoding an antigen.

The present invention is also directed to a method of delivering an agent to a subject. The methods may comprise administering to the subject a hyaluronidase polypeptide or a polynucleotide encoding a hyaluronidase polypeptide in an amount sufficient to degrade glycosaminoglycans, and administering to the subject the agent. The methods may further comprise administering to the subject a polynucleotide encoding an antigen.

### ii) Method of Treating a Disease or Disorder

The present invention is also directed to a method of treating a disease or disorder in a subject. The methods may comprise administering to the subject a chondroitinase polypeptide or a polynucleotide encoding a chondroitinase polypeptide, and administering to the subject an agent. The methods may comprise administering to the subject a hyaluronidase polypeptide or a polynucleotide encoding a hyaluronidase polypeptide, and administering to the subject an agent.

Diseases may include, but are not limited to, cardiovascular disease such as coronary heart disease, atherosclerosis, hypertension, cardiac hypertrophy, myocardial infarction, ventricular or atrial fibrillation, and cardiomyopathy; neurological disease such as neuropathy or neurodegenerative disease; metabolic disease such as diabetes; inflammatory disorders including inflammatory bowel disease such as Crohn's disease and ulcerative colitis; dermatological disorders; autoimmune disease such as Alzheimer's disease, multiple sclerosis, psoriasis, systemic dermatomyositis, deterioration of immune responses to antigens, atherosclerosis, rheumatoid arthritis, and lupus erythematosus; osteoporosis; osteoarthritis; diseases caused by viral, bacterial, parasitic, or fungal infections; and cancer. The viral disease may be Middle East Respiratory Syndrome, for example. The subject administered the agent and chondroitinase can have an increased or boosted immune response as compared to the subject administered the agent. The increased immune response can be used to treat and/or prevent disease in the subject.

The disease can be cancer, for example, an HPV-associated cancer, HBV-associated cancer, ovarian cancer, prostate cancer, breast cancer, brain cancer, head and neck cancer, throat cancer, lung cancer, liver cancer, cancer of the pancreas, kidney cancer, bone cancer, melanoma, metastatic cancer, hTERT-associated cancer, FAP-antigen associated cancer, non-small cell lung cancer, blood cancer, esophageal squamous cell carcinoma, cervical cancer, bladder cancer, colorectal cancer, gastric cancer, anal cancer, synovial carcinoma, testicular cancer, recurrent respiratory papillomatosis, skin cancer, glioblastoma, hepatocarcinoma, stomach cancer, acute myeloid leukemia, triple-negative breast cancer, and primary cutaneous T cell lymphoma.

The method can further include reducing the size of an established tumor or lesion in the subject. The tumor can be reduced in size by about 50% to about 100%, about 60% to about 100%, about 70% to about 100%, about 80% to about 100%, about 90% to about 100%, about 50% to about 95%, about 60% to about 95%, about 70% to about 95%, about 80% to about 95%, about 90% to about 95%, about 50% to about 90%, about 60% to about 90%, about 70% to about 90%, or about 80% to about 90%, compared to administering the agent without chondroitinase and/or hyaluronidase, for example. The tumor can be reduced in size by about 80%, by about 81%, by about 82%, by about 83%, by about 84%, by about 85%, by about 86%, by about 87%, by about 88%, by about 89%, by about 90%, by about 91%, by about 92%, by about 93%, by about 94%, by about 95%, by about 96%, by about 97%,by about 98%, by about 99%, or by about 100%, compared to administering the agent without chondroitinase and/or hyaluronidase, for example.

The method can further include increasing tumor regression in the subject as compared to the subject administered the agent without chondroitinase and/or hyaluronidase. Administration of the agent with chondroitinase and/or hyaluronidase can increase tumor regression by about 40% to about 60%, about 45% to about 55%, or about 50%, compared to administering the agent without chondroitinase and/or hyaluronidase, for example. Administration of the agent with chondroitinase and/or hyaluridase can also increase the rate of tumor regression. Administration of the agent with chondroitinase and/or hyaluronidase can further achieve tumor regression in the subject of about 80% to about 100%, about 85% to about 100%, about 90% to about 100%, about 95% to about 100%, about 80% to about 95%, about 85% to about 95%, about 90% to about 95%, about 80% to about 90%, or about 85% to about 90%, compared to administering the agent without chondroitinase and/or hyaluronidase. Tumor regression can be about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% in the subject administered the agent with chondroitinase and/or hyaluronidase, compared to administering the agent alone. Tumor regression in the subject administered the agent with chondroitinase and/or hyaluronidase can further be about 90% or about 100%.

The method can further include preventing cancer or tumor growth in the subject administered the subject administered the agent with chondroitinase. This prevention can allow the subject administered the agent with chondroitinase to survive a future cancer. In other words, the agent with chondroitinase affords protection against cancer to the subject administered the agent with chondroitinase. The subject administered the agent with chondroitinase can have about 90% to about 100% survival of cancer, compared to administering the agent alone. The subject administered the agent with chondroitinase can have about 90%, about 91%, about 92%, about 93%, about 94%, about 95%, about 96%, about 97%, about 98%, about 99%, or about 100% survival of cancer, compared to administering the agent alone.

The present invention has multiple aspects, illustrated by the following nonlimiting examples.

### 5) Examples

### EXAMPLE 1

### Methods for Examples 2 and 3

A total of 20 mice for either study INO-16-056 (Balb/c) or INO-16-057 (C57BL/6) were divided into four groups of 5 female mice (6-7 weeks old) and were immunized with pGX9214, a DNA plasmid encoding human antibody (DMAb) reactive to Pseudomonas aeruginosa.

The left TA muscle of each mouse in experimental group 2 (see table 1) was pretreated with 30 µl hyaluronidase (400 Units/ml; purified from bovine testes) 30 min before pGX9214 delivery. Mice of group 3 received pretreatments with 30 µl Chondroitinase AC from Flavibacterium heparinum and mice of group 4 were given pretreatments containing a combination of hyaluronidase and chondroitinase at the same concentrations into their left TA muscle followed by pGX9214 plus EP after 30 min. Mice of the control group (group 1) were injected with PBS before DNA delivery and EP.

The plasmid DNA was administrated at a concentration of 0.1 mg in 30 µl SSC intramuscularly at the sites that received enzyme pretreatment (group 2, 3 and 4) or nonpretreated sites (group 1), and electroporation was performed immediately after injection. Electroporation was applied to the site of injection using CELLECTRA^{®}-3P (2 mm electrodes) device (Inovio Pharmaceuticals, Inc.). Parameters were:
- Number of pulses = 2 sets of 2 pulses (2x2),
- Current Strength = 0.1 Amp,
- Maximum Voltage = 200V,
- Electroporation pulse duration = 52 milliseconds,
- Interval separating pulses = 0.2 seconds between pulses, and 3 seconds between each set of pulses.

On days 0, 3, 7 mice were bled (50 µl) and the levels of human IgG kappa (DMAb PseudoV2L2MD) were measured in the serum by ELISA.

For analysis of humoral responses against hIgG, an ELISA against the hIgG kappa standard protein was performed.

### EXAMPLE 2

### Chondroitinase Treatment of Muscle Prior to pMAb Vaccine Delivery - Balb/c

The level of expression of DNA-encoded monoclonal antibody in the serum of Balb/c mice treated with pGX9214 (Pseudo V2L2MD) was determined. Pretreatment of the muscle delivery site with Chondroitinase AC from Flavobacterium heparinum, similar to hyaluronidase derived from bovine testis, significantly enhances systemic serum levels of hIgG detected.

**Table 1: Experimental Details for Example 2**

| **Group Number (n/group)** | **Animal Eartag #** | **PreTreatment** | **Plasmid** | **# of Injection Site(s) / Location / Tx** | **EP Device & Inj Method** | **Injection Volume (uL)** | **DNA dose per site (mg)** | **Total DNA dose/plas mid (mg)** |
|---|---|---|---|---|---|---|---|---|
| **1. 5** | 831-835 | PBS | pGX9214 | 1/TA, left site | CELL., 3P | 30 | 0.1 | 0.1 |
| **2. 5** | 836-840 | *Hyaluroni dase (bovine testes) | pGX9214 | 1/TA, left site | CELL., 3P | 30 | 0.1 | 0.1 |
| **3. 5** | 841-845 | **Chondroi tinase AC *(F. Heparinum* ) | pGX9214 | 1/TA, left site | CELL., 3P | 30 | 0.1 | 0.1 |
| **4. 5** | 846-850 | Chondroiti nase AC *(F. Heparinum* )⁺ Hyaluronid ase (bovine testes) | pGX9214 | 1/TA, left site | CELL., 3P | 30 | 0.1 | 0.1 |

| | | | | | | | | |
|---|---|---|---|---|---|---|---|---|
| * Hyaluronidase: Sigma Aldrich; catalog #H4272 - 30 mg; 400U/ml; 4µl/100µl PBS. ** Chondroitinase; Sigma Aldrich; catalog # C2780; 0.5U/ml; dilute 1: 10 from stock in PBS. | | | | | | | | |

Treatment of enzymes was performed 30 minutes before DMAb delivery.

**Table 2: Plasmid Details for Example 2**

| **Code** | **Name** | **R&D Lot#** | **Mfg. Date** |
|---|---|---|---|
| pGX9214 | Pseudo V2L2MD | D150827 | 9/1/2015 |

### EXAMPLE 3

### Chondroitinase Treatment of Muscle Prior to pDNA Vaccine Delivery - C57BL/6

Similar to Balb/c mice, C57BL/6 mice treated with chondroitinase and pGX9214 show an increased level of expression of DNA-encoded monoclonal antibody in their serum in comparison to mice that received pGX9214 plus PBS only. The hIgG levels were comparable to mice pretreated with hyaluronidase before DNA injection and EP, thus showing that pretreatment of mouse TA muscle delivery site with chondroitinase significantly enhances systemic serum levels of hIgG. See Figure 2.

**Table 3: Experimental Details for Example 3**

| **Group Number (n/group)** | **Animal Eartag #** | **Pre-Treatmen t** | **Plasmid** | **# of Injection Site(s) / Location / Tx** | **EP Device & Inj Method** | **Injection Volume (uL)** | **DNA dose per site (mg)** | **Total DNA dose/plas mid (mg)** |
|---|---|---|---|---|---|---|---|---|
| **1.5** | 876-880 | PBS | pGX9214 | 1/TA, left site | CELL., 3P | 30 | 0.1 | 0.1 |
| **2. 5** | 881-885 | *Hyaluron idase (bovine testes) | pGX9214 | 1/TA, left site | CELL., 3P | 30 | 0.1 | 0.1 |
| **3.5** | 886-890 | **Chondr oitinase AC (F. *Heparinu* m) | pGX9214 | 1/TA, left site | CELL., 3P | 30 | 0.1 | 0.1 |
| **4. 5** | 891-895 | Chondroiti nase AC *(F. Heparinu* m) + Hyaluroni dase (bovine testes) | pGX9214 | 1/TA, left site | CELL., 3P | 30 | 0.1 | 0.1 |

| | | | | | | | | |
|---|---|---|---|---|---|---|---|---|
| * Hyaluronidase: Sigma Aldrich; catalog #H4272 - 30 mg; 400U/ml; 4µl/100µl PBS. * * Chondroitinase; Sigma Aldrich; catalog # C2780; 0.5U/ml; dilute 1: 10 from stock in PBS. | | | | | | | | |

Treatment of enzymes was performed 30 minutes before DMAb delivery.

**Table 4: Plasmid Details for Example 2**

| **Code** | **Name** | **R&D Lot#** | **Mfg. Date** |
|---|---|---|---|
| pGX9214 | Pseudo V2L2MD | D150827 | 9/1/2015 |

Summary of Examples 2 and 3 - These studies demonstrate that the administration of chondroitinase into the TA muscle of Balb/c mice, as well as C57BL/6 mice, prior to delivery of a DMAb plus EP enhances the expression of hIgG in the serum. Figure 1 shows that there was a 13.5 fold increase in hIgG serum expression in Balb/c mice at the peak time point (day 7) in group 3 pretreated with chondroitinase (mean of 2904.53 ng/ml) compared to group 1 (mean of 214.86 ng/ ml). C57BL/6 that received the same treatment (study INO-16-057) showed an 8.8 fold increase in hIgG serum expression at day 7 in group 3 pretreated with chondroitinase (mean of 5428.49 ng/ml) compared to the control group 1 (mean of 618.21 ng/ml). The hIgG serum expression levels of the groups pretreated with chondroitinase were comparable to the hIgG serum expression levels shown in the groups pretreated with hyaluronidase in both murine strains.

Chondroitinase pretreatment of the muscle may be used to enhance DMAb expression in mouse serum.

### EXAMPLE 4

### Methods for Example 5

Four groups of female Guinea pigs containing five animals each were administered pGX9207, a DNA plasmid encoding human antibody (DMAb) reactive to Middle East Respiratory Syndrome corona virus (MERS-coV). The DNA injection followed by electroporation was performed in both quadriceps muscles in all groups.

Guinea pigs of group 3 were treated with 200 µl Chondroitinase AC (0.5 Units/ml) into each muscle 90 minutes before DMAb delivery. Control groups were either pretreated with PBS (group 1) or hyaluronidase (group 2; 400 Units/ml; purified from bovine testes). To test if chondroitinase shows an additive effect for degrading the cavy extracellular matrix of the quadriceps muscles upon delivery with hyaluronidase, animals were pretreated with both enzymes, hyaluronidase and chondroitinase, 90 minutes before DMAb delivery and EP in group 4.

PGX 9207 was injected at a concentration of 0.45 mg in 200 µl SSC intramuscularly at the sites that received enzyme pretreatment (Group 2, 3 and 4) or non-pretreated sites (Group 1), and EP was performed immediately after injection. Electroporation was applied to the site of injection using CELLECTRA^{®}-3P (8 mm electrodes) device (Inovio Pharmaceuticals, Inc.).

The parameters were:
Number of pulses: 2 sets of 2 pulses (2x2);
Current strength: 0.2 Amp;
Maximum voltage: 200V;
Electroporation pulse duration: 52 milliseconds;
Interval separating pulses: 0.2 seconds between pulses, and 3 seconds between each set of pulses.

On days 0, 3, 7, 10, 14, 17, and 21, Guinea pigs were bled (200 µl) and the levels of human IgG kappa (DMAb MERS-hIgG) were measured in the serum by ELISA.

**Table 5: Experimental Details for Example 5**

| **Group Number (n/group)** | **Animal Eartag #** | **Pre-Treatment** | **Plasmid** | **# of Injection Site(s) / Location / Tx** | **EP Device & Inj Method** | **Injection Volume (uL)** | **DNA dose per site (mg)** | **Total DNA dose/plas mid (mg)** |
|---|---|---|---|---|---|---|---|---|
| **1.5** | 201-205 | PBS | pGX9207 | 1/quad muscle, left and right site | CELL., 8mm, elongated | 0.2 | 0.45 | 0.9 |
| **2.5** | 206-210 | *Hyaluroni dase (bovine testes) | pGX9207 | 1/quad muscle, left and right site | CELL., 8mm, elongated | 0.2 | 0.45 | 0.9 |
| **3.5** | 211-215 | **Chondroi tinase AC *(F. Heparinum* ) | pGX9207 | 1 /quad muscle, left and right site | CELL., 8mm, elongated | 0.2 | 0.45 | 0.9 |
| **4. 5** | 216-220 | Chondroiti nase AC (*F. Heparinum* ) + Hyaluronid ase (bovine testes) | pGX9207 | 1/quad muscle, left and right site | CELL., 8mm, elongated | 0.2 | 0.45 | 0.9 |

| | | | | | | | | |
|---|---|---|---|---|---|---|---|---|
| * Hyaluronidase: 400U/ml. ** Chondroitinase: 0.5U/ml. | | | | | | | | |

Treatment of enzymes was performed 90 minutes before DMAb delivery.

**Table 4: Plasmid Details for Example 2**

| **Code** | **Name** | **R&D Lot#** | **Concentration (mg/ml)** | **Mfg. Date** |
|---|---|---|---|---|
| pGX9207 | MERS IgG | Pooled of Below | 8.7 mg/ml | 2/26/2016 |
| | | D150803A | 13.9 | 8/6/2015 |
| | | D150730 | 10.6 | 8/3/2015 |
| | | D150528 | 10.4 | 5/28/2015 |
| | | D150728 | 8.45 | 7/28/2015 |
| | | D150311B | 5.6 | 3/11/2015 |
| | | D163682 | 1 | |
| | | D160216 | 8.93 | 2/6/2016 |

### EXAMPLE 5

### Treatment with Chondroitinase Enhances Systemic DNA-based Antibody Expression

The action of Chondroitinase AC derived from the bacterium *Flavobacterium heparanum* enhances systemic serum levels of hIgG in Guinea pigs after administration of pGX9207 by injection and electroporation (EP).

The kinetics of serum hIgG levels in Hartley guinea pigs is displayed in Figure 3. In each group, peak expression is observed on day 7. Hartley guinea pigs treated with 0.5 Units/ml chondroitinase AC in 200 µl into both quadriceps muscles 90 minutes before DMAb delivery and EP had 8.6-fold higher levels of hIgG in their serum (day 7 mean of 485.39 ng/ml) than those guinea pigs pretreated with the control reagent PBS (day 7 mean of 98.79 ng/ml; see Figure 3). Pretreatments with chondroitinase led to 1.74-fold higher levels of hIgG than pretreatment with hyaluronidase (day 7 mean of 278.41 ng/ml) used as a positive control. Simultaneous application of both enzymes 90 minutes before DMAb delivery and EP (mean of 849.57 ng/ml) suggest an additive effect (3-fold increase compared to hyaluronidase and 1.75-fold increase compared to chondroitinase).

IgG concentrations detected in the guinea pig serum of animals exposed to a combination of hyaluronidase and chondroitinase enzymes were higher when compared to levels in guinea pigs pretreated with a single enzyme. Example expression kinetics of pGX9207 are depicted in Figure 4.

### EXAMPLE 6

### Chondroitinase enhances plasmid-encoded protein expression in mice (INO-16-056 and INO-16-057)

A total of 10 mice for either the INO-16-056 (Balb/c) or INO-16-057 (C57BL/6) were divided into two groups of 5 female mice (6-7 weeks old) and received an injection of pGX9214 (PseudoV2L2MD (Lot # D150827)), a DNA plasmid encoding human antibody (DMAb) reactive to Pseudomonas aeruginosa.

The left skeletal muscle of each mouse in experimental group 2 (Table 6) was pretreated with 30 µl Chondroitinase AC (purified from Flavibacterium heparinum; Sigma Aldrich) 30 min before pGX9214 delivery. Mice of the control group (group 1) were injected with PBS before DNA delivery and EP.

**Table 6: Experimental Details for Example 6**

| **Group Number (n/group)** | **Animal Eartag #** | **Pre-Treatment** | **Plasmid** | # **of Injection Site(s)** / **Location** / **Tx** | **EP Device & Inj Method** | **Injection Volume (uL)** | **DNA dose per site (mg)** | **Total DNA dose/plas mid (mg)** |
|---|---|---|---|---|---|---|---|---|
| **1.5** | 831-835 | PBS | pGX9214 | 1/TA, left side | CELL., 3P | 30 | 0.1 | 0.1 |
| **2. 5** | 841-845 | *Chondroiti nase AC | pGX9214 | 1/TA, left side | CELL., 3P | 30 | 0.1 | 0.1 |

| | | | | | | | | |
|---|---|---|---|---|---|---|---|---|
| * Table 6 Protocol INO-16-056 - Chondroitinase AC (Sigma Aldrich; catalog # C2780; 0.5U/ml; treatment of enzyme was performed 30 minutes before pDNA delivery. Mouse strain: Balb/c. | | | | | | | | |

**Table 7: Additional Experimental Details for Example 6**

| **Group Number (n/group)** | **Animal Eartag #** | **Pre-Treatment** | **Plasmid** | # **of Injection Site(s)** / **Location** / **Tx** | **EP Device & Inj Method** | **Injection Volume (uL)** | **DNA dose per site (mg)** | **Total DNA dose/plas mid (mg)** |
|---|---|---|---|---|---|---|---|---|
| **1.5** | 876-880 | PBS | pGX9214 | 1/TA, left side | CELL., 3P | 30 | 0.1 | 0.1 |
| **2.5** | 886-890 | *Chondroiti nase AC | pGX9214 | 1/TA, left side | CELL., 3P | 30 | 0.1 | 0.1 |

| | | | | | | | | |
|---|---|---|---|---|---|---|---|---|
| * Table 7 Protocol INO-16-057- Chondroitinase AC (Sigma Aldrich; catalog # C2780; 0.5U/ml; treatment of enzyme was performed 30 minutes before pDNA delivery. Mouse strain: C57BL/6. | | | | | | | | |

The plasmid DNA was administrated at a concentration of 0.1 mg in 30 µl SSC intramuscularly at the sites that received enzyme pretreatment (Group 2) or non-pretreated sites (Group 1), and electroporation was performed immediately after injection. Electroporation was applied to the site of injection using CELLECTRA^{®}-3P (2 mm electrodes) device (Inovio Pharmaceuticals, Inc.).

Parameters in mice for all experiments in this report were:
Number of pulses = 2 sets of 2 pulses (2x2),
Current Strength = 0.1 Amp,
Maximum Voltage = 200V,
Electroporation pulse duration = 52 milliseconds,
Interval separating pulses = 0.2 seconds between pulses, and 3 seconds between each set of pulses.

On days 0, 3, 7 mice were bled (50 µl) and the levels of human IgG kappa (DMAb PseudoV2L2MD) were measured in the serum by ELISA.

### Results INO-16-056:

The level of expression of DNA-encoded monoclonal antibody in the serum of Balb/c mice treated with pGX9214 (PseudoV2L2MD) is reported here. Pretreatment of the muscle delivery site with Chondroitinase AC from Flavobacterium heparinum, significantly enhances systemic serum levels of hIgG detected (Figure 5).

### Results INO-16-057:

Similar to Balb/c mice, C57BL/6 mice treated with chondroitinase and pGX9214 show an increased level of expression of DNA-encoded monoclonal antibody in their serum in comparison to mice that received pGX9214 plus PBS only (Figure 6).

### Conclusion for studies INO-16-056 and INO-16-057:

These studies demonstrate that the administration of chondroitinase into the skeletal muscle of Balb/c mice as well as C57BL/6 mice prior to delivery of a DMAb plus EP enhances the expression of hIgG in the serum. Figure 5 shows that there was a 13.5 fold increase in hIgG serum expression in Balb/c mice at the peak time point (day 7) in group 2 pretreated with chondroitinase (mean of 2904.53 ng/ml) compared to group 1 (mean of 214.86 ng/ ml). C57BL/6 that received the same treatment (study INO-16-057) showed an 8.8 fold increase in hIgG serum expression at day 7 in group 2 pretreated with chondroitinase (mean of 5428.49 ng/ml) compared to the control group 1 (mean of 618.21 ng/ml).

In view of the foregoing, chondroitinase pretreatment of the muscle can be used to enhance pDNA expression.

### EXAMPLE 7

### Clinical grade Cho-ABC leads to increased levels of plasmid-encoded proteins (INO-16-083B and INO-16-097)

To eventually transfer the basic preclinical research of chondroitinase into the clinic, a clinical grade Chondroitinase ABC was tested. According to the manufacturer, this product is purified from *Proteus vulgaris* by cation exchange chromatography, has low endotoxin levels and shows no contaminants such as chondrosulfatases, proteases, heparinases and heparitinases. The enzyme shows similar activity to Chondroitinase ABC (Condoliase) from Seikagaku. Furthermore, we added GALNS, a human recombinant chondroitinase (R&D system) to our assay. This enzyme, also known as lysosomal N-Acetylgalactosamine-6-Sulfatase (GalNAc6S), hydrolyzes the 6-sulfate groups of the N-acetyl-D-galactosamine 6-sulfate units of chondroitin sulfate and of the D-galactose 6-sulfate units of keratan sulfate. In the clinic, this enzyme is applied for the treatment of Morquio A syndrome or mucopolysaccharidosis 4A, a lysosomal storage disorder. The disease is characterized by intracellular accumulation of keratan sulfate and chondroitin-6-sulfate due to the lack of GalNAc6S. Source of this protein is Spodoptera frugiperda, Sf 21 (baculovirus)-derived Ala27-His522, with an N-terminal 6-His tag.

The objective was to determine the ability of Chondroitinase AC (F. heparinum, Sigma Aldrich) clinical grade Chondroitinase ABC (P. vulgaris, Amsbio) and GALNS (human recombinant chondroitinase, R&D systems) to enhance pDNA expression. Subsequently, the effect of various doses of Chondroitinase ABC was examined.

INO-16-083B: In order to assess the effectiveness of the three different types of chondroitinases, Balb/c mice were split into 4 groups with 5 mice per group. Each mouse received an injection of 0.1 mg of plasmid DNA pGX9203 encoding for a Dengue virus-specific monoclonal antibody hIgG-DVSF-1 (Lot # D160222A) into the left skeletal muscle followed by electroporation. The parameters of the procedures were performed as described for study 1. Mice were treated with respective enzymes or PBS as a control 30 minutes before DNA administration as shown in table 2.1. Blood samples were collected on day 0, 3 and 7 and serum human IgG lambda levels were measured by ELISA.

Data suggests, that both, Chondroitinase AC and Chondroitinase ABC enzymes enhance gene expression (Figure 7). However, the recombinant protein GALNS does not.

INO-16-097: In order to decide on an optimal concentration for following experiments, the effect of various doses of the clinical grade Chondroitinase ABC were tested. The detailed experimental strategy is described in table 2.3. Four different doses of Chondroitinase ABC ranging from 0.1 U/ml to 5 U/ml were applied in Balb/c mice (group 2-5) prior to gene transfer of pGX9207 into the left skeletal muscle and compared to the PBS control group 1.

Increasing Chondroitinase ABC concentrations further enhanced the expression of the transgene with 2.5 U/ml as an optimal dose for our animal models (Figure 8).

For studies INO-16-083B and INO-16-097: When animals were pretreated with the respective chondroitinases, the clinical grade enzyme resulted in a similar increase of gene expression as Chondroitinase AC which was successfully shown in previous experiments. By applying a concentration of 2.5 U/ml before injection of pGX9207 we found a 4-fold increase in human serum IgG levels in comparison to the PBS control group. This observation confirmed that Chondroitinase ABC leads to a significant elevation of plasmid-encoded protein levels and provided an excellent starting point for further testing.

**Table 8: Experimental details for Example 7.**

| **Group #** | **Animal Eartag #** | **Plasmid** | **Pre-treatment** | **# of Injection per site / Location** | **EP Device & Inj Method** | **Injection Volume per treatment (ul)** | **DNA dose per site (mg)** | **Total DNA dose / plasmid (mg)** |
|---|---|---|---|---|---|---|---|---|
| **1.5** | 201-205 | pGX9203 | PBS | 1/ TA, left site | CELL., 3P | 30 | 0.1 | 0.1 |
| **2.5** | 211-215 | pGX9203 | *Chondroitinase AC | 1/ TA, left site | CELL., 3P | 30 | 0.1 | 0.1 |
| **3.5** | 216-220 | pGX9203 | **Chondroitinase ABC | 1/ TA, left site | CELL., 3P | 30 | 0.1 | 0.1 |
| **4.5** | 221-225 | pGX9203 | ***GALNS | 1/ TA, left site | CELL., 3P | 30 | 0.1 | 0.1 |

Protocol INO-16-083B. *Chondroitinase AC (F. heparinum); Sigma Aldrich; catalog # C2780; 0.5U/ml; **Chondroitinase ABC (P. vulgaris); Amsbio, catalog # AMS.E1028-10; 0.5U/ml, recombinant Human GALNS Protein, R&D systems, catalog #8269-SU-050; 20µg/ml. Enzyme treatments were performed 30min before pDNA delivery; mouse strain: Balb/c.

**Table 9: Additional experimental details for Example 7.**

| **Group #** | **Animal Eartag #** | **Plasmid** | **Pre-treatment** | **# of Injection per site / Location** | **EP Device & Inj Method** | **Injection Volume per treatment (ul) - Enzyme Conc. (U/ml).** | **DNA dose per site (mg)** | **Total DNA dose / plasmid (mg)** |
|---|---|---|---|---|---|---|---|---|
| **1.5** | 201-205 | pGX9207 | PBS | 1/ TA, left site | CELL., 3P | 30 -- | 0.1 | 0.1 |
| **2.5** | 211-215 | pGX9207 | Chondroitinase ABC | 1/ TA, left site | CELL., 3P | 30 - 0.1 | 0.1 | 0.1 |
| **3.5** | 216-220 | pGX9207 | Chondroitinase ABC | 1/ TA, left site | CELL., 3P | 30 - 0.2 | 0.1 | 0.1 |
| **4.5** | 221-225 | pGX9207 | Chondroitinase ABC | 1/ TA, left site | CELL., 3P | 30 - 0.5 | 0.1 | 0.1 |
| **5.5** | 351-355 | pGX9207 | Chondroitinase ABC | 1/TA, left site | CELL., 3P | 30 - 2.5 | 0.1 | 0.1 |

Protocol INO-16-097 Chondroitinase ABC (*P. vulgaris*); Amsbio, catalog # AMS.E1028-10; Enzyme treatments were performed 30min before pDNA delivery; mouse strain: Balb/c. pGX9207 (MERS IgG) - Lot # 63682.

### EXAMPLE 8

### Cho-ABC administration results in enhanced protein expression (INO-16-188)

Previous experiments indicated that clinical grade Chondroitinase ABC is applicable for enhancing plasmid-dependent gene expression in mice. To further confirm this finding, the next step was to provide visual images of this enhanced gene expression in chondroitinase-treated skeletal mouse muscle. Therefore, a reporter gene and measured protein expression based on fluorescence.

For quantification of reporter gene expression enzyme (2.5 U/ ml; 30 min standard pretreatment), PBS (control) and pDNA (; pGX9902, 50 µg dose per leg) were delivered into the left and right murine skeletal muscle by electroporation (table 10). After 72 hours murine hindlimbs were dissected and skin was removed. Fluorescence intensity in the treated mouse muscles was measured by a fluorescence imaging system (ProteinSimple) and quantified by AlphaView SA.

The data reveals that reporter gene expression in mouse muscles upon treatment (30 min) with Chondroitinase ABC was enhanced (Figure 9).

Evidence was generated that shows Chondroitinase significantly enhanced gene expression by visualization of the protein after 72 hours of the treatments. We achieved a 6.7-fold improvement by applying the enzyme before treatment with the reporter pDNA in comparison to our control group. This finding of preclinical mouse experiments further assured that Chondroitinase ABC might potentially advance DNA-based immunotherapies.

**Table 10: Experimental details for Example 8.**

| **Group #** | **Animal Eartag #** | **Plasmid** | **Pre-treatment** | **# of Injection per site / Location** | **EP Device & Inj Method** | **Injection Volume per treatment (ul)** | **DNA dose per site (µg)** | **Total DNA dose / plasmid (mg)** |
|---|---|---|---|---|---|---|---|---|
| **1.3** | 151-153 | pGX9902 | PBS | 1/ TA, left and right site | CELL., 3P | 30 | 50 | 0.1 |
| **2.3** | 154-156 | pGX9902 | Chondroitinase ABC | 1/ TA, left and right site | CELL., 3P | 30 | 50 | 0.1 |

Protocol INO-16-188. Mice were pretreated with Chondroitinase ABC (2.5 U/ ml) or PBS (control) into the skeletal muscle on the left and right side 30 min before pDNA (pGX9902) injection. Mouse strain: Balb/c.

### EXAMPLE 9

### Histological analysis of Chondroitinase ABC treated muscles (INO-16-195)

To test if Chondroitinase ABC leads to morphological changes in murine muscle tissue, the histopathology of the treated hind limbs at the site of injection was examined.

Four groups of mice received either a treatment with chondroitinase (group 2 and group 3) or with PBS (group 1 and group 4) in to their left and right skeletal muscles of the hind limb (table 11). Plasmid DNA (pGX9207 - MERS IgG (Lot# 163682)) was delivered by electroporation into the muscles of mice belonging to group 1 and 2. After 72 hours murine hindlimbs were dissected and the skeletal muscles were isolated for Haemotoxylin and Eosin (H&E) staining. H&E staining as well as the scanning of the slides were performed by a contract research organization (Reveal Biosciences). After obtaining the images of the whole muscle, representative examples of the muscle histopathology were selected by using the software Case Viewer.

Experimental animals did not show any signs of changes in their behavior and appeared healthy during and after the experimental treatment procedures. After completing the analysis, evidence of spontaneous inflammation was not observed and no tissue damage or other apparent histological changes could be observed when mouse muscles were treated with only chondroitinase compared to the PBS control (figure 10). When pDNA was additionally delivered by electroporation infiltrating immune cells in both, chondroitinase and PBS pretreated mice was observed.

These data show that electroporation, but not the enzyme causes reversibly changes in the tissue morphology, indicating that the administration of the enzyme not to be mediating serious adverse events (SAE).

A slightly higher number of infiltrating cells in the chondroitinase plus pDNA/EP group is likely to be due to the higher pDNA transfection efficiency.

**Table 11: Additional experimental details for Example 9.**

| **Group #** | **Animal Eartag** # | **Plasmid** | **Pre-treatment** | **# of Injection per site / Location** | **EP Device & Inj Method** | **Injection Volume per treatment (ul)** | **Enzyme conc. (U/ ml)** | **DNA dose per site (µg)** | **Total DNA dose / plasmid (mg)** |
|---|---|---|---|---|---|---|---|---|---|
| **1.3** | 326-330 | pGX9207 | PBS | 1/ TA, left and right site | CELL., 3P | 30 | --- | 50 | 0.1 |
| **2.3** | 336-340 | pGX9207 | Chondroitinase ABC | 1/ TA, left and right site | CELL., 3P | 30 | 2.5 | 50 | 0.1 |
| **3.3** | 341-345 | No plasmid | Chondroitinase ABC | 1/ TA, left and right site | CELL., 3P | 30 | 2.5 | 50 | 0.1 |
| **4.3** | 346-350 | No plasmid | PBS | 1/ TA, left and right site | CELL., 3P | 30 | --- | 50 | 0.1 |

### EXAMPLE 10

### Cho-ABC enhances plasmid-encoded protein expression in New Zealand rabbits (INO-16-157)

To further study the effects of chondroitinase in DNA-based immunotherapy, an investigation was conducted into whether intramuscular pretreatment with Chondroitinase ABC was potent in enhancing protein expression in the rabbit.

2 mg of pGX9207 (MERS IgG (Lot# 63682)) was administered to 12 New Zealand rabbits (group 1 and group 2, animal age: 9 weeks, table 12). Electroporation was applied to the site of injections in the skeletal muscle using CELLECTRA^{®}-device.

Parameters in rabbits were for all experiments in this report:
- Number of pulses = 3 pulses
- Current Strength = 0.5 Amp,
- Electroporation pulse duration = 52 milliseconds,
- Interval separating pulses = 1 second between pulses

Animals of group 2 were pretreated with chondroitinase into the same site as pDNA delivery and animals of group 1 served as PBS controls. Bleeds were performed at day 0, 3, 5 and 6. Human IgG levels were measured by ELISA.

The data demonstrated significant enhancement of hIgG serum levels in groups whose muscles were pretreated with the enzyme compared to the PBS treatment.

The significant improvement of DNA-based gene expression in the rabbit by injecting the matrix-degrading enzyme (4.9-fold in comparison to the control) is consistent with mouse data and further consolidates the potency of the use of Chondroitinase ABC in plasmid DNA-based gene therapy.

**Table 12. Experimental detail for Example 10.**

| **Group #** | **Animal Eartag #** | **Plasmid** | **Pre-treatment** | **# of Injection per site / Location** | **EP Device & Inj Method** | **Injection Volume per treatment (ml)** | **Enzyme conc. (U/ ml)** | **DNA dose per site (µg)** | **Total DNA dose / plasmid (mg)** |
|---|---|---|---|---|---|---|---|---|---|
| **1.6** | 1, 2, 3, 4, 5, 6 | pGX9207 | PBS | 2/ left quad | CELL., 5P, IM | 1 | --- | 1 | 2 |
| **2.6** | 21, 22, 23, 24, 25, 26 | pGX9207 | Chondroitinase ABC | 2/ left quad | CELL., 5P, IM | 1 | 2.5 | 1 | 2 |

### EXAMPLE 11

### Co-formulation of Cho ABC with pDNA in mice (INO-16-99B, INO-16-201, INO-16-188)

Previous experiments showed that the accurate pretreatment timing of chondroitinase is not essential for optimal gene expression. No significant differences in mice were seen when we pretreated them 5 min, 15 min, 30 min, 2 hrs, 24 hrs or 48 hrs before pDNA delivery and EP. This raised the question whether it is possible to co-formulate Chondroitinase ABC with pDNA without negatively impacting gene expression.

To this purpose 14 mice were treated with the enzyme (2.5 U/ ml) and pDNA (pGX9702, MERS IgG (Lot #s: 63682 and 72883)) in one single injection (group 3). EP was performed 1 min after injection due to the necessary reaction time of the chondroitinase. Two control groups were included in this experiment: mice of group 1 were pretreated with PBS and mice of group received an injection of the enzyme (group 2). Delivery of the pDNA by electroporation was performed in both groups after 30 min.

Parameters in mice for all co-formulation experiments in this report were:
- Number of pulses = 2 sets of 2 pulses (2x2),
- Current Strength = 0.1 Amp,
- Maximum Voltage = 200V,
- Electroporation pulse duration = 52 milliseconds,
- Interval separating pulses = 0.2 seconds between pulses, and 3 seconds between each set of pulses.

On days 0, 3, 6 mice were bled (50 µl) and the levels of human IgG kappa (DMAb PseudoV2L2MD) were measured in the serum by ELISA.

Animals that received the co-formulated drug, showed a high significant increase of hIgG in their serum in comparison to the PBS control group (group 1). Notably, there was no significant difference in protein production between animals that underwent the standard procedure (30 min pretreatment; group 2) and the animals that received the novel gene transfer procedure (group 3).

The results show that a co-formulation of chondroitinase with pDNA is feasible. Gene expression in animals that received the single injection (enzyme/ pDNA) was 5.6-fold higher than the expression levels in the PBS control group. The enhanced protein expression levels were equivalent to the original tissue pretreatment procedure. Co-formulation techniques and methods may be less complicated, less technically challenging and timesaving.

**Table 13: Experimental detail for Example 11**

| **Group #** | **Animal Eartag #** | **Plasmid** | **Enzyme or PBS** | **Timepoint of enzyme administration or co-delivery** | **# of Injection per site / Location** | **EP Device & Inj Method** | **Injection Volume per treatment (ul)** | **DNA dose per site (mg)** | **Total DNA dose / plasmid (mg)** |
|---|---|---|---|---|---|---|---|---|---|
| **1.14** | 437-442, 250-257 | pGX9207 | PBS | 30min before DMAb/EP | 1/ TA, left site | CELL., 3P | 30 | 0.1 | 0.1 |
| **2.14** | 413-418, 266-273 | pGX9207 | Chondroitinase ABC | 30min before DMAb/EP | 1/ TA, left site | CELL., 3P | 30 | 0.1 | 0.1 |
| **3.14** | 425-430, 282-289 | pGX9207 | Chondroitinase ABC | DMAb co-delivery with enzyme, 1min EP delay | 1/ TA, left site | CELL., 3P | 30 | 0.1 | 0.1 |

### EXAMPLE 12

### Co-formulation of Chondroitinase with pDNA results in enhanced fluorescent protein expression (INO-16-188)

The next subsequent step was to validate observations with a fluorescence reporter plasmid.

To visualize reporter gene expression, chondroitinase (2.5 U/ml), PBS (control) and pDNA (pGX9902) were delivered as a single injection into the left and right murine skeletal muscle by electroporation (table 14). Dissection of the hindlimbs was performed 72 hours after treatments. Fluorescence intensity in the treated mouse muscles was measured by an imager (FluorChem M system, ProteinSimple) and quantified by AlphaView SA.

Consistent to previous data, coformulation of Chondroitinase ABC and pDNA leads to enhanced gene expression in comparison to the PBS control. No significant differences in gene expression could be seen between group 3 (coformulation) and group 2 (30 min standard pretreatment).

Visualization of gene expression by using a reporter plasmid confirms that coformulation of enzyme and pDNA in a single injection is beneficial in the mouse model.

**Table 14: Experimental detail for Example 12.**

| **Group #** | **Animal Eartag #** | **Plasmid** | **Enzyme or PBS** | **Timepoint of enzyme administration or co-delivery** | **# of Injection per site / Location** | **EP Device & Inj Method** | **Injection Volume per treatment (ul)** | **DNA dose per site (mg)** | **Total DNA dose / plasmid (mg)** |
|---|---|---|---|---|---|---|---|---|---|
| **1.3** | 151-153 | pGX9902 | PBS | 30min before DMAb/EP | 1/ TA, left and right site | CELL., 3P | 30 | 0.1 | 0.1 |
| **2.3** | 154-156 | pGX9902 | Chondroitinase ABC | 30min before DMAb/EP | 1/ TA, left and right site | CELL., 3P | 30 | 0.1 | 0.1 |
| **3.14** | 160-162 | pGX9202 | Chondroitinase ABC | Enzyme/ pDNA CoF, 1min EP delay | 1/ TA, left site | CELL., 3P | 30 | 0.1 | 0.1 |

Protocol INO-16-188. Mice of group 3 received a single injection of Chondroitinase ABC (2.5 U/ml) and pDNA (pGX9902) into the skeletal muscle on the left and right side 1 min before EP. Mice of control groups were treated after the standard protocol: they either were pretreated with chondroitinase or PBS 30 min before pDNA delivery and EP. Mouse strain: Balb/c. CoF = coformulation.

### EXAMPLE 13

### Co-formulatioin of Cho ABC with pDNA in rabbit (INO-16-180)

To assess the effect of coformulated Chondroitinase ABC/ pDNA on gene expression of large muscles, an experiment was carried out on the rabbit skeletal muscle. In addition, whether a delay of electroporation is advantageous in this model was tested. Finally, whether a 1 hour incubation of the enzyme with the pDNA has an impact on gene expression in vivo was studied.

To this purpose 3 groups of rabbits (New Zealand rabbits, 6 animals per group, age 12 weeks) were injected with the enzyme/ pDNA (pGX9207) coformulation into the left skeletal muscle (two injections; table 15). EP was immediately performed after enzyme/ pDNA delivery in group 3. In group 4 and 5 electroporation was delayed and performed 1 min after enzyme/ pDNA administration. Animals of group 5 received a mixture of chondroitinase and pDNA which was incubated on ice 1 hour prior treatments. Animals of group 1 served as PBS controls and animals of group 2 were pretreated according to our 30 min standard protocol. Electroporation was applied to the site of injections in the skeletal muscle using CELLECTRA^{®}-device. Bleeds were performed at day 0, 4 and 5. Human IgG levels were measured by ELISA.

By measuring human IgG levels in the rabbit serum, protein production was significantly increased in rabbits that were administered with the coformulation in comparison to the control (figure 14). Furthermore, the 1 min EP delay was associated with enhanced human IgG serum levels, and equivalent expression upon coformulation 1 hour before delivery with coformulation several minutes before delivery was shown.

Results demonstrate the ability to co-formulate chondroitinase with pDNA to enhance gene expression. Further advancement of this new protocol by adding an EP delay of 1 min led to a 6.41-fold increase of expression in comparison to the control. After an incubation of the enzyme/ pDNA coformulation for 1 hour, equivalent gene expression levels in comparison to immediate injection after formulation was achieved.

Coformulating enzyme and pDNA improved protein production in this preclinical study.

**Table 15: Experimental detail for Example 13.**

| **Group #** | **Animal Eartag** # | **Plasmid** | **Enzyme or PBS** | **Timepoint of enzyme administration or co-delivery** | # **of Injection per site / Location** | **EP Device & Inj Method** | **Injection Volume per treatment (ul)** | **DNA dose per site (mg)** | **Total DNA dose / plasmid (mg)** |
|---|---|---|---|---|---|---|---|---|---|
| **1. 6** | 25, 26, 27, 28, 29, 30 | pGX9907 | PBS | 30min before DMAb/EP | 2/ left quad | CELL., 5P, IM | 1 | --- | 1 |
| **2. 6** | 1,2,3, 4, 5, 6 | pGX9907 | Chondroitinase ABC | 30min before DMAb/EP | 2/ left quad | CELL., 5P, IM | 1 | 2 | 1 |
| **3. 6** | 7, 8, 9, 10, 11, 12 | pGX9907 | Chondroitinase ABC | Enzyme/ pDNA CoF, immediate EP | 2/ left quad | CELL., 5P, IM | 1 | 2 | 1 |
| **4. 6** | 13, 14, 15, 16, 17, 18 | pGX9907 | Chondroitinase ABC | Enzyme/ pDNA CoF, 1min EP delay | 2/ left quad | CELL., 5P, IM | 1 | 2 | 1 |
| **5. 6** | 19,20, 21, 22,23, 24 | pGX9907 | Chondroitinase ABC | Enzyme/ pDNA CoF, 1 hour incubation of reagent, 1min EP delay, | 2/ left quad | CELL., 5P, IM | 1 | 2 | 1 |

### EXAMPLE 14

### Co-formulation of Cho ABC with pDNA - in vitro pDNA stability test (INO-16-252A)

To further test the possibility of combining chondroitinase and pDNA in one shot, pDNA stability upon coformulation under different conditions was evaluated. The purpose of stability testing is to provide evidence on how the quality of the product varies with time under the influence of environmental factors, such as temperature, and to establish a shelf life for the future drug product and recommended storage conditions.

To test stability and quality of pDNA when incubated with chondroitinase, molecular weight and conformation of the pDNA was examined by agarose gel electrophoresis. Seven samples contained pDNA (pGX9207, 250 ng) with Chondroitinase ABC (2.5 U/ ml, table 9.1). Seven further samples served as controls and consisted of pDNA and PBS. Samples of groups 1-2 were tested by gel electrophoresis (TAE, 1 % agarose, EmbiTec) after formulation, groups 3-4 were incubated at room temperature (RT, defined as 21°C) for 10 min. Incubation at RT was performed in a PCR cycle. Further incubations were performed as followed: 5-6: 6°C, 120 min, 7-8: RT, 120 min, 9-10: 6°C, 24 hrs, 11-12: RT, 24 hrs, and13-14: 6°C, 10 min. After gel electrophoresis the gel was analyzed by Gene Sys software (no binning, EDR, 120 ms exposure).

The construct pGX9207 has a supercoiled conformation and a molecular weight of 5171 bp. All DNA samples (enzyme-containing and control samples) in the original conformation with the original weight. No differences could be seen between the bands and samples, respectively (figure 15).

As the bands for all samples represent a supercoiled DNA with the original molecular weight this initial pDNA stability approach indicates that chondroitinase has no impact on plasmid structure and drug quality respectively.

**Table 16: Experimental detail for Example 14.**

| | **Plasmid** | **Enzyme or PBS** | **Incubation time** | **DNA concentration in CoF** | **Enzyme concentration** | **Total volume of CoF in gel** |
|---|---|---|---|---|---|---|
| **1.** | pGX9207 | PBS | No incubation time | 500 ng/10ul | 2.5 U/ml | 5ul |
| **2.** | pGX9207 | CHO-ABC | No incubation time | 500 ng/10ul | 2.5 U/ml | 5ul |
| **3.** | pGX9207 | PBS | 10 min, RT | 500 ng/10ul | 2.5 U/ml | 5ul |
| **4.** | pGX9207 | CHO-ABC | 10 min, RT | 500 ng/10ul | 2.5 U/ml | 5ul |
| **5.** | pGX9207 | PBS | 120 min, 6°C | 500 ng/10ul | 2.5 U/ml | 5ul |
| **6.** | pGX9207 | CHO-ABC | 120 min, 6°C | 500 ng/10ul | 2.5 U/ml | 5ul |
| **7.** | pGX9207 | PBS | 120 min, RT | 500 ng/10ul | 2.5 U/ml | 5ul |
| **8.** | pGX9207 | CHO-ABC | 120 min, RT | 500 ng/10ul | 2.5 U/ml | 5ul |
| **9.** | pGX9207 | PBS | 24 hours, 6°C | 500 ng/10ul | 2.5 U/ml | 5ul |
| **10.** | pGX9207 | CHO-ABC | 24 hours, 6°C | 500 ng/10ul | 2.5 U/ml | 5ul |
| **11.** | pGX9207 | PBS | 24 hours, RT | 500 ng/10ul | 2.5 U/ml | 5ul |
| **12.** | pGX9207 | CHO-ABC | 24 hours, RT | 500 ng/10ul | 2.5 U/ml | 5ul |
| **13.** | pGX9207 | PBS | 10 min, 6°C | 500 ng/10ul | 2.5 U/ml | 5ul |
| **14.** | pGX9207 | CHO-ABC | 10 min, 6°C | 500 ng/10ul | 2.5 U/ml | 5ul |

### EXAMPLE 15

### Co-formulation of Cho ABC with pDNA - in vivo pDNA stability study (INO-16-237)

Whether chondroitinase coformulation with pDNA can be prepared up to 24 hrs prior to in vivo delivery was investigated. Therefore, the effect of co-formulations made 10, 120 minutes or 24 hours before delivery had on gene expression in mice was tested.

Coformulations of enzyme (2.5 U/ ml) and pDNA (pGX9207, 0.1 mg), were prepared 10 min, 120 min, and 24 hours prior drug injection (intramuscular) and electroporation (1 min delayed, table 10.1). As controls two groups of mice received either chondroitinase (group 2) or PBS (group 1) 30 min pretreatment. Blood was collected at day 0, 3, 6 and serum hIgG levels were determined by ELISA.

No decline of gene expression in mice did occur when enzyme/ pDNA coformulations were stored for 24 hours.

This data demonstrates that our reagents containing both, Chondroitinase ABC and pDNA, can be stored for at least 24 hours. See Fig. 16.

**Table 17: Experimental detail for Example 15.**

| **Group #** | **Animal eartag #** | **Plasmid** | **Enzyme** | **Incubation time** | **# of Injection per site / Location** | **DNA dose per site (mg)** | **Total DNA dose/ plasmid (mg)** |
|---|---|---|---|---|---|---|---|
| **1. 8** | 301-308 | pGX9207 | PBS | 30 min pretreatment | 1/ TA, left site | 0.1 | 0.1 |
| **2. 8** | 309-316 | pGX9207 | CHO ABC | 30 min pretreatment | 1/ TA, left site | 0.1 | 0.1 |
| **3.8** | 325-332 | pGX9207 | CHO ABC | 10 min, on ice; CoF, 1 min EP delay | 1/ TA, left site | 0.1 | 0.1 |
| **4 8** | 333-340 | pGX9207 | CHO ABC | 120 min, on ice; CoF, 1 min EP delay | 1/ TA, left site | 0.1 | 0.1 |
| **5. 8** | 341, 342, 343, 345, 346, 347, 348, 349 | pGX9207 | CHO ABC | 24 hours, on ice CoF, 1 min EP delay | 1/ TA, left site | 0.1 | 0.1 |

### EXAMPLE 16

### Methods for Examples 17 and 18

### Hyaluronidase Treatment on Systemic Monoclonal Antibody Expression

Nine guinea pigs were divided into two groups of 3 female Hartley Guinea pigs (6 weeks old) and were immunized with pGX9203, a DNA plasmid encoding human antibody ((DMAb) reactive to Dengue virus, while the control group received PBS only.

The leg muscles of experimental group I (see table below) were pretreated with 0.2 ml hyaluronidase ((0.4 Unites/ml) purified from bovine testes) at 6 separate sites three hours before pGX9203 delivery. The pGX 9203 delivery was performed by injection of 0.33 mg of plasmid in 200 µl SSC intramuscularly at the sites that received hyaluronidase pretreatment (Group 1) or non-pretreated sites (Group 2), and electroporation was performed immediately after injection. Electroporation was applied to the sited of injection using CELLECTRA^{®} -3P (5 mm electrodes) device (Inovio Pharmaceuticals, Inc.). Parameters were:
- Number of pulses = 2 sets of 2 pulses (2x2),
- Current Strength = 0.1 Amp,
- Maximum Voltage = 200V,
- Electroporation pulse duration = 52 milliseconds, and
- Interval separating pulses = 0.2 seconds between pulses, and 3 seconds between each set of pulses.

On days 0, 7, 10, 14, 17, 21, and 24, Guinea pigs were bled (250 µl) and the levels of human IgG lambda (DMAb DVSF-1) were measured in the serum by ELISA.

For analysis of humoral responses against hIgG, an ELISA against the hIgG kappa standard protein was performed.

**Table 1.**

| Group Number (n/Group) | Plasmid | Animal Eartag # | # of Injection Sites/ Location/ Tx | EP Device and Injection Method | Injection Volume per Treatment (mL) | DNA dose per site (mg) | Total DNA dose/plasmid (mg) |
|---|---|---|---|---|---|---|---|
| * 1/3 | pGX9203 | 793, 793, 794 | 6/Quad and TA muscles | Elongated 3P | 0.2 | 0.33 | 2 |
| 2/3 | PGX9203 | 795, 796, 797 | 6/Quad and TA muscles | Elongated 3P | 0.2 | 0.33 | 2 |
| 3/3 | pbs | 798, 799, 800 | N/A | N/A | 0.1 | 0 | 0 |
| *Group 1 was treated with 0.2 hyaluronidase (0.4) Unites/ml) in quad and TA muscles on each flank 3 hours before DMAb delivery, Sigma: H4272-30MG Lot#SLBM1476V. | | | | | | | |

**Table 2.**

| Code | Name | R&D Lot # | Mfg. Date |
|---|---|---|---|
| PGX9203 | DVSF-1 in pVAX | D150408A | August 8, 2015 - |

The level of expression of DNA-encoded monoclonal antibody in the serum of guinea pigs treated with pGX9203 (pDVSF-1) is reported. Pretreatment of the muscle delivery site with hyaluronidase significantly enhances systemic serum levels of hIgG detected. See figures 17 and 18. The addition of hyaluronidase (HYA) pretreatment of the leg muscle of Guinea pigs to a dNAb plus EP immunization regimen enhances the expression of hIgG in the serum. Figure 17 shows that there was an 18 fold increase in hIgG serum expression at the peak time point (day 14) in Group 1 pretreated with HYA (mean of 1.700 ng/ml) compared to Group 2 (mean of 92 ng/ml). The host humoral response raised against hIgG lambda was minimal in both Groups 1 (16.66 binding titer) and 2 (216.66 binding titer detected on day 28. Hyaluronidase pretreatment of the muscle may be utilized to enhance DMAb expression in the serum.

### EXAMPLE 17

### Hyaluronidase Treatment of Muscle Prior to pDNA Vaccine Delivery

Two groups of 4 female Hartley Guinea pigs (10 weeks old) were immunized with pGX2013, a DNA plasmid encoding Influenza NP PR8, while the control group received PBS only. The left TA muscle of experimental group 1 (see table 3 below) was pretreated with approximately 0.2 ml hyaluronidase ((about 0.4 Units/ml) purified from bovine testes) about 2 hours before vaccine delivery. Immunizations were performed on days 1 and 15. The vaccine delivery was performed by injection of about 20 µg of plasmid in approximately 200 µl SSC intramuscularly in TA, and electroporation was performed immediately after injection. Electroporation was applied to the site of injection using CELLECTRA^{®}-3P (5 mm electrodes) device (Inovio Pharmaceuticals, Inc.). Parameters were:
- Number of pulses = 2 sets of 2 pulses (2x2),
- Current Strength = 0.1 Amp,
- Maximum Voltage = 200 V,
- Electroporation pulse duration = 52 milliseconds, and
- Interval separating pulses = 0.2 seconds between pulses, and 3 seconds between each set of pulses.

14 days after the first and 7 days after the second immunizations Guinea pigs were bled (about 3 mls), and antigen specific cellular responses were measured in the harvested PBMC populations by a IFNγ ELISpot, which used overlapping peptides spanning the Influenza NP PR8 antigen. The assay was performed according to a modified ELISpot protocol.

Briefly, theELISpot protocol used is as follows: 3 mls of peripheral blood was drawn and transferred immediately into EDTA+ (Lavender cap) tubes on ice. Blood was diluted 1: 1 with Balanced Salts solution. Blood was slowly layered over 4.5 ml Ficoll density gradient in a 15 ml tube. Cells were spun at 2000 rpm, 30 mins, room temperature, with no brake. Buffy coat was harvested and diluted in R10 to 15 mls. Cells were then pelleted via centrifugation at 1500 rpm for 5 min at 4 °C. Cells were washed twice and passed through another 70 µm screen, counted and diluted to 1 × 10⁶ viable cells per ml in RPMI media with 10% (v/v) FBS and 2×antibiotic-antimycotic. Viability was determined by trypan blue staining. Wells of 2 x 96-well Millipore IP plates (Millipore, Billerica, MA) were coated with 100 µl primary anti-IFN-y antibody solution (5 µg/ml in PBS, pH 7.4, X-D11 (Stock at 1.78 mg/ml) for 24 h at 4 °C. Non-specific binding was blocked with 200 µl of blocking buffer 2 h at room temperature. After blocking and washing, 1 × 10⁵ splenocytes in 100 µl of RPMI were mixed with 50 µl stimulant in triplicate. After incubation in humidified 5% CO₂ at 37 °C for 18 h, cells were removed by washing and 100 µl of biotinylated secondary anti-IFN-y antibody (2 µg/ml, N-G3) in blocking buffer was added to each well. Following a 2 hr incubation and washing, alkaline phosphatase-conjugated streptavidin (SEL002, R&D Systems Inc., Minneapolis, MN) was diluted1:100 in blocking buffer and wells were incubated with 100 µl for 1 h at room temperature. Following washes, wells were incubated for 20 minutes at room temperature with 100 µl of BCIP/NBT detection reagent (SEL002, R & D Systems). The NP peptide pool used is the influenza nucleocapsid protein (Influenza A virus (A/Puerto Rico/8.34(H1N1)), which was split into pools of approximately 40 15-mers. Each NP peptide pool (1-3) was diluted 1 in 66 in Guinea Pig R10, 30 ul in 2 ml. The spot-forming units (SFU's) were counted and analyzed by ImmunoSpot Analyzers (Cellular Technology Ltd. in Shaker Heights, OH).

SFU's per million splenocytes for each individual Guinea pig and the mean +/-SEM of each group of mice were calculated. The statistical difference between a pair of immunized groups was assessed using a two-tailed unpaired t test that generated a specific P-value. P-values <0.05 were considered to be statistically different, and therefore significant.

For analysis of humoral responses the plasma fraction was collected from the whole blood preparations. An ELISA against Influenza NP antigen was performed and binding titers determined.

**Table 3: Experimental Details for Example 2**

| **Group Number (n/group)** | **Animal Eartag #** | **Plasmid** | **# of Injection Site(s) / Location / Tx** | **EP Device & Inj Method** | **Injection Volume (uL)** | **DNA dose / plasmid** |
|---|---|---|---|---|---|---|
| ***1. 4** | 190, 191, 192, 150 | pGX2013 | Left TA muscle | Elongated 3P | 200 | 20 µg |
| **2. 4** | 798**, 799, 176, 469 | pGX2013 | Left TA muscle | Elongated 3P | 200 | 20 µg |
| **3. 3** | 187, 188, 189 | PBS | Left TA muscle | Elongated 3P | 200 | 0 |

| | | | | | | |
|---|---|---|---|---|---|---|
| * treated with approximately 0.2 ml Hyaluronidase (about 0.4 Units/µl) in TA muscle 2 hours before vaccine delivery | | | | | | |

**Table 4: Plasmid Details for Example 2**

| **Code** | **Name** | **R&D Lot#** |
|---|---|---|
| pGX2013 | Influenza NP | D141202B |

Figure 19 shows IFN-y responses in PBMCs after stimulation with over-lapping peptide pools spanning the lengths of the Influenza NP PR8 antigen, as detected by ELISpot analysis 14 days after first (prime) or 7 days after second immunization (boost). Plasma IgG binding titers against Influenza NP (IMR-274) antigen were determined by ELISA and are shown in Figure 20.

This Example demonstrates that the addition of hyaluronidase (HYA) pretreatment of the TA muscle to a pDNA plus EP immunization regimen enhances the elicited host immune response. Figure 19 shows that there was about a 3.5-fold increase in IFN-y spots per million in the pNP vaccine group pretreated with HYA (about 922 SFU/million) compared to the pNP vaccine group not pretreated (about 258 SFU/million); these responses were measured 14 days after the prime immunization. This increase was approximately 3-fold 7 days after boost (about 3012 vs. about 1032 SFU/million). Figure 20 demonstrates that the humoral response was also increased in the pNP vaccine HYA pretreated group compared to the pNP vaccine group not pretreated with HYA.

### EXAMPLE 18

### DNA-Delivery Of Monospecific And Bispecific Monoclonal Antibodies Targeting Pseudomonas aeruginosa Protect Mice From Lethal Pneumonia

The opportunistic bacterial pathogen Pseudomonas aeruginosa is often multi-drug resistant and associated with poor clinical outcomes. Growing drug resistance and the lack of novel mechanism antibiotics in development require alternative antimicrobial strategies including pathogen-specific monoclonal antibodies (mAbs). MAbs targeting the *P. aeruginosa* type III secretion protein PcrV (V2L2-MD) and the Psl exopolysaccharide (EPS), each conferring potent individual and synergistic combined protective activities in preclinical infection models, are components of bispecific clinical candidate antibody1-1. DNA delivery of such mAbs could have significant advantages in clinical applications. Use of these mAb DNA sequences was explored to determine the feasibility of an alternative mAb delivery strategy by plasmid DNA delivery (DMAb) via electroporation, which was engineered for expressing both antibody heavy and light chains of full length human IgG1 in vivo.

Intramuscular injection (IM) sites consisted of both tibialis anterior muscles and the right biceps femoris muscle. Injections were administered parallel to the musculature. One hour prior to electroporation, hyaluronidase was delivered by IM injections, at 8U in 30µL per site. 100 µg in 30µL of each DMAb was delivered by IM injections at all three exact sites previously injected with hyaluronidase, then immediately electroporated. Mice were challenged intranasally with *P. aeruginosa* 5 days after electroporation procedure. DMAb in vivo IgG expression level was evaluated prior to infection.

Anti-PcrV monospecific V2L2-MD and bispecific antibody 1-1 heavy and light chain sequences were cloned into plasmid pGX001, resulting in DMAb-V2L2-MD and DMAb-antibody1-2. Each candidate was confirmed for expression in HEK293T cells before intramuscular injection followed by electroporation (IM-EP) in BALB/c mice. *In vivo* antibody expression was monitored up to 7 days post IM-EP as was the functional activity of the expressed mAbs (anti-PcrV anti-cytotoxic activity). DMAb-V2L2MD and DMAb-antibody 1-2 were also evaluated in a *P. aeruginosa* acute pneumonia model.

Serum antibody concentrations from both DMAb-V2L2-MD and DMAb-antibody 1-2 treated animals at day 7 post IM-EP correlated with measured ex vivo anti-cytotoxic activity, indicating that both V2L2-MD and antibody1-1 were expressed and functional. In addition, in an acute murine P. aeruginosa lung infection model, both DMAb-V2L2MD and DMAb-antibody 1-2 exhibited significant in vivo protective activity compared to a control IgG DMAb (90% and 100% survival vs. 0%, respectively; P<0.0001).

DMAb-V2L2-MD and DMAb-antibody1-2 are shown *in vivo* to prevent lethality in a murine lung infection model. In addition, our results suggest that DNA delivery of full length IgG mAbs may be a feasible platform strategy for preventing serious bacterial infections, and possibly adaptable for prophylaxis against other infectious agents for which highly potent mAbs have been identified and characterized.

### EXAMPLE 19

### Dose Sparing Effect Associated with Incorporation of Hyaluronidase in Vaccine Formulation

To determine whether a co-formulation of a pDNA vaccine with hyaluronidase has a dose sparing effect on the host immune response elicited, a total of 12 groups of BALB/c mice (6 per group) were treated with doses between 10 and 0.125 ug of pGX2013 (pDNA encoding for influenza nucleoprotein (NP)). Groups 1-6 received pGX2013 co-formulated with hyaluronidase (200U/ml), and groups 7-12 with SSC buffer only. Group 13 received SSC only (no vaccine). Details are presented in table 1. Immunization were performed on day 0. The vaccine delivery was performed by injection of 30 µl formulation intramuscularly (TA leg muscle), and electroporation was performed immediately or 60 seconds after injection in the without HYA and with HYA groups, respectively. Electroporation was applied to the site of injection using CELLECTRA^{®}-3P (3 mm electrodes) device (Inovio Pharmaceuticals, Inc.). Parameters were:
Number of pulses = 2 sets of 2 pulses (2x2),
Current Strength = 0.1 Amp,
Maximum Voltage = 200V,
Electroporation pulse duration = 52 milliseconds,
Interval separating pulses = 0.2 seconds between pulses, and 3 seconds between each set of pulses.

On days 7 and 14 mice were bleed and serum harvested. Humoral responses were detected using an ELISA against A/PR8/34 (H1N1) influenza virus nucleoprotein recombinant antigen. End-point binding titers were plotted.

On day 14 mice were sacrificed and spleens harvested. Antigen specific cellular responses to the A/PR8/34 (H1N1) influenza virus nucleoprotein H2d-restricted epitopes NP55-69 (class II) and NP147-155 (class I) were measured by a IFNγ ELISpot. The spot-forming units (SFU's) were counted and analyzed by ImmunoSpot Analyzers (Cellular Technology Ltd. in Shaker Heights, OH). SFU's per million splenocytes for each individual mouse and the mean +/- SEM of each group of mice were calculated and graphed. The statistical difference between a pair of immunized groups was assessed using a two-tailed unpaired t test that generated a specific P-value. P-values <0.05 were considered to be statistically different, and therefore significant.

**TABLE 5.**

| Group Number (n/group) | **Animal Eartag #** | **Plasmid** | **# of Injection Site(s) / Location / Tx** | **EP Device & Inj Method** | **Injection Volume (uL)** | **DNA dose / plasm id (ug)** |
|---|---|---|---|---|---|---|
| **1.* 6** | 301,302 ,303, 304,305 ,306 | pGX2013 | 1/left TA | CELLECTRA-3P | 30 | 10 |
| **2.* 6** | 307,308 ,309, 310,311 ,312 | pGX2013 | 1/left TA | CELLECTRA-3P | 30 | 5 |
| **3.* 6** | 313,314 ,315, 316,317 ,318 | pGX2013 | 1/left TA | CELLECTRA-3P | 30 | 1 |
| **4.* 6** | 319,320 ,321, 322,323 ,324 | pGX2013 | 1/left TA | CELLECTRA-3P | 30 | 0.5 |
| **5.* 6** | 325,326 ,327, 328,329 ,330 | pGX2013 | 1/left TA | CELLECTRA-3P | 30 | 0.25 |
| **6.* 6** | 331,332 ,333, 334,335,336 | pGX2013 | 1/left TA | CELLECTRA-3P | 30 | 0.125 |
| **7. 6** | 337,338 ,339, 340,341 ,342 | pGX2013 | 1/left TA | CELLECTRA-3P | 30 | 10 |
| **8. 6** | 343,344 ,345, 346,347 ,348 | pGX2013 | 1/left TA | CELLECTRA-3P | 30 | 5 |
| **9. 6** | 349,350 ,351, 352,353 ,354 | pGX2013 | 1/left TA | CELLECTRA-3P | 30 | 1 |
| **10. 6** | 355,356 ,357, 358,359 ,360 | pGX2013 | 1/left TA | CELLECTRA-3P | 30 | 0.5 |
| **11. 6** | 361,362 ,363, 364,365 ,366 | pGX2013 | 1/left TA | CELLECTRA-3P | 30 | 0.25 |
| **12. 6** | 367,368 ,369, 370,371 ,372 | pGX2013 | 1/left TA | CELLECTRA-3P | 30 | 0.125 |
| **13. 6 (neg control for ELISPOT)** | 373,374 ,375 376,377 ,378 | none | 1/left TA | CELLECTRA-3P | 30 | 0 |

| | | | | | | |
|---|---|---|---|---|---|---|
| * receive 200U/ml Hyaluronidase (Intrapharma) co-formulated with pDNA | | | | | | |

**Table 6: Plasmid Details for Example 4**

| **Code** | **Name** | **R&D Lot#** |
|---|---|---|
| pGX2013 | Influenza NP | D160926A |

ELISpot IFN-γ responses in splenocyte populations were enumerated after stimulation with PR8 nucleoprotein NP55 (CD4+ T cell) and NP147 (CD8+ T cell) peptide epitopes (Fig. 27). Serum IgG binding titers against PR8 nucleoprotein recombinant antigen were determined by ELISA (Fig. 28).

The additive effect on the host immune response elicited in BALB/c mice by co-formulation the pDNA vaccine with hyaluronidase is shown. The dose sparing effect of this delivery strategy is demonstrated here. Groups of BALB/c mice were immunized with 0.125 to 10 µg pNP vaccine delivered with or without hyaluronidase. Figure 27 shows equivalent cellular immune responses in groups immunized with pNP doses above 0.5 µg, however at lower doses there was a significant decrease in the CD8+ T cell responses to NP147 at 0.25 and 0.125 ug doses and the CD4+ T cell responses to NP55 at 0.125 ug in the groups immunized without hyaluronidase. The dose sparing effect of hyaluronidase co-formulation was also observed for humoral responses to the pNP vaccine at day 14 (Fig. 28). Furthermore at day 7 after immunization we could detect significant anti-NP antigen binding titers in the serum of the mice treated with higher doses of pNP with hyaluronidase, but not in mice without hyaluronidase.

A dose sparing effect is associated with the incorporation of hyaluronidase in the pDNA vaccine formulation. Furthermore, this co-formulation may be associated with an accelerated immune response at higher doses. See also figure 42.

### EXAMPLE 20

### Materials and Methods for Examples 21- 24

DNA-encoded monoclonal antibody construction and in vitro expression. Plasmid DNA-encoded monoclonal antibody (DMAb) constructs were engineered as previously described14, 15. pMERS was generated by use of synthetic oligonucleotides with several modifications encoding the light (VL) and heavy (VH) chains for the full-length anti-MERS envelope glycoprotein monoclonal antibody, and the final sequence was cloned into a human CMV driven promoter expression system. The resulting modified and enhanced immunogens were codon and RNA optimized, followed by cloning into the pVax1 expression vector by GenScript (Picastaway, NJ) with subsequent large-scale production of these constructs. The VH and VL genes were inserted between the BamH1 and Xho1 restriction sites. To confirm in vitro DMAb expression, human embryonic kidney 293T cells (ATCC) were transfected with 3 µg per 1 x 10⁶ cells of pMERS using Lipofectamine^{®} 3000 transfection reagent (Invitrogen, Carlsbad, CA). 48 hours later culture supernatants were harvested an MERS-CoV binding antibodies and hIgG levels were measured by ELISA (described below).

Animals. Female BALB/c and Crl:Nu-Foxn1 ^{nu} mice (7-8 weeks old) were purchased from Charles River Laboratories (Wilmington, MA). Female New Zealand White rabbits (10-12 weeks old, 2 to 2.5 kg) were purchased from Charles River Laboratories. Rhesus macaques 2.35 to 4.20 kg were purchased from WWP, Inc. (Miami, FL), and placed in quarantine for 33 days before study start. Mice were group housed, and rabbits and rhesus macaques were individually housed with ad libitum access to food and water. All animals were housed and handled at BTS Research (San Diego, CA) according to the standards of the Institutional Animal Care and Use Committee (IACUC).

Intramuscular pDNA delivery. Purified plasmid DNA was formulated in saline-sodium citrate (SSC) for subsequent administration into animals. For pretreatments, animals received an intramuscular pre-injection of 200U/ml Hyaluronidase purified from bovine testes (Sigma) in 1xDPBS (Thermofisher, MA). Mice received 30 µl into TA muscle, rabbit and rhesus macaque received 1ml into the quad muscle. After 30 minutes plasmid DNA was injected at the same site followed by immediate IM electroporation treatment. pDNA delivery into mouse TA muscle was assisted with the CELLECTRA^{®} 3P, and the CELLECTRA^{®} 5P was used for treatments of rabbits and rhesus macaques.

Human IgG quantification ELISA. 96-well assay plates (Thermo Scientific^{™} Nunc^{™}) were coated with 100 µl/well 10 µg/ml goat anti-huIgG Fc fragment antibody (Bethyl, TX) in 1x DPBS (Thermofischer, MA) overnight at 4°C. Next day plates were washed with 0.2% (v/v)TWEEN in 1xPBS wash buffer and blocked with 10%(v/v)FBS in 1xDPBS for 1hr at room temperature. The serum samples were diluted in 1% (v/v) FBS in 0.2% (v/v) TWEEN-1xPBS and 100 µl of this mix were added to the assay plate after another washing step. Additionally standard dilutions of purified human kappa light chain (Bethyl, TX) were prepared as 1:2 serial dilutions starting at 500 ng/ml in dilution buffer was prepared and added in duplicates to each assay plate. Samples and standard were incubated for 1hr at room temperature. After washing, the plates were incubated with a 1:10,000 dilution of goat anti-human IgG kappa light chain HRP (Bethyl, TX) for 1hr at room temperature. For detection 100 µl/well SureBlue Substrate solution (KPL, MD) was added to the washed plates. The reaction was stopped by adding 100 µl/well of TMB Stop Solution (KPL, MD) after 6min to the assay plates. The O.D. were read at 450nm. The serum-level expression was interpolated from the standard curve using a sigmoidal four parameter logistic curve fit for log of the concentration.

Antigen Binding ELISA. Assay plates were coated with 100 µl/well 1 µg/ml MERS-CoV Spike protein S1 (SinoBiological, China) in 1xDPBS (Thermofisher, MA) overnight at 4°C. Plates were washed with 1xPBS buffer with 0.05% TWEEN. 250ul/well of 3% (w/v) BSA in 1xPBS with 0.05% TWEEN were added and incubated for 1hr at room temperature. Serum samples were diluted in 1% (w/v) BSA in 1xPBS with 0.05% TWEEN. After washing the assay plates were filled with 100 µl/well 1%BSA PBS/TWEEN buffer. For antigen binding of reciprocal serum dilutions 1:3 50 µl serial dilutions were performed with pre-diluted serum samples on assay plates. Plates were incubated 1hr at room temperature. After washing 100 µl of 1:10000 diluted goat anti-human IgG heavy and light chain monkey-adsorbed antibody (bethyl, TX) was added and incubated for 1hr at roomtemperature. For development the SureBlue/TMB Stop Solution (KPL, MD) was used and O.D. was recorded at 450nm.

ADA ELISA. Assay plates were coated overnight at 4°C with 100 µl/well 0.3 µg/ml MERS DMAb purified from in-vitro transfected cell culture supernatant. Plates were washed with 1xPBS 0.05% TWEEN wash buffer and 250µl/well 3%BSA/PBS/TWEEN blocking buffer was added and incubated for 1hr at room temperature. Serum samples were pre-diluted 1:25 in 1%BSA/PBS/TWEEN dilution buffer. After washing the assay plates, 100ul/well of pre-diluted samples were added and incubated for 2hr at room temperature. For detection of ADA responses in rhesus macaques 100 µl/well 1: 10000 goat-anti human lambda light chain HRP antibody (Bethyl, TX) was added and incubated for 1hr at room temperature. Sufficient cross-reactivity of this detection antibody to rhesus macaque IgG and low cross-reactivity to the coating protein pMERS was confirmed before (data not shown). For ADA detection in mouse samples goat anti- mouse IgG Peroxidase (Sigma), for rabbit goat anti-rabbit IgG (Sigma) was diluted 1: 10000. SureBlue/TMB Stop Solution were used for plate development and O.D. values were recorded at 450nm.

### EXAMPLE 21

### Design, in vitro, and in vivo Characterization of DMAbs and Delivery Enhancement Strategies

DMAbs are defined as DNA plasmids encoding the light and heavy immunoglobulin (Ig) chains of a monoclonal antibody. Specifically, the DNA cassettes contain cDNAs for the coding sequences of the variable light (VL) and heavy (VH) Ig chains of the full length mAbs which have been optimized for expression and cloned into the pVax1 mammalian expression vector. For efficient separation of the heavy and light chain to permit the formation of a full length antibody from a single open-reading frame, a furin cleavage site and a 2A self-processing peptide were included in the design (Fig. 29a).

For these delivery optimization studies a plasmid DNA construct (pMERS) which encodes a human anti-Middle Eastern Respiratory Syndrome (MERS)-coronavirus (CoV) mAb was selected. pMERS was generated by use of synthetic oligonucleotides with several modifications encoding the light (VL) and heavy (VH) chains for the full-length anti-MERS envelope glycoprotein monoclonal antibody, the final sequence was cloned into a human CMV driven promoter expression system. Immunogloubulin heavy and light chain leader sequences were incorporated in order to improve expression. The resulting modified and enhanced immunogens were codon-and RNA optimized, and cloned into the pVax1 expression vector (Fig. 29a).

Production of mAbs from pMERS transfected cells was initially confirmed in vitro. Human 293T cells were transfected with the pMERS or the empty pVax plasmid. The levels of secreted antibody were quantified in the supernatant 48 hours after transfection by enzyme-linked immunosorbent assay (ELISA), Fig. 29b. To confirm the production of functional antibodies, anti-MERS antibody binding levels were measured against the MERS-CoV antigen by ELISA in the harvested supernatants (Fig. 29c). In summary, data demonstrated functional MERS-CoV antigen binding antibodies were secreted from transfected cells in vitro.

The in vivo expression of the anti-MERS DMAb following intramuscular delivery in the BALB/c mouse was investigated. Delivery of naked pDNA doses between 6.25 and 100 µg into the tibalis anterior (TA) muscle failed to produce serum human IgG (hIgG) levels above background (Fig. 30a). With the goal to increase the systemic hIgG levels in the serum, gene delivery strategies were studied which have been demonstrated to enhance in vivo gene transfer. Previous DMAb studies demonstrated enhanced expression with the employment of in vivo EP as a delivery aide. Electroporation (EP) is an established in vivo delivery aide for pDNA, increasing gene expression over 100 fold to naked pDNA delivery alone. The theory behind EP is that it increases the permeability of the cell membrane to permit efficient passage of large molecules (e.g pDNA) into the cell. At the low electrical parameters employed, the EP effect on the cell is transient, and the cell to remains fully functional. Here, the CELLECTRA^{®}-3P EP was used to target the TA muscle of the BALB/c mouse. The application of EP at the TA muscle delivery site immediately after DMAb injection led to increased serum hIgG levels (average 670 ng/ml for 50 µg pMERS dose at day 6 after delivery, Fig. 30b). Pharmacokinetic analysis of serum hIgG levels revealed peak expression at day 6 post treatment (Fig. 34). hIgG levels rapidly decreased after day 6, which coincided with the elicitation of a host immune response directed against the foreign human IgG protein (Fig. 35).

The use of hyaluronidase (HYA) in in vivo pDNA + EP delivery protocols has been previously demonstrated to enhance gene expression. HYA catalyzes the hydrolysis of hyaluronan in the ECM, allowing for a transient increase in tissue permeability. This allows for greater dispersion of the injected pDNA across the muscle tissue, and an increase in the number of myocytes accessible for transfection. HYA was therefore added to the delivery protocol with the aim of further increasing hIgG serum concentration. Pretreatment of the TA muscle with 200U/ml of HYA 30 minutes before gene delivery with electroporation resulted significantly higher levels (mean 2160 ng/ml (100 µg dose of pMERS on day 6 after delivery)) of serum hIgG (Fig. 30c). Delivery of pMERS using HYA pretreatment in the absence of EP was not associated with enhanced serum hIgG concentration (data not shown). The in vivo production of functional antibody (serum binding to MERS CoV antigen) by ELISA was performed. Reciprocal serum dilution binding was plotted on day 6 after pMERS delivery (Fig. 30d). Additionally, other reagents were studied to determine whether they enhanced intracellular gene delivery such as vitamin D, sucrose and polymers, or possessing ECM structure modifying capabilities such as elastase and collagenase, and determined whether their inclusion in the DMAb delivery protocol could significantly enhance hIgG levels. The addition of these reagents to the EP + pMERS delivery protocol failed to significantly enhance serum hIgG levels (Fig. 36). Furthermore, there was no increase in serum hIgG levels upon the addition of any of these reagents to the EP + HYA pMERS delivery protocol (data not shown).

The striking effect of HYA pretreatment of the delivery muscle on gene expression was observed visually upon delivery of reporter gene (pRFP) to mouse TA muscle (Fig. 30e). Additionally, a strong immunofluorescence signal upon assay for hIgG expression in myocytes at the site of treatment (Fig. 30f) was observed, confirming production of the antibody by the muscle cells at the site of pMERS delivery. In summary, data presented above delineates a DMAb delivery protocol which includes EP and HYA that can be adopted to enhance the expression of functional hIgG in the BALB/c mouse model.

### EXAMPLE 22

### Sustained DMAb Expression in Immunodeficient Mice

Studies in BALB/c or B6 mice successfully demonstrated in vivo expression of DMAb and the importance of delivery aides in enhancing tissue transfection and consequently systemic hIgG levels. However, the reaction of the host immune system against foreign hIgG protein negatively impacted expression kinetics (Figs. 34 and 35). To avoid the negative effect of the host immune response on DMAb expression, we investigated the PK of serum hIgG levels in immunodeficient BALB/c nude mice. In the absence of functional adaptive immune system hIgG serum levels did not crash after day 6, but continued to rise and plateau between days 21 and 35 (Fig. 31a). This resulted in higher concentrations of serum hIgG observed in nude mice than BALB/c mice (12.5 µg/ml vs 2.2 µg/ml mean peak in at 100 µg pMERS dose). DMAb expression was also sustained. Duration of expression analysis revealed hIgG serum levels of between 0.77 µg/ml (12.5 µg dose group) and 3.84 µg/ml (100 µg dose group) at 160 days after delivery (Fig. 31a).

It was hypothesized that increasing the number of myocytes transfected would result in higher the systemic hIgG levels. Thus targeting multiple muscle sites would be advantageous. 100 µg per site of pMERS was delivered to 1, 2, 3 or 4 muscles. Fig. 31b demonstrates increasing the number of muscle tissue sites targeted is advantageous. Delivering pMERS to the left and right TA and Quad muscles (4 sites in total) resulted in 28.8 µg/ml of hIgG detected in the serum on day 21. Delivering doses above 100 µg to a single TA muscle failed to further increase serum hIgG levels. In summary data in this section indicates in the absence of an anti-hIgG response, systemic levels DNA-encoded monoclonal antibody are increased and sustained. Furthermore, targeting multiple delivery sites is advantageous over increasing the local dose.

### EXAMPLE 23

### Sustained DMAb Expression in Immunodeficient Mice

A first step in the scaling up process was from the mouse (approximately 20 g in weight with a peripheral blood total volume of 2 ml) to the rabbit (approximately 2.5 kg in weight with a peripheral blood total volume of 140 ml), a species which is phylogenetically closer to primates than rodents. Furthermore the large size of the rabbit quadriceps muscle is compatible with the CELLECTRA^{®}-5P, the human intramuscular EP device currently being used in clinical trials.

The pMERS expression kinetics was determined in terms of serum human IgG in the New Zealand white rabbit. Rabbits were treated with a total of 2 mg pMERS delivered with CELLECTRA^{®}-5P EP into HYA-pretreated quadriceps muscle. Robust serum hIgG levels peaked on day 6 (Fig. 37a), before rapidly falling to background at day seven. This rapid loss of serum hIgG coincided with a strong anti-drug antibodies (ADA) developing between days 6 and 7 (Fig. 37b). However, a robust and consistent systemic expression at this early time point (day 6) in this model using the CELLECTRA^{®} 5P delivery system was obtained. The effect of hyaluronidase pretreatment of the muscle delivery site on hIgG expression was then demonstrated. Enhanced levels of serum hIgG was associated with HYA pretreatment of muscle compared to PBS pretreatment (Fig. 32a), 770 vs. 122 ng/ml (p < 0.0001) on day 6, respectively (Fig. 32b). Furthermore, the resulting hIgG was functional as determined by MERS CoV antigen binding (Fig. 32c).

Electrical settings for the CELLECTRA^{®} 5P IM EP device were previously optimized for delivery and immunogenicity of DNA vaccines, not DMAb plasmid. The optimal EP electrical parameters in terms of voltage for DMAb delivery in the context of HYA pretreatment was investigated. The hIgG serum levels associated with pMERS IM delivery at 20, 35, 50 and 65 volts was analyzed. To perform this voltage course the 5P array and pulse pattern settings were transferred to the BTS-12 box - a device capable of delivering a wide range of voltages. The 65 volts setting was comparable to the voltage delivered by the CELLECTRA^{®}-5P device in rabbit quad muscle. Results clearly demonstrated a significant loss in hIgG serum levels associated with a decrease in voltage (Fig. 32d). Due to the potential for an increase in tissue damage and a decrease in treatment tolerability, higher voltages were not investigated.

In conclusion, the presence of robust serum levels of functional hIgG at an early time point (day 6) after pMERS delivery with our optimized delivery protocol supported the advancement of the DMAb platform to larger animals.

### EXAMPLE 24

### Application of DMAb Delivery Optimizations to Nonhuman Primates

With a comparable physiology and immune system to the human, the nonhuman primate provides an exceptional model to study the translational potential of an antibody drug candidate into the clinic. Applying the DMAb delivery optimizations delineated in smaller animal models, the levels of hIgG in the serum of Rhesus macaques (Maccaca mulatto) were assessed. pMERS was delivered with CELLECTRA^{®} 5P EP into HYA-pretreated quad muscles of 5 Rhesus macaques. Systemic levels of hIgG were detected in the serum of all the NHPs (Fig. 33a). Serum hIgG Levels peaked between days 11 and 21 with a range of 1.30 to 4.97 µg/ml. To confirm the production of functional antibodies, serum antibody binding to MERS CoV antigen was confirmed by ELISA. MERS antigen binding values displayed a comparable kinetic profile to hIgG levels in the serum (Fig. 33b), and reciprocal serum dilution binding for each rhesus macaque was plotted on day 17 (Fig. 33c). Together these results confirmed the production of robust levels of human anti-MERS-CoV antibodies in NHPs.

To determine whether the elicitation of an ADA response to foreign hIgG impacted the pharmacokinetics of DMAb expression in NHPs, serum antibody binding to pMERS encoded purified protein hIgG by ELISA was assayed. An ADA response to hIgG in other experimental animal models was detected (Fig. 35 and 37b). In all NHPs we detected the elicitation of antibodies against DMAb (Fig. 33d), however, in four out of the five NHPs these ADA's were delayed compared to previous observations in other animals. The magnitude of the ADA correlated inversely with the hIgG serum levels (Spearman r = - 0.5811 (p = 0.0072), Fig. 33e). This strongly suggested the host immune response against the "non-self" human IgG negatively impacted DMAb expression levels in rhesus macaques.

In summary, the development of a delivery protocol which can be applied to significantly enhance the systemic expression of DNA-based monoclonal antibodies delivered to the muscle of small and large preclinical animal models is delineated. As shown here, the combined use of EP and hyaluronidase at the site of delivery permits one to achieve systemic serum hIgG levels in the µg/ml range in nonhuman primates.

### EXAMPLE 25

### Co-formulation of Hyaluronidase with pDNA in Rabbits and Rhesus macaques

Data show a pDNA/hyaluronidase (HYA) co-formulation can be delivered into the rabbit muscle without a loss of expression compared to standard HYA pretreatment protocol. See Figure 38. An EP delay of 60 seconds after injection of pDNA/HYA is associated with increased DMAb expression. See Figure 39.

Data also shows a pDNA /HY A co-formulation can be delivered into the NHP muscle without a loss of expression compared to standard HYA pretreatment protocol. An EP delay of 60 seconds after injection of pDNA/HYA is associated with increased DMAb expression. See figure 40.

### EXAMPLE 26

### Optimization of EP Delay

Experiments were conducted to optimize EP delay after co-formulation. Data shows that a 20-second EP delay after co-formulation injection may be optimal. See figure 41. Accordingly, significantly enhanced gene expression upon the incorporation of a time delay between drug delivery and EP compared to tissue pre-treatment with HYA protocols. Equivalent levels of gene expression were observed without the time delay when compared to tissue pre-treatment with HYA protocols.

### EXAMPLE 27

### Augmentation of Immune Response to Tumor Antigen with DNA Vaccine HYA Formulation

It was hypothesized that the inclusion of HYA to the vaccine formulation will enhance the host immune response elicited to a vaccine encoding a tumor associated self-antigen. The experimental results are shown in figure 43.

Various changes and modifications to the disclosed embodiments will be apparent to those skilled in the art. Such changes and modifications, including without limitation those relating to the chemical structures, substituents, derivatives, intermediates, syntheses, compositions, formulations, or methods of use of the invention, may be made without departing from the scope thereof.

It is understood that the foregoing detailed description and accompanying examples are merely illustrative and are not to be taken as limitations upon the scope of the invention, which is defined solely by the appended claims.

## Claims

1. A DNA plasmid encoding a monoclonal antibody for use in a method of treating a disease or disorder in a subject, in combination with chondroitinase AC or chondroitinase ABC, the method comprising:
administering to the subject chondroitinase AC or chondroitinase ABC prior to or concurrently with the DNA plasmid encoding a monoclonal antibody, and
administering the DNA plasmid via electroporation.

2. The DNA plasmid encoding a monoclonal antibody for use according to claim 1, wherein the monoclonal antibody is expressed *in vivo.*

3. The DNA plasmid encoding a monoclonal antibody for use according to claim 1 or 2, wherein the chondroitinase AC or chondroitinase ABC is administered to the subject prior to administration of the DNA plasmid encoding a monoclonal antibody.

4. The DNA plasmid encoding a monoclonal antibody for use according to claim 3, wherein the chondroitinase AC or chondroitinase ABC is administered to the subject at least about 15 minutes to about 24 hours prior to administration of the DNA plasmid encoding a monoclonal antibody.

5. The DNA plasmid encoding a monoclonal antibody for use according to claim 1 or 2, wherein the chondroitinase AC or chondroitinase ABC and the DNA plasmid encoding a monoclonal antibody are administered to the subject concurrently.

6. The DNA plasmid encoding a monoclonal antibody for use according to any preceding claim, wherein the chondroitinase AC or chondroitinase ABC and the DNA plasmid encoding a monoclonal antibody are administered to the subject subcutaneously or intramuscularly.

7. The DNA plasmid encoding a monoclonal antibody for use according to any preceding claim, wherein the chondroitinase AC or chondroitinase ABC and the DNA plasmid encoding a monoclonal antibody are co-formulated prior to administration.

## Patentansprüche

1. DNA-Plasmid, codierend einen monoklonalen Antikörper, zur Verwendung bei einem Verfahren zur Behandlung einer Krankheit oder Störung bei einem Individuum in Kombination mit Chondroitinase AC oder Chondroitinase ABC, wobei das Verfahren Folgendes umfasst:
Verabreichen von Chondroitinase AC oder Chondroitinase ABC an das Individuum vor oder gleichzeitig mit dem einen monoklonalen Antikörper codierenden DNA-Plasmid und Verabreichen des DNA-Plasmids über Elektroporation.

2. DNA-Plasmid, codierend einen monoklonalen Antikörper, zur Verwendung nach Anspruch 1, wobei der monoklonale Antikörper *in vivo* exprimiert wird.

3. DNA-Plasmid, codierend einen monoklonalen Antikörper, zur Verwendung nach Anspruch 1 oder 2, wobei die Chondroitinase AC oder Chondroitinase ABC dem Individuum vor Verabreichung des einen monoklonalen Antikörper codierenden DNA-Plasmids verabreicht wird.

4. DNA-Plasmid, codierend einen monoklonalen Antikörper, zur Verwendung nach Anspruch 3, wobei die Chondroitinase AC oder Chondroitinase ABC dem Individuum mindestens etwa 15 Minuten bis etwa 24 Stunden vor Verabreichung des einen monoklonalen Antikörper codierenden DNA-Plasmids verabreicht wird.

5. DNA-Plasmid, codierend einen monoklonalen Antikörper, zur Verwendung nach Anspruch 1 oder 2, wobei die Chondroitinase AC oder Chondroitinase ABC und das einen monoklonalen Antikörper codierende DNA-Plasmid dem Individuum gleichzeitig verabreicht werden.

6. DNA-Plasmid, codierend einen monoklonalen Antikörper, zur Verwendung nach einem vorhergehenden Anspruch, wobei die Chondroitinase AC oder Chondroitinase ABC und das einen monoklonalen Antikörper codierende DNA-Plasmid dem Individuum subkutan oder intramuskulär verabreicht werden.

7. DNA-Plasmid, codierend einen monoklonalen Antikörper, zur Verwendung nach einem vorhergehenden Anspruch, wobei die Chondroitinase AC oder Chondroitinase ABC und das einen monoklonalen Antikörper codierende DNA-Plasmid vor der Verabreichung zusammen formuliert werden.

## Revendications

1. Plasmide d'ADN codant pour un anticorps monoclonal destiné à être utilisé dans un procédé de traitement d'une maladie ou d'un trouble chez un sujet, en combinaison avec la chondroïtinase AC ou la chondroïtinase ABC, le procédé comprenant :
l'administration au sujet de chondroïtinase AC ou de chondroïtinase ABC avant ou en même temps que le plasmide d'ADN codant pour un anticorps monoclonal, et
l'administration du plasmide d'ADN via une électroporation.

2. Plasmide d'ADN codant pour un anticorps monoclonal destiné à être utilisé selon la revendication 1, dans lequel l'anticorps monoclonal est exprimé *in vivo.*

3. Plasmide d'ADN codant pour un anticorps monoclonal destiné à être utilisé selon la revendication 1 ou 2, dans lequel la chondroïtinase AC ou la chondroïtinase ABC est administrée au sujet avant l'administration du plasmide d'ADN codant pour un anticorps monoclonal.

4. Plasmide d'ADN codant pour un anticorps monoclonal destiné à être utilisé selon la revendication 3, dans lequel la chondroïtinase AC ou la chondroïtinase ABC est administrée au sujet au moins environ 15 minutes à environ 24 heures avant l'administration du plasmide d'ADN codant pour un anticorps monoclonal.

5. Plasmide d'ADN codant pour un anticorps monoclonal destiné à être utilisé selon la revendication 1 ou 2, dans lequel la chondroïtinase AC ou la chondroïtinase ABC et le plasmide d'ADN codant pour un anticorps monoclonal sont administrés au sujet simultanément.

6. Plasmide d'ADN codant pour un anticorps monoclonal destiné à être utilisé selon l'une quelconque des revendications précédentes, dans lequel la chondroïtinase AC ou la chondroïtinase ABC et le plasmide d'ADN codant pour un anticorps monoclonal sont administrés au sujet par voie sous-cutanée ou par voie intramusculaire.

7. Plasmide d'ADN codant pour un anticorps monoclonal destiné à être utilisé selon l'une quelconque des revendications précédentes, dans lequel la chondroïtinase AC ou la chondroïtinase ABC et le plasmide d'ADN codant pour un anticorps monoclonal sont co-formulés avant administration.
